# EUROPEAN PATENT SPECIFICATION

(11) **EP 2 032 552 B1**
(45) Date of publication and mention of the grant of the patent: **24.02.2016**
(21) Application number: 07764648.7
(22) Date of filing: 14.06.2007
(51) Int. Cl.: C07D 311/58, C09K 19/34

(54) **Mesogenic compounds, liquid crystal medium and liquid crystal display**
Mesogene Verbindungen, flüssigkristallines Medium und Flüssigkristallanzeige
Composés mésogènes, milieu à cristaux liquides et affichage à cristaux liquides

(30) Priority: 23.06.2006 EP 06012948
(43) Date of publication of application: 11.03.2009
(73) Proprietor: Merck Patent GmbH, 64293 Darmstadt (DE)
(72) Inventor: BERNATZ, Georg, 64289 Darmstadt (DE); BEST, Peter, 64289 Darmstadt (DE); GOETZ, Achim, 64665 Alsbach-Haehnlein (DE); KIRSCH, Peer, Kanagawa 224-0033 (JP)
(86) International application number: PCT/EP2007/005232
(87) International publication number: WO 2007/147516

(56) References cited:
- WO-A-2006/040009
- WO-A1-2004/046805
- DE-A1-102005 045 849

## Description

### Field of the invention

The present invention relates to compounds, media comprising these compounds and to electro-optical displays comprising these media as light modulation media. Preferably the compounds of the present invention are mesogenic compounds and they are preferably used in liquid crystalline media. In particular the electro-optical displays according to the present invention are displays, which are operated at a temperature, at which the mesogenic modulation media are in an optically isotropic phase, preferably in a blue phase.

### Problem to be solved and state of the art

Electro-optical displays and mesogenic light modulation media, which are in the isotropic phase when being operated in the display are described in DE 102 17 273 A. Electro-optical displays, and mesogenic light modulation media, which are in the optically isotropic blue phase, when being operated in the display are described in WO 2004/046 805.

The mesogenic media and displays described in these references provide several significant advantages compared to well-known and widely used displays using liquid crystals in the nematic phase, like for example liquid crystal displays (LCDs) operating in the twisted nematic (TN)-, the super twisted nematic (STN)-, the electrically controlled birefringence (ECB)-mode with its various modifications and the in-plane switching (IPS)-mode as for example described in WO 2006/040009 A and US 6,045, 878. Amongst these advantages are most pronounced their much faster switching times, and significantly wider optical viewing angle.

Whereas, compared to displays using mesogenic media in another liquid crystalline phase, as e.g. in the smectic phase in surface stabilized ferroelectric liquid crystal displays (SSF LCDs), the displays of DE 102 17 273.0 and WO 2004/046 805 are much easier to manufacture.

For example, they do not require a very thin cell gap and in addition the electro-optical effect is not very sensitive to small variations of the cell gap.

However, the liquid crystal media described in these patent applications mentioned still require operating voltages, which are not low enough for some applications. Further the operating voltages of these media vary with temperature, and it is generally observed, that at a certain temperature the voltage dramatically increases with increasing temperature. This limits the applicability of liquid crystal media in the blue phase for display applications. A further disadvantage of the liquid crystal media described in these patent applications is their moderate reliability which is insufficient for very demanding applications. This moderate reliability may be for example expressed in terms of the voltage holding ratio (VHR) parameter, which in liquid crystal media as described above may be below 90%.

Some compounds and compositions have been reported which possess a blue phase between the cholesteric phase and the isotropic phase and can usually be observed by optical microscopy. These compounds or compositions for which the blue phases are observed are typically single mesogenic compounds or mixtures showing a high chirality. However, generally the blue phases observed only extend over a very small temperature range, which is typically less than 1 degree centigrade wide, and/or the blue phase is located at rather inconvenient temperatures.

In order to operate the novel fast switching display mode of WO 2004/046 805 the light modulation medium to be used has to be in the blue phase over a broad range of temperatures encompassing ambient temperature, however. Thus, a light modulation medium possessing a blue phase, which is as wide as possible and conveniently located is required.

Therefore there is a strong need for a modulation medium with a blue phase with a wide phase range, which may be achieved either by an appropriate mixture of mesogenic compounds themselves or, preferably by mixing a host mixture with appropriate mesogenic properties with a single dopant or a mixture of dopants that stabilises the blue phase over a wide temperature range.

Summarizing, there is a need for liquid crystal media, which can be operated in liquid crystal displays, which are operated at temperatures where the media is in the blue phase, which provide the following technical improvements:
- a reduced operating voltage,
- a reduced temperature dependency of the operating voltage and
- an improved reliability, e.g. VHR.

### Present invention

Surprisingly, it now has been found that mesogenic media comprising a dielectrically positive component, component A, comprising a) at least one compound of formula I 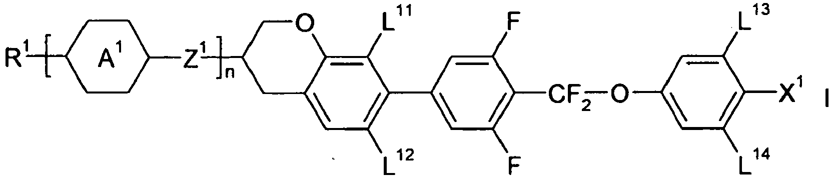 wherein
- L¹¹ to L¹⁴: are, independently of each other, H or F, 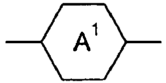 is an aromatic and/or alicyclic ring, or a group comprising two or more fused aromatic or alicyclic rings, wherein these rings optionally contain one or more hetero atoms selected from N, O and/or S, and are optionally mono-, di- or polysubstituted by R,
- R: is halogen, preferably F or Cl, CN, NCS, SCN, SF₅, SO₂CF₃ or alkyl, which is straight chain or branched, preferably has 1 to 20 C-atoms, is unsubstituted, mono-or poly-substituted by F, Cl, or CN, preferably by F, and in which one or more CH₂ groups are optionally replaced, in each case independently from one another, by -O-, -S-, -NH-, -NR⁰¹-, -SiR⁰¹R⁰²- -CO-, -COO-, -OCO-, -OCO-O-, -S-CO-, -CO-S-, -CY⁰¹=CY⁰¹- or -C≡C- in such a manner that O and/or S atoms are not linked directly to one another, preferably halogen, n-alkyl, n-alkoxy with 1 to 9 C-atoms, preferably 2 to 5 C-atoms, alkenyl, alkenyloxy or alkoxyalkyl with 2 to 9 C-atoms, preferably with 2 to 5 C-atoms or CN, NCS, F, Cl, halogenated alkyl, alkenyl or alkoxy, preferably mono-, di fluorinated or oligofluorinated alkyl, alkenyl or alkoxy, especially preferred n-alkyl, n-alkoxy, alkenyl, alkenyloxy or alkoxyalkyl,
- R¹: has the meaning given for R
- Z¹: is -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰¹-, -CR⁰¹=CH-, -CY⁰¹=CY⁰²-, -C=C-, -(CH₂)₄-, -CH=CH-CO-O-, -O-CO-CH=CH- or a single bond, preferably -CO-O-, -O-CO-, -CF₂-O-, -O-CF₂- or a single bond, and more preferably a single bond
- Y⁰¹ and Y⁰²: are, independently of each other, F, Cl or CN, and alternatively one of them may be H,
- R⁰¹ and R⁰²: are, independently of each other, H or alkyl with 1 to 12 C-atoms,
- X¹: is halogen, preferably F or Cl, OCF₃, CF₃, OCHF₂, CN, NCS, SCN, SF₅ or SO₂CF₃, preferably F, OCF₃, CF₃, CN, or SF₅, and
- n: is 0 or 1, preferably 1,

b) 1-20 % by weight of at least one chiral compound or more chiral compounds with a HTP of ≥ 20 µm⁻¹, and
   said mesogenic media of a) and b) exhibiting a Blue Phase allow to realize media with an acceptably high clearing point and/or a rather high stability of the voltage holding ratio against temperature and/or UV-load and in particular against the latter.

Preferred are compounds of formula I wherein the parameters have the following meaning
- L¹¹: is F and/or
at least two, more preferably at least three of
- L¹¹ to L¹⁴: are F, and/or
- R¹: is alkyl or alkenyl and/or 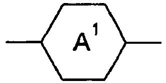 preferably is, 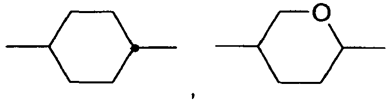 or 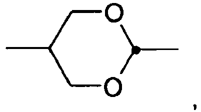 and/or
- Z¹¹: is -CO-O-, -CF₂-O-, or a single bond, more preferably a single bond.

The compounds of formula I are preferably selected from the compounds its sub-formulae I-1 to I-8 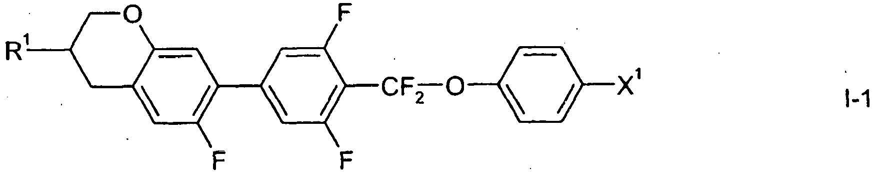 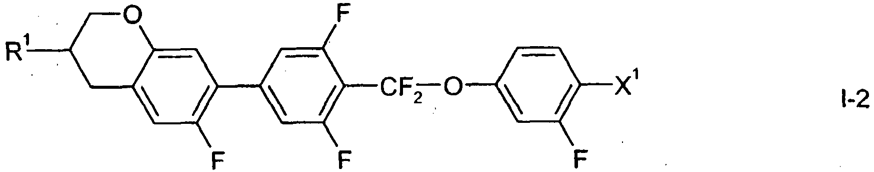 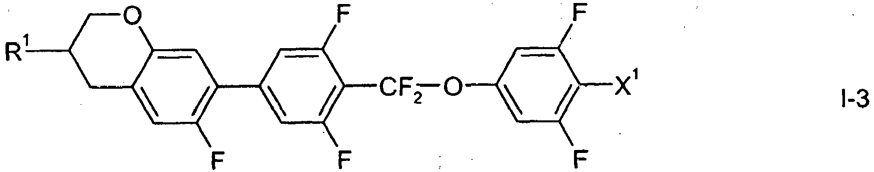 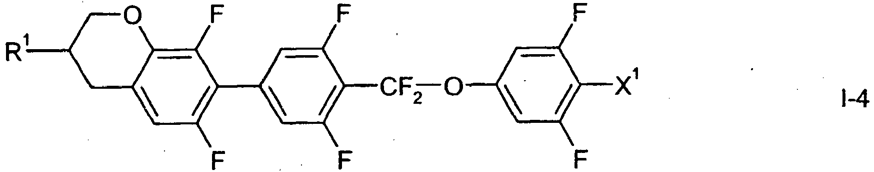 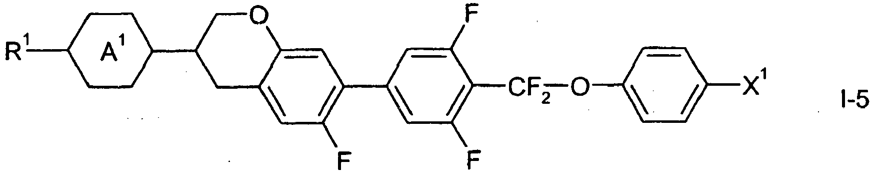 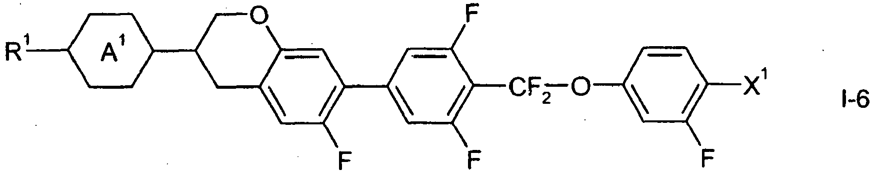 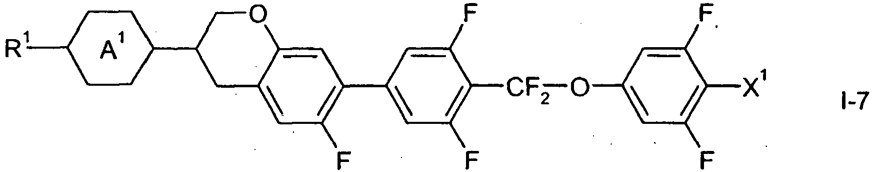 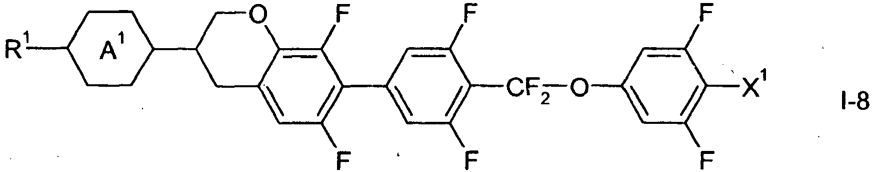 wherein the parameters have the respective meanings given above, and preferably 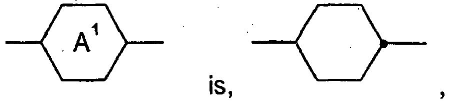 and/or
- R¹: is alkyl or alkenyl, and/or
- X¹: is F, Cl, OCF₃, CF₃, CN, or SF₅.

An alkyl or an alkoxy radical, i.e. an alkyl where the terminal CH₂ group is replaced by -O-, in this application may be straight-chain or branched. It is preferably straight-chain, has 1, 2, 3, 4, 5, 6, 7 or 8 carbon atoms and accordingly is preferably methyl, ethyl, propyl, butyl, pentyl, hexyl, heptyl, octyl, ethoxy, propoxy, butoxy, pentoxy, hexoxy, heptoxy, or octoxy, furthermore nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. an alkyl group in which one non-terminal CH₂ group is replaced by -O-, is preferably straight-chain 2-oxapropyl (= methoxymethyl), 2- (= ethoxymethyl) or 3-oxabutyl (= 2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

An alkenyl group, i.e. an alkyl group wherein one or more CH₂ groups are replaced by -CH=CH-, may be straight-chain or branched. It is preferably straight-chain, has 2 to 10 C atoms and accordingly is preferably vinyl, prop-1-, or prop-2-enyl, but-1-, 2- or but-3-enyl, pent-1-, 2-, 3- or pent-4-enyl, hex-1-, 2-, 3-, 4- or hex-5-enyl, hept-1-, 2-, 3-, 4-, 5- or hept-6-enyl, oct-1-, 2-, 3-, 4-, 5-, 6- or oct-7-enyl, non-1-, 2-, 3-, 4-, 5-, 6-, 7- or non-8-enyl, dec-1-, 2-, 3-, 4-, 5-, 6-, 7-, 8- or dec-9-enyl.

Especially preferred alkenyl groups are C₂-C₇-1E-alkenyl, C₄-C₇-3E-alkenyl, C₅-C₇-4-alkenyl, C₆-C₇-5-alkenyl and C₇-6-alkenyl, in particular C₂-C₇-1E-alkenyl, C₄-C₇-3E-alkenyl and C₅-C₇-4-alkenyl. Examples for particularly preferred alkenyl groups are vinyl, 1 E-propenyl, 1 E-butenyl, 1 E-pentenyl, 1 E-hexenyl, 1 E-heptenyl, 3-butenyl, 3E-pentenyl, 3E-hexenyl, 3E-heptenyl, 4-pentenyl, 4Z-hexenyl, 4E-hexenyl, 4Z-heptenyl, 5-hexenyl, 6-heptenyl and the like. Groups having up to 5 C-atoms are generally preferred.

In an alkyl group, wherein one CH₂ group is replaced by -O- and one by -CO-, these radicals are preferably neighboured. Accordingly these radicals together form a carbonyloxy group -CO-O- or an oxycarbonyl group -O-CO-. Preferably such an alkyl group is straight-chain and has 2 to 6 C atoms.

It is accordingly preferably acetyloxy, propionyloxy, butyryloxy, pentanoyloxy, hexanoyloxy, acetyloxymethyl, propionyloxymethyl, butyryloxymethyl, pentanoyloxymethyl, 2-acetyloxyethyl, 2-propionyloxy-ethyl, 2-butyryloxyethyl, 3-acetyloxypropyl, 3-propionyloxypropyl, 4-acetyloxybutyl, methoxycarbonyl, ethoxycarbonyl, propoxycarbonyl, butoxycarbonyl, pentoxycarbonyl, methoxycarbonylmethyl, ethoxycarbonylmethyl, propoxycarbonylmethyl, butoxycarbonylmethyl, 2-(methoxycarbonyl)ethyl, 2-(ethoxycarbonyl)ethyl, 2-(propoxy-carbonyl)ethyl, 3-(methoxycarbonyl)propyl, 3-(ethoxycarbonyl)propyl, 4-(methoxycarbonyl)-butyl.

An alkyl group wherein two or more CH₂ groups are replaced by -O- and/or -COO-, it can be straight-chain or branched. It is preferably straight-chain and has 3 to 12 C atoms. Accordingly it is preferably bis-carboxy-methyl, 2,2-bis-carboxy-ethyl, 3,3-bis-carboxy-propyl, 4,4-bis-carboxy-butyl, 5,5-bis-carboxy-pentyl, 6,6-bis-carboxy-hexyl, 7,7-bis-carboxy-heptyl, 8,8-bis-carboxy-octyl, 9,9-bis-carboxy-nonyl, 10,10-bis-carboxy-decyl, bis-(methoxycarbonyl)-methyl, 2,2-bis-(methoxycarbonyl)-ethyl, 3,3-bis-(methoxycarbonyl)-propyl, 4,4-bis-(methoxycarbonyl)-butyl, 5,5-bis-(methoxycarbonyl)-pentyl, 6,6-bis-(methoxycarbonyl)-hexyl, 7,7-bis-(methoxycarbonyl)-heptyl, 8,8-bis-(methoxycarbonyl)-octyl, bis-(ethoxycarbonyl)-methyl, 2,2-bis-(ethoxycarbonyl)-ethyl, 3,3-bis-(ethoxycarbonyl)-propyl, 4,4-bis-(ethoxycarbonyl)-butyl, 5,5-bis-(ethoxycarbonyl)-hexyl.

A alkyl or alkenyl group that is monosubstituted by CN or CF₃ is preferably straight-chain. The substitution by CN or CF₃ can be in any desired position.

An alkyl or alkenyl group that is at least monosubstituted by halogen, it is preferably straight-chain. Halogen is preferably F or Cl, in case of multiple substitution preferably F. The resulting groups include also perfluorinated groups. In case of monosubstitution the F or Cl substituent can be in any desired position, but is preferably in ω-position. Examples for especially preferred straight-chain groups with a terminal F substituent are fluoromethyl, 2-fluoroethyl, 3-fluoropropyl, 4-fluorobutyl, 5-fluoropentyl, 6-fluorohexyl and 7-fluoroheptyl. Other positions of F are, however, not excluded.

Halogen means F, Cl, Br and I and is preferably F or Cl, most preferably F. Each of R¹, R⁵, R, R' and R" may be a polar or a non-polar group. In case of a polar group, it is preferably selected from CN, SF₅, halogen, OCH₃, SCN, COR⁵, COOR⁵ or a mono- oligo- or polyfluorinated alkyl or alkoxy group with 1 to 4 C atoms. R⁵ is optionally fluorinated alkyl with 1 to 4, preferably 1 to 3 C atoms. Especially preferred polar groups are selected of F, Cl, CN, OCH₃, COCH₃, COC₂H₅, COOCH₃, COOC₂H₅, CF₃, CHF₂, CH₂F, OCF₃, OCHF₂, OCH₂F, C₂F₅ and OC₂F₅, in particular F, Cl, CN, CF₃, OCHF₂ and OCF₃. In case of a non-polar group, it is preferably alkyl with up to 15 C atoms or alkoxy with 2 to 15 C atoms.

Each of R¹ and R may be an achiral or a chiral group. In case of a chiral group it is preferably of formula I*: 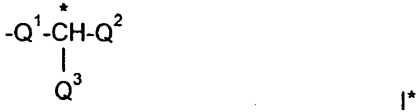 wherein
- Q¹: is an alkylene or alkylene-oxy group with 1 to 9 C atoms or a single bond,
- Q²: is an alkyl or alkoxy group with 1 to 10 C atoms which may be unsubstituted, mono- or polysubstituted by F, Cl, Br or CN, it being also possible for one or more non-adjacent CH₂ groups to be replaced, in each case independently from one another, by -C≡C-, -O-, -S-, -NH-, -N(CH₃)-, -CO-, -COO-, -OCO-, -OCO-O-, -S-CO- or -CO-S- in such a manner that oxygen atoms are not linked directly to one another,
- Q³: is F, Cl, Br, CN or an alkyl or alkoxy group as defined for Q² but being different from Q².

In case Q¹ in formula I* is an alkylene-oxy group, the O atom is preferably adjacent to the chiral C atom.

Preferred chiral groups of formula I* are 2-alkyl, 2-alkoxy, 2-methylalkyl, 2-methylalkoxy, 2-fluoroalkyl, 2-fluoroalkoxy, 2-(2-ethin)-alkyl, 2-(2-ethin)-alkoxy, 1,1,1-trifluoro-2-alkyl and 1,1,1-trifluoro-2-alkoxy.

Particularly preferred chiral groups I* are 2-butyl (=1-methylpropyl), 2-methylbutyl, 2-methylpentyl, 3-methylpentyl, 2-ethylhexyl, 2-propylpentyl, in particular 2-methylbutyl, 2-methylbutoxy, 2-methylpentoxy, 3-methylpentoxy, 2-ethylhexoxy, 1-methylhexoxy, 2-octyloxy, 2-oxa-3-methylbutyl, 3-oxa-4-methylpentyl, 4-methylhexyl, 2-hexyl, 2-octyl, 2-nonyl, 2-decyl, 2-dodecyl, 6-methoxyoctoxy, 6-methyloctoxy, 6-methyloctanoyloxy, 5-methylheptyloxycarbonyl, 2-methylbutyryloxy, 3-methylvaleroyloxy, 4-methylhexanoyloxy, 2-chlorpropionyloxy, 2-chloro-3-methylbutyryloxy, 2-chloro-4-methylvaleryloxy, 2-chloro-3-methylvaleryloxy, 2-methyl-3-oxapentyl, 2-methyl-3-oxahexyl, 1-methoxypropyl-2-oxy, 1-ethoxypropyl-2-oxy, 1-propoxypropyl-2-oxy, 1-butoxypropyl-2-oxy, 2-fluorooctyloxy, 2-fluorodecyloxy, 1,1,1-trifluoro-2-octyloxy, 1,1,1-trifluoro-2-octyl, 2-fluoromethyloctyloxy for example. Very preferred are 2-hexyl, 2-octyl, 2-octyloxy, 1,1,1-trifluoro-2-hexyl, 1,1,1-trifluoro-2-octyl and 1,1,1-trifluoro-2-octyloxy.

In addition, compounds containing an achiral branched alkyl group may occasionally be of importance, for example, due to a reduction in the tendency towards crystallization. Branched groups of this type generally do not contain more than one chain branch. Preferred achiral branched groups are isopropyl, isobutyl (= methylpropyl), isopentyl (= 3-methylbutyl), isopropoxy, 2-methyl-propoxy and 3-methylbutoxy.

In a preferred embodiment of the present invention one or more of R¹, R, and R' are -SG-PG.

Especially preferred are compounds of formula I and its sub-formulae wherein R¹ is -SG-PG and additionally preferred m is 0 at the same time.

The polymerisable or reactive group PG is preferably selected from CH₂=CW¹-COO-, 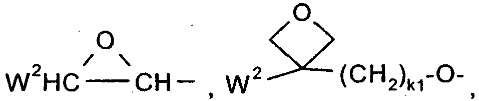 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

Especially preferably PG is a vinyl group, an acrylate group, a methacrylate group, an oxetane group or an epoxy group, especially preferably an acrylate or methacrylate group.

As for the spacer group SG all groups can be used that are known for this purpose to those skilled in the art. The spacer group SG is preferably of formula SG'-X, such that PG-SG- is PG-SG'-X-, wherein
- SG': is alkylene with up to 20 C atoms which may be unsubstituted, mono-or poly-substituted by F, Cl, Br, I or CN, it being also possible for one or more non-adjacent CH₂ groups to be replaced, in each case independently from one another, by -O-, -S-, -NH-, -NR⁰¹-, -SiR⁰¹R⁰²-, -CO-, -COO-, -OCO-, -OCO-O-, -S-, -CO-, -CO-S-, -CH=CH- or -C≡C-in such a manner that O and/or S atoms are not linked directly to one another,
- X: is -O-, -S-, -CO-, -COO-, -OCO-, -O-COO-, -CO-NR⁰¹-, -NR⁰¹-CO-,-OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰¹-, -CY⁰¹=CY⁰²-, -C≡C-, -CH=CH-COO-, -OCO-, -CH=CH- or a single bond,and
- R⁰¹, R⁰², Y⁰¹ and Y⁰²: have one of the respective meanings given above.
- X: is preferably -O-, -S-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CY⁰²=CY⁰²-, -C≡C- or a single bond, in particular -O-, -S-, -C≡C-, -CY⁰¹=CY⁰²- or a single bond, very preferably a group that is able to from a conjugated system, such as -C≡C- or -CY⁰¹=CY⁰²-, or a single bond.

Typical groups SG' are, for example, -(CH₂)ₚ-, -(CH₂CH₂O)_{q} -CH₂CH₂-, -CH₂CH₂-S-CH₂CH₂- or -CH₂CH₂-NH-CH₂CH₂- or -(SiR⁰R⁰⁰-O)ₚ-, with p being an integer from 2 to 12, q being an integer from 1 to 3 and R⁰, R⁰⁰ and the other parameters having the meanings given above.

Preferred groups SG' are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyl-iminoethylene, 1-methylalkylene, ethenylene, propenylene and butenylene for example.

In another preferred embodiment SG' is a chiral group of formula I*': 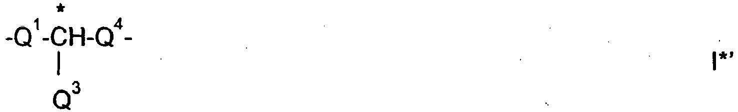 wherein
- Q¹ and Q³: have the meanings given in formula I*, and
- Q⁴: is an alkylene or alkylene-oxy group with 1 to 10 C atoms or a single bond, being different from Q¹,
with Q¹ being linked to the polymerisable group PG.

Further preferred are compounds with one or two groups PG-SG- wherein SG is a single bond.

In case of compounds with two groups PG-SG, each of the two polymerisable groups PG and the two spacer groups SG can be identical or different.

Preferable compounds of formula I according to the instant invention are the following exemplary compounds 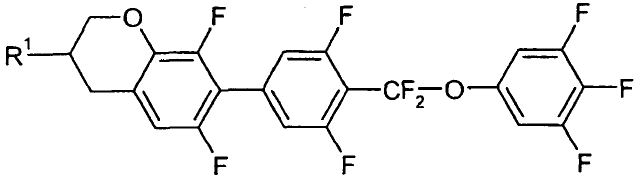 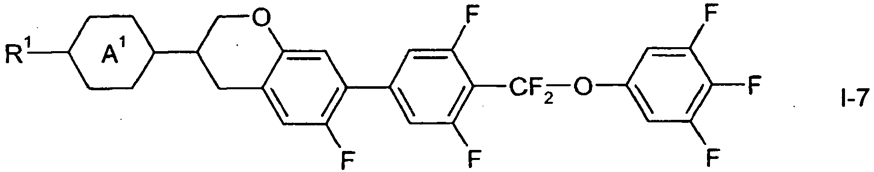 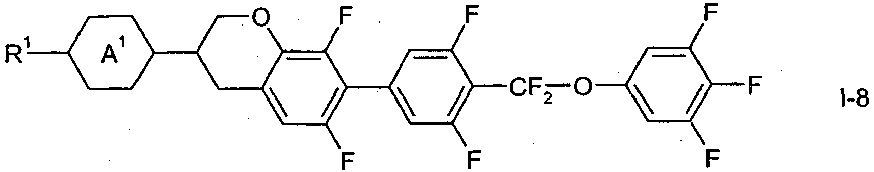 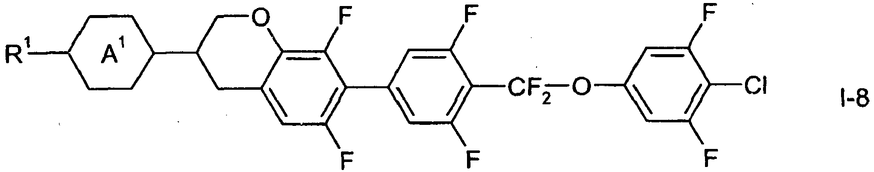 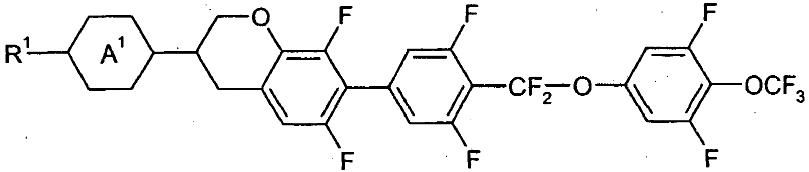 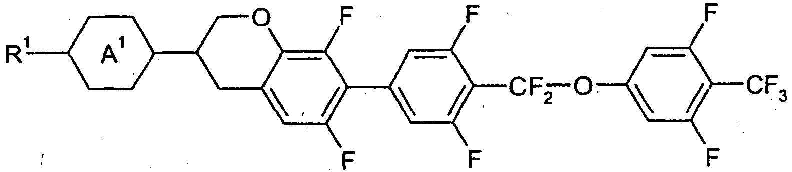 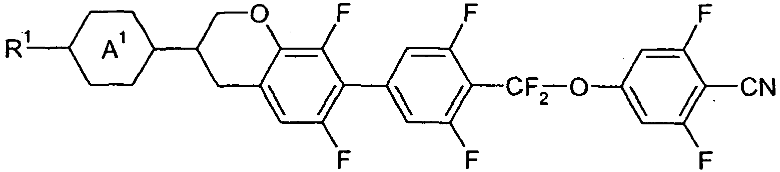 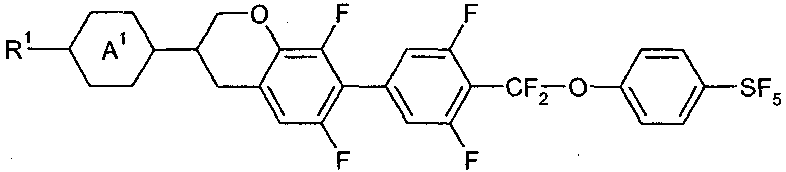 wherein the parameters have the respective meanings given above and preferably 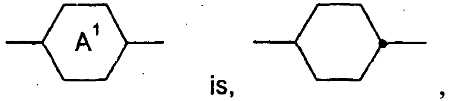 and/or
- R¹: is alkyl or alkenyl.

The compounds of formula I are accessible by the usual methods known to the expert. Starting materials may be, e.g., compounds of the following types, which are either commercially available or accessible by published methods:
Preferably the liquid crystalline media according to the instant invention contain a component A comprising, preferably predominantly consisting of and most preferably entirely consisting of compounds of formula I.

Comprising in this application means in the context of compositions that the entity referred to, e.g. the medium or the component, contains the compound or compounds in question, preferably in a total concentration of 10 % or more and most preferably of 20 % or more.

Predominantly consisting, in this context, means that the entity referred to contains 80 % or more, preferably 90 % or more and most preferably 95 % or more of the compound or compounds in question.

Entirely consisting, in this context, means that the entity referred to contains 98 % or more, preferably 99 % or more and most preferably 100.0 % of the compound or compounds in question.

The concentration of the compounds according to the present application are contained in the media according to the present application preferably is in the range from 0.5 % or more to 70 % or less, more preferably in the range from 1 % or more to 60 % or less and most preferably in the range from 5 % or more to 50 % or less.

In a preferred embodiment the mesogenic modulation media according to the instant invention comprise
- a component A, preferably in a concentration of 1 % to 25 % by weight, comprising, preferably predominantly consisting of and most preferably entirely consisting of, one compound or more compounds of the formula I 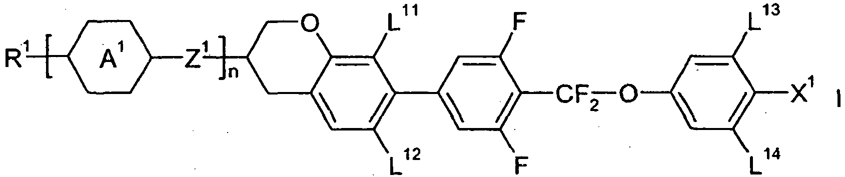 wherein
   - L¹¹ to L¹⁴: are, independently of each other, H or F, 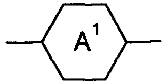 is an aromatic and/or alicyclic ring, or a group comprising two or more fused aromatic or alicyclic rings, wherein these rings optionally contain one or more hetero atoms selected from N, O and/or S, and are optionally mono-, di- or polysubstituted by R,
   - R: is halogen, preferably F or Cl, CN, NCS, SCN, SF₅, SO₂CF₃ or alkyl, which is straight chain or branched, preferably has 1 to 20 C-atoms, is unsubstituted, mono-or poly-substituted by F, Cl, or CN, preferably by F, and in which one or more CH₂ groups are optionally replaced, in each case independently from one another, by -O-, -S-, -NH-, -NR⁰¹. -SiR⁰¹R⁰²-, -CO-, -COO-. -OCO-, -OCO-O-, -S-CO-, -CO-S-, -CY⁰¹=CY⁰¹- or -C≡C- in such a manner that O and/or S atoms are not linked directly to one another, preferably halogen, n-alkyl, n-alkoxy with 1 to 9 C-atoms preferably 2 to 5 C-atoms, alkenyl, alkenyloxy or alkoxyalkyl with 2 to 9 C-atoms, preferably with 2 to 5 C-atoms or CN, NCS, F, Cl, halogenated alkyl, alkenyl or alkoxy, preferably mono-, di fluorinated or oligofluorinated alkyl, alkenyl or alkoxy, especially preferred n-alkyl, n-alkoxy, alkenyl, alkenyloxy or alkoxyalkyl, .
   - R¹: has the meaning given for R
   - Z¹: is -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰¹-, -CR⁰¹=CH-, -CY⁰¹=CY⁰²-, -C≡C-, -(CH₂)₄-, -CH=CH-CO-O-, -O-CO-CH=CH- or a single bond, preferably -CO-O-, -O-CO-, -CF₂-O-, -O -CF₂ - or a single bond, and more preferably a single bond,
   - Y⁰¹ and Y⁰²: are, independently of each other, F, Cl or CN, and alternatively one of them may be H,
   - R⁰¹ and R⁰²: are, independently of each other, H or alkyl with 1 to 12 C-atoms,
   - X¹: is halogen, preferably F or Cl, OCF₃, CF₃, OCHF₂, CN, NCS, SCN, SF₅ or SO₂CF₃, preferably F, OCF₃, CF₃, CN, or SF₅, and
   - n: is 0, 1, preferably 1, and
- optionally a dielectrically positive component B comprising, preferably predominantly consisting of and most preferably entirely consisting of one compound or of more compounds of formula II 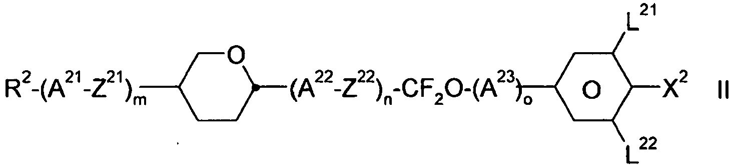 wherein
   - R²: has the meaning given under formula I for R¹,
   - A²¹, A²² and A²³: are, each independently of each other, 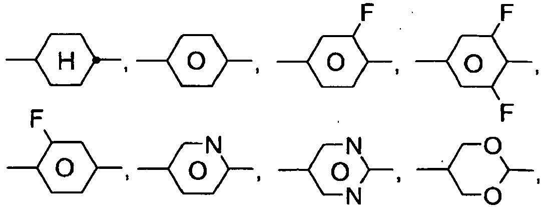 whereby each of A²¹ and A²² may have the same or a different meaning if present twice,
   - Z²¹ and Z²²: are, each independently of each other, a single bond, -(CH₂)₄)-, -CH₂CH₂-, -CF₂-CF₂-, -CF₂-CH₂-, -CH₂-CF₂-. -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- or -O-CO-, whereby each of Z²¹ and Z²² may have the same or a different meaning if present twice,
   - X²: is halogen, -CN, -NCS, -SF₅, -SO₂CF₃, alkyl, alkenyl, alkenyloxy or alkylalkoxy or alkoxy radical each mono-or polysubstituted by CN and/or halogen,
   - L²¹ and L²²: are, each independently of each other, H or F, and
   - m: is 0, 1 or 2,
   - n: is 0, 1, 2 or 3,
   - o: is 0, 1 or 2, preferably 0 or 1 and
   - m + n + o: is 3 or less, preferably 2 or less,
- optionally a component C, preferably in a concentration of 1 % to 25 % by weight, comprising, preferably predominantly consisting of and most preferably entirely consisting of one compound or of more compounds of formula III 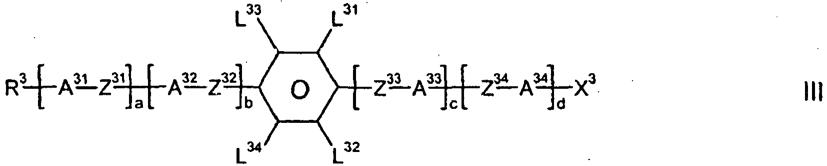 wherein
   - a, b, c and d: are each independently of each other 0, 1 or 2, whereby
   - a + b + c + d: is 4 or less,
   - A³¹, A³², A³³ and A³⁴: are, each independently of each other, 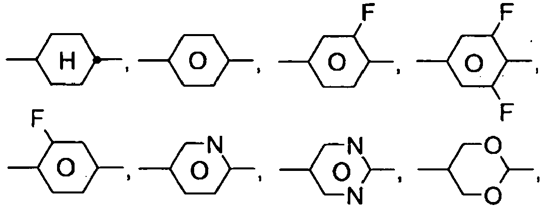 whereby each of A³¹, A³², A³³ and A³⁴ may have the same or a different meaning if present twice,
   - Z³¹, Z³², Z³³ and Z³⁴: are, each independently of each other, a single bond, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O- -O(CH₂)₃-, -CH=CF-, -C≡D-, -CH₂O-, -OCH₂-, -CF₂O-, -OCF₂-, -CO-O- or -O-CO-, whereby each of Z³¹ , Z³², Z³³ and Z³⁴ may have the same or a different meaning if present twice,
   - R³: is an alkyl or alkoxy radical having from 1 to 15 carbon atoms, wherein one or more methylene groups of said alkyl or alkoxy radical may be replaced independently of each other by -O-, -S-, -SiR^{x}R^{y}-, -CH=CH-, -C≡D-, -CO-O- and/or -O-CO- such that oxygen and/or sulfur atoms are not linked directly to each other, said alkyl or alkoxy radical being unsubstituted or mono-substituted with a CN group or mono- or poly-substituted with halogen, preferably R³ is a straight-chain alkyl, alkoxy, alkenyl, alkenyloxy or-O-alkylene-O-alkyl radical with up to 10 carbon atoms, said radicals being unsubstituted or mono- or poly-substituted with halogen,
   - L³¹, L³², L³³ and L³⁴: are each independently of each other hydrogen, halogen, a CN group, an alkyl or alkoxy radical having from 1 to 15 carbon atoms wherein one or more methylene groups of said alkyl or alkoxy radical may be replaced independently of each other by -O-, -S-, -SiR^{x}R^{y}-, -CH=CH-, -C≡D-, -CO-O- and/or -O-CO- such that oxygen and/or sulfur atoms are not linked directly to each other, said alkyl or alkoxy radical being unsubstituted or mono-substituted with a -CN group or mono- or poly-substituted with halogen, with the proviso that at least one of L³¹, L³², L³³ and L³⁴ is not hydrogen,
   - X³: is F, Cl, CF₃, OCF₃, CN, NCS, SF₅ or SO₂-R^{z},
   - R^{x} and R^{y}: are independently of each other hydrogen or an alkyl radical having from 1 to 7 carbon atoms; preferably R^{x} and R^{y} are both methyl, ethyl, propyl or butyl, and
   - R^{z}: is an alkyl radical having from 1 to 7 carbon atoms, said alkyl radical being unsubstituted or mono- or poly-substituted with halogen; preferably R^{z} is CF₃, C₂F₅ or n-C₄F₉,
- 1-20 % by weight of component D comprising one chiral compound or more chiral compounds with a HTP of ≥ 20 µm⁻¹,
- optionally a component E, which consists of compounds enhancing the phase range of the blue phase and/or decrease the temperature dependence of the electro-optical effect, and.
- optionally a component E, which is a polymer precursor, comprising reactive compounds, preferably comprising reactive mesogens, which upon polymerisation stabilize the phase range of the blue phase and/or decrease the temperature dependence of the electro-optical effect.

The inventive mixtures preferably contain 1-75 wt.%, preferably 2-70 wt.% and most preferably 3-65 wt.% of component A.

Suitable chiral compounds of component D are those, which have an absolute value of the helical twisting power of 20 µm⁻¹ or more, preferably of 40 µm or more and most preferably of 60 µm⁻¹ or more. The HTP is measured in MLC-6260 at a temperature of 20°C.

The chiral component D comprises preferably one or more chiral compounds which have a mesogenic structure und exhibit preferably one or more meso-phases themselves, particularly at least one cholesteric phase. Preferred chiral compounds being comprised in the chiral component D are, amongst others, well known chiral dopants like cholesteryl-nonanoate (CN), R/S-811, R/S-1011, R/S-2011, R/S-3011, R/S-4011, R/S-5011, CB-15 (Merck KGaA, Darmstadt, Germany). Preferred are chiral dopants having one or more chiral moieties and one or more mesogenic groups or having one or more aromatic or alicyclic moieties forming, together with the chiral moiety, a mesogenic group. More preferred are chiral moieties and mesogenic chiral compounds disclosed in DE 34 25 503, DE 35 34 777, DE 35 34 778, DE 35 34 779, DE 35 34 780, DE 43 42 280, EP 01 038 941 and DE 195 41 820 that disclosure is incorporated within this application by way of reference. Particular preference is given to chiral binaphthyl derivatives as disclosed in EP 01 111 954.2, chiral binaphthol derivatives as disclosed in WO 02/34739, chiral TADDOL derivatives as disclosed in WO 02/06265 as well as chiral dopants having at least one fluorinated linker and one end chiral moiety or one central chiral moiety as disclosed in WO 02/06196 and WO 02/06195.

The controlling medium of the present invention has a characteristic temperature, preferably a clearing point, in the range from about -30°C to about 90°C, especially up to about 70°C or even 80°C.

The inventive mixtures contain one ore more (two, three, four or more) chiral compounds in the range of 1-25 wt.%, preferably 2-20 wt.%. Especially preferred are mixtures containing 3-15 wt.% total of one or more chiral compounds.

Preferred embodiments are indicated below:
- The medium comprises one, two, three or more compounds of formula I and/or
- Component B preferably contains besides one compound ore more compounds of formula II one ester compound or more ester compounds of the formula Z 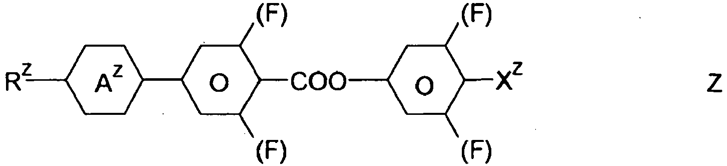 wherein R^{Z} has the meaning given under formula I for R, 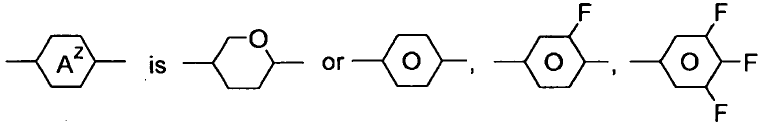
   - X^{Z}: is F, Cl, CN, NCS, OCF₃, CF₃ or SF₅.
   wherein R^{Z} has the meaning given under formula II for R².

Especially preferred are mixtures containing 5 % to 35 %, preferably 10 % to 30 % and especially preferred 10 % to 20 % of compounds of formula Z.
- The component B preferably contains additionally one or more compounds of formula N 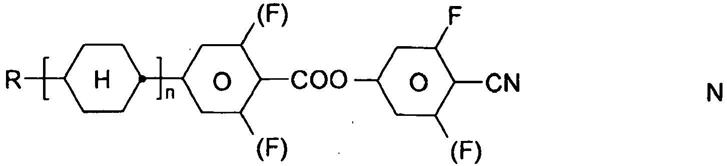 wherein
   - R: has the meaning given under formula I for R and preferably is alkyl or Alkyl-C≡C,
   - "Alkyl": is alkyl with 1 to 7 C-atoms, preferably n-alkyl, and
   - n: is 0 or 1.
- The component B preferably additionally comprises one or more compounds selected from the group of ester compounds of formula E 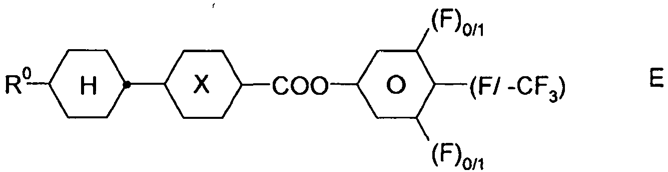 in which R⁰ has the meaning given for R under formula I and preferably is alkyl and 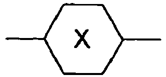 is 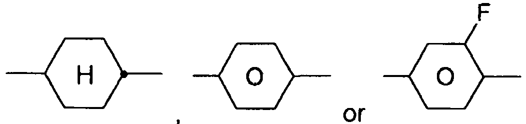
- The proportion of the compounds of formula E is preferably 10-30% by weight, in particular 15 % to 25 %.
- The medium preferably comprises one compound or more compounds selected from the group of formulae Q-1 and Q-2 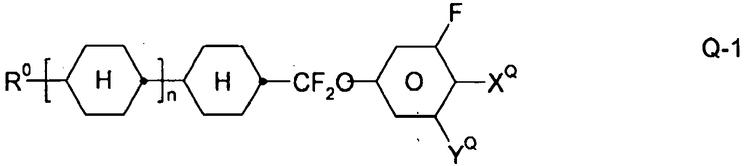 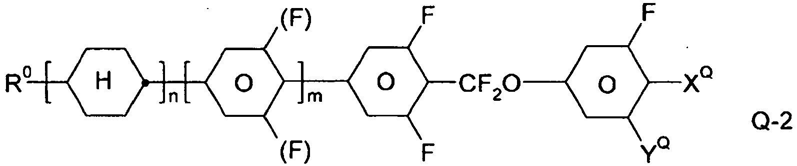 wherein R⁰ has the meaning given for R under formula I and n and m are, independently of each other 0 or 1.
- The medium preferably comprises one compound or more compounds selected from the group of compounds of formula II in which R⁰ is methyl.
- The medium preferably comprises one dioxane compound, two or more dioxane compounds, preferably one dioxane compound or two dioxane compounds, selected from the group of formulae Dx-1 and Dx-2 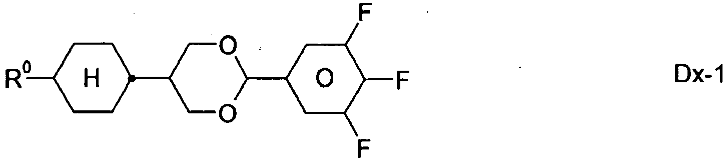 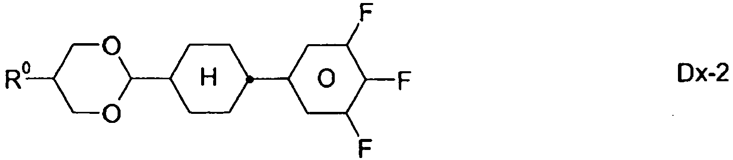 wherein R⁰ has the meaning given for R under formula I.

Preferably the compounds of formula II are selected from its sub-formulae II-1 to II-4 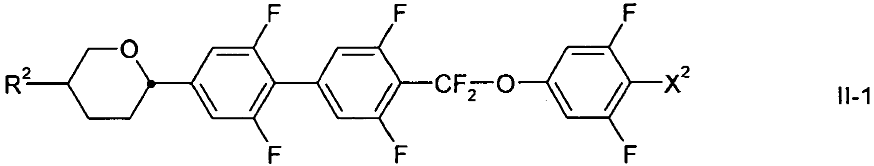 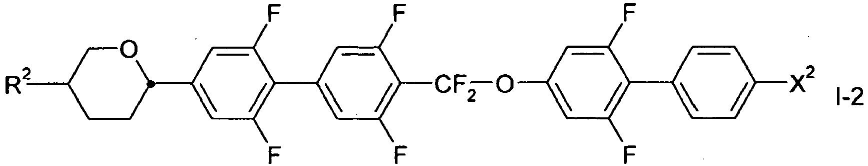 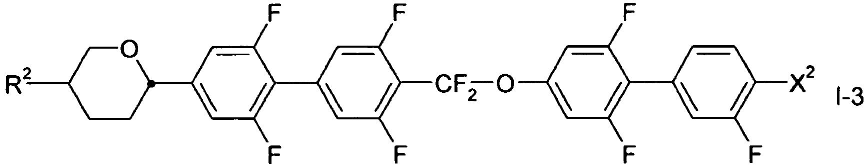 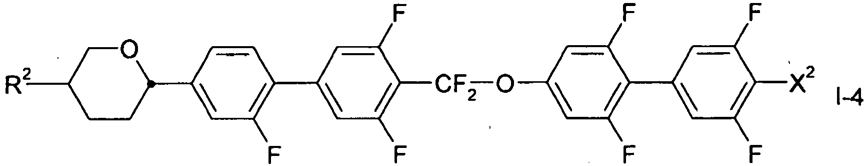 wherein the parameters have the respective meanings given above, and preferably
- R²: is alkyl or alkenyl, and/or
- X²: is F, Cl, OCF₃, CF₃, CN, or SF₅.

It has been found that even a relatively small proportion of compounds of the formula I mixed with conventional liquid-crystal materials, but in particular with one or more compounds of the formulae II and III, leads to a lower operating voltage and a broader operating temperature range. Preference is given, in particular, to mixtures which, besides one or more compounds of the formula I, comprise one or more compounds of the formula II, in particular compounds of the formula II in which X² is F, Cl, CN, NCS, CF₃ or OCF₃. The compounds of the formulae I to III are colourless, stable and readily miscible with one another and with other liquid-crystalline materials.

The optimum mixing ratio of the compounds of the formulae I and II and III depends substantially on the desired properties, on the choice of the components of the formulae I, II and/or III, and on the choice of any other components that may be present. Suitable mixing ratios within the range given above can easily be determined from case to case.

The total amount of compounds of the formulae I to III in the mixtures according to the invention is not crucial. The mixtures can therefore comprise one or more further components for the purposes of optimisation of various properties. However, the observed effect on the operating voltage and the operating temperature range is generally greater, the higher the total concentration of compounds of the formulae I to III.

In a particularly preferred embodiment, the media according to the invention comprise compounds of the formula III which X³ is F, OCF₃, OCHF₂, OCH=CF₂, OCF=CF₂ or OCF₂-CF₂H. A favourable synergistic effect with the compounds of the formula I results in particularly advantageous properties. In particular, mixtures comprising compounds of formula I and of formula II and of formula III are distinguished by their low operating voltages.

The individual compounds of the formulae II to III, which can be used in the media according to the invention, are either known or can be prepared analogously to the known compounds.

The construction of the MLC display according to the invention from polarisers, electrode base plates and surface-treated electrodes corresponds to the conventional construction for displays of this type. The term conventional construction is broadly drawn here and also covers all derivatives and modifications of the MLC display, in particular including matrix display elements based on poly-Si TFT or MIM, however, particularly preferred are displays, which have electrodes on just one of the substrates, i.e. so called inter-digital electrodes, as those used in IPS displays, preferably in one of the established structures.

A significant difference between the displays according to the invention and the conventional displays based on the twisted nematic cell consists, however, in the choice of the liquid-crystal parameters of the liquid-crystal layer.

The media according to the invention are prepared in a manner conventional per se. In general, the components are dissolved in one another, advantageously at elevated temperature. By means of suitable additives, the liquid-crystalline phases in accordance with the invention can be modified in such a way that they can be used in all types of liquid crystal display elements that have been disclosed hitherto. Additives of this type are known to the person skilled in the art and are described in detail in the literature (H. Kelker and R. Hatz, Handbook of Liquid Crystals, Verlag Chemie, Weinheim, 1980). For example, pleochroic dyes can be added for the preparation of coloured guest-host systems or substances can be added in order to modify the dielectric anisotropy, the viscosity and/or the alignment of the nematic phases. Furthermore, stabilisers and antioxidants can be added.

The mixtures according to the invention are suitable for TN, STN, ECB and IPS applications and isotropic switching mode (ISM) applications. Hence, there use in an electro-optical device and an electro-optical device containing liquid crystal media comprising at least one compound according to the invention are subject matters of the present invention.

The inventive mixtures are highly suitable for devices, which operate in an optically isotropic state. The mixtures of the invention are surprisingly found to be highly suitable for the respective use.

Electro-optical devices that are operated or operable in an optically isotropic state recently have become of interest with respect to video, TV, and multi-media applications. This is, because conventional liquid crystal displays utilizing electro-optical effects based on the physical properties of liquid crystals exhibit a rather high switching time, which is undesired for said applications. Furthermore most of the conventional displays show a significant viewing angle dependence of contrast that in turn makes necessary measures to compensate this undesired property.

With regard to devices utilizing electro-optical effects in an isotropic state the German Patent Application DE 102 17 273 A1 for example discloses light-controlling (light modulation) elements in which the mesogenic controlling medium for modulation is in the isotropic phase at the operating temperature. These light controlling elements have a very short switching time and a good viewing angle dependence of contrast. However, the driving or operating voltages of said elements are very often unsuitably high for some applications.

German Patent Application DE 102 41 301 yet unpublished describes specific structures of electrodes allowing a significant reduction of the driving voltages. However, these electrodes make the process of manufacturing the light controlling elements more complicated.

Furthermore, the light controlling elements, for example, disclosed in both DE 102 17 273 A1 and DE 102 41 301 show significant temperature dependence. The electro-optical effect that can be induced by the electrical field in the controlling medium being in an optical isotropic state is most pronounced at temperatures close to the clearing point of the controlling medium. In this range the light controlling elements have the lowest values of their characteristic voltages and, thus, require the lowest operating voltages. As temperature increases, the characteristic voltages and hence the operating voltages increase remarkably. Typical values of the temperature dependence are in the range from about a few volts per centigrade up to about ten or more volts per centigrade. While DE 102 41 301 describes various structures of electrodes for devices operable or operated in the isotropic state, DE 102 17 273 A1 discloses isotropic media of varying composition that are useful in light controlling elements operable or operated in the isotropic state. The relative temperature dependence of the threshold voltage in these light controlling elements is at a temperature of 1 centigrade above the clearing point in the range of about 50%/centigrade. That temperature dependence decreases with increasing temperature so that it is at a temperature of 5 centigrade above the clearing point of about 10%/centigrade. However, for many practical applications of displays utilizing said light controlling elements the temperature dependence of the electro-optical effect is too high. To the contrary, for practical uses it is desired that the operating voltages are independent from the operating temperature over a temperature range of at least some centi-grades, preferably of about 5 centi-grades or more, even more preferably of about 10 centi-grades or more and especially of about 20 centi-grades or more.

Now it has been found that the use of the inventive mixtures are highly suitable as controlling media in the light controlling elements as described above and in DE 102 17 273 A1, DE 102 41 301 and DE 102 536 06 and broaden the temperature range in which the operating voltages of said electro-optical operates. In this case the optical isotropic state or the blue phase is almost completely or completely independent from the operating temperature.

This effect is even more distinct if the mesogenic controlling media exhibit at least one so-called "blue phase" as described in yet unpublished WO 2004/046 805. Liquid crystals having an extremely high chiral twist may have one or more optically isotropic phases. If they have a respective cholesteric pitch, these phases might appear bluish in a cell having a sufficiently large cell gap. Those phases are therefore also called "blue phases" (Gray and Goodby, "Smectic Liquid Crystals, Textures and Structures", Leonhard Hill, USA, Canada (1984)). Effects of electrical fields on liquid crystals existing in a blue phase are described for instance in H.S. Kitzerow, "The Effect of Electric Fields on Blue Phases", Mol. Cryst. Liq. Cryst. (1991), Vol. 202, p. 51-83, as well as the three types of blue phases identified so far, namely BP I, BP II, and BP III, that may be observed in field-free liquid crystals. It is noteworthy, that if the liquid crystal exhibiting a blue phase or blue phases is subjected to an electrical field, further blue phases or other phases different from the blue phases I, II and III might appear.

The inventive mixtures can be used in an electro-optical light-controlling element which comprises
- one or more, especially two substrates;
- an assembly of electrodes;
- one or more elements for polarizing the light; and
- said controlling medium;
whereby said light-controlling element is operated (or operable) at a temperature at which the controlling medium is in an optically isotropic phase when it is in a non-driven state.

The controlling medium of the present invention has a characteristic temperature, preferably a clearing point, in the range from about -30°C to about 90°C, especially up to about 70°C to 80 °C.

The operating temperature of the light controlling elements is preferably above the characteristic temperature of the controlling medium said temperature being usually the transition temperature of the controlling medium to the blue phase; generally the operating temperature is in the range of about 0.1 ° to about 50 °, preferably in the range of about 0.1 ° to about 10 ° above said characteristic temperature. It is highly preferred that the operating temperature is in the range from the transition temperature of the controlling medium to the blue phase up to the transition temperature of the controlling medium to the isotropic phase which is the clearing point. The light controlling elements, however, may also be operated at temperatures at which the controlling medium is in the isotropic phase.

(For the purposes of the present invention the term "characteristic temperature" is defined as follows:
- If the characteristic voltage as a function of temperature has a minimum, the temperature at this minimum is denoted as characteristic temperature.
- If the characteristic voltage as a function of temperature has no minimum and if the controlling medium has one or more blue phases, the transition temperature to the blue phase is denoted as characteristic temperature; in case there are more than one blue phase, the lowest transition temperature to a blue phase is denoted as characteristic temperature.
- If the characteristic voltage as a function of temperature has no minimum and if the controlling medium has no blue phase, the transition temperature to the isotropic phase is denoted as characteristic temperature.)

In the context of the present invention the term "alkyl" means, as long as it is not defined in a different manner elsewhere in this description or in the claims, straight-chain and branched hydrocarbon (aliphatic) radicals with 1 to 15 carbon atoms. The hydrocarbon radicals may be unsubstituted or substituted with one or more substituents being independently selected from the group consisting of F, Cl, Br, I or CN.

The dielectrics may also comprise further additives known to the person skilled in the art and described in the literature. For example, 0 to 5% of pleochroic dyes, antioxidants or stabilizers can be added.

C denotes a crystalline phase, S a smectic phase, S_{C} a smectic C phase, N a nematic phase, I the isotropic phase and BP the blue phase.

V_{X} denotes the voltage for X% transmission. Thus e.g. V₁₀ denotes the voltage for 10% transmission and V₁₀₀ denotes the voltage for 100% transmission (viewing angle perpendicular to the plate surface). tₒₙ (respectively τₒₙ) denotes the switch-on time and t_{off} (respectively τ_{off}) the switch-off time at an operating voltage corresponding the value of V₁₀₀, respectively of Vₘₐₓ.

Δn denotes the optical anisotropy. Δε denotes the dielectric anisotropy (Δε = ε_{∥} - ε_{⊥}, where ε_{∥} denotes the dielectric constant parallel to the longitudinal molecular axes and ε_{∥} denotes the dielectric constant perpendicular thereto). The electro-optical data are measured in a TN cell at the 1^{st} minimum of transmission (i.e. at a (d · Δn) value of 0.5 µm) at 20°C, unless expressly stated otherwise. The optical data are measured at 20°C, unless expressly stated otherwise.

Optionally, the light modulation media according to the present invention can comprise further liquid crystal compounds in order to adjust the physical properties. Such compounds are known to the expert. Their concentration in the media according to the instant invention is preferably 0 % to 30 %, more preferably 0 % to 20 % and most preferably 5 % to 15 %.

Preferably inventive media have a range of the blue phase or, in case of the occurrence of more than one blue phase, a combined range of the blue phases, with a width of 20° or more, preferably of 40° or more, more preferably of 50° or more and most preferably of 60° or more.

In a preferred embodiment this phase range at least from 10°C to 30°C, most preferably at least from 10°C to 40°C and most preferably at least from 0°C to 50°C, wherein at least means, that preferably the phase extends to temperatures below the lower limit and at the same time, that it extends to temperatures above the upper limit.

In another preferred embodiment this phase range at least from 20°C to 40°C, most preferably at least from 30°C to 80°C and most preferably at least from 30°C to 90°C. This embodiment is particularly suited for displays with a strong backlight, dissipating energy and thus heating the display.

In the present application the term dielectrically positive compounds describes compounds with Δε > 1,5, dielectrically neutral compounds are compounds with -1,5 ≤ Δε ≤ 1,5 and dielectrically negative compounds are compounds with Δε < -1,5. The same holds for components. Δε is determined at 1 kHz and 20 °C. The dielectric anisotropies of the compounds is determined from the results of a solution of 10 % of the individual compounds in a nematic host mixture. The capacities of these test mixtures are determined both in a cell with homeotropic and with homogeneous alignment. The cell gap of both types of cells is approximately 20 µm. The voltage applied is a rectangular wave with a frequency of 1 kHz and a root mean square value typically of 0.5 V to 1.0 V, however, it is always selected to be below the capacitive threshold of the respective test mixture.

For dielectrically positive compounds the mixture ZLI-4792 and for dielectrically neutral, as well as for dielectrically negative compounds, the mixture ZLI-3086, both of Merck KGaA, Germany are used as host mixture, respectively. The dielectric permittivities of the compounds are determined from the change of the respective values of the host mixture upon addition of the compounds of interest and are extrapolated to a concentration of the compounds of interest of 100 %.

Components having a nematic phase at the measurement temperature of 20 °C are measured as such, all others are treated like compounds.

The term threshold voltage refers in the instant application to the optical threshold and is given for 10 % relative contrast (V₁₀) and the term saturation voltage refers to the optical saturation and is given for 90 % relative contrast (V₉₀) both, if not explicitly stated otherwise. The capacitive threshold voltage (V₀, also called Freedericksz-threshold V_{Fr}) is only used if explicitly mentioned.

The ranges of parameters given in this application are all including the limiting values, unless explicitly stated otherwise.

Throughout this application, unless explicitly stated otherwise, all concentrations are given in mass percent and relate to the respective complete mixture, all temperatures are given in degrees centigrade (Celsius) and all differences of temperatures in degrees centigrade. All physical properties have been and are determined according to "Merck Liquid Crystals, Physical Properties of Liquid Crystals", Status Nov. 1997, Merck KGaA, Germany and are given for a temperature of 20 °C, unless explicitly stated otherwise. The optical anisotropy (Δn) is determined at a wavelength of 589.3 nm. The dielectric anisotropy (Δε) is determined at a frequency of 1 kHz. The threshold voltages, as well as all other electro-optical properties have been determined with test cells prepared at Merck KGaA, Germany. The test cells for the determination of Δε had a cell gap of 22 µm. The electrode was a circular ITO electrode with an area of 1.13 cm² and a guard ring. The orientation layers were lecithin for homeotropic orientation (ε_{∥}) and polyimide AL-1054 from Japan Synthetic Rubber for homogenous orientation (ε_{⊥}). The capacities were determined with a frequency response analyser Solatron 1260 using a sine wave with a voltage of 0.3 or 0.1 Vᵣₘₛ. The light used in the electro-optical measurements was white light. The set up used was a commercially available equipment of Otsuka, Japan. The characteristic voltages have been determined under perpendicular observation. The threshold voltage (V₁₀), mid-grey voltage (V₅₀) and saturation voltage (V₉₀) have been determined for 10 %, 50 % and 90 % relative contrast, respectively.

The mesogenic modulation material has been filled into an electro optical test cell prepared at the respective facility of Merck KGaA. The test cells had inter-digital electrodes on one substrate side. The electrode width was 10 µm, the distance between adjacent electrodes was 10 µm and the cell gap was also 10 µm. This test cell has been evaluated electro-optically between crossed polarisers.

At low temperatures, the filled cells showed the typical texture of a chiral nematic mixture, with an optical transmission between crossed polarisers without applied voltage. Upon heating, at a first temperature (T₁) the mixtures turned optically isotropic, being dark between the crossed polarisers. This indicated the transition from the chiral nematic phase to the blue phase at that temperature. Up to a second temperature (T₂) the cell showed an electro-optical effect under applied voltage, typically of some tens of volts, a certain voltage in that range leading to a maximum of the optical transmission. Typically at a higher temperature the voltage needed for a visible electro-optical effect increased strongly, indicating the transition from the blue phase to the isotropic phase at this second temperature (T₂).

The temperature range (ΔT(BP)), where the mixture can be used electro-optically in the blue phase most beneficially has been identified as ranging from T₁ to T₂. This temperature range (ΔT(BP)) is the temperature range given in the examples of this application. The electro-optical displays can also be operated at temperatures beyond this range, i.e. at temperatures above T₂, albeit only at significantly increased operation voltages.

The liquid crystal media according to the present invention can contain further additives and chiral dopants in usual concentrations. The total concentration of these further constituents is in the range of 0 % to 10 %, preferably 0.1 % to 6 %, based in the total mixture. The concentrations of the individual compounds used each are preferably in the range of 0.1 to 3 %. The concentration of these and of similar additives is not taken into consideration for the values and ranges of the concentrations of the liquid crystal components and compounds of the liquid crystal media in this application.

The inventive liquid crystal media according to the present invention consist of several compounds, preferably of 3 to 30, more preferably of 5 to 20 and most preferably of 6 to 14 compounds. These compounds are mixed in conventional way. As a rule, the required amount of the compound used in the smaller amount is dissolved in the compound used in the greater amount. In case the temperature is above the clearing point of the compound used in the higher concentration, it is particularly easy to observe completion of the process of dissolution. It is, however, also possible to prepare the media by other conventional ways, e.g. using so called pre-mixtures, which can be e. g. homologous or eutectic mixtures of compounds or using so called multi-bottle-systems, the constituents of which are ready to use mixtures themselves.

By addition of suitable additives, the liquid crystal media according to the instant invention can be modified in such a way, that they are usable in all known types of liquid crystal displays, either using the liquid crystal media as such, like TN-, TN-AMD, ECB-, VAN-AMD and in particular in composite systems, like PDLD-, NCAP- and PN-LCDs and especially in HPDLCs.

The melting point: T(K,N), T(K,S) or T(K,I), respectively, the transition temperature from one smectic phase (Sₓ) to another smectic phase (S_{Y}): T(Sₓ,S_{Y}), the transition temperature from the smectic (S) to the nematic (N) phase: T(S,N), the clearing point: T (N,I), and the glass transition temperature: T_{g} of the liquid crystals, as applicable, as well as any other temperature throughout this application, are given in degrees centi-grade (i.e. Celsius).

The compounds of formula I wherein L¹¹ is H and L¹² is F, i.e. 6-fluorochromans, can be prepared according to the following scheme by nucleophilic ring opening of oxetanes with 4-bromo-2,5-difluorophenyllithium followed by cyclisation of an intermediate alkoholate *via* intramolecular substitution of fluorine. The obtained 6-fluoro-7-bromochromans can be further reacted, e.g. in a Suzuki coupling to give the target compounds.

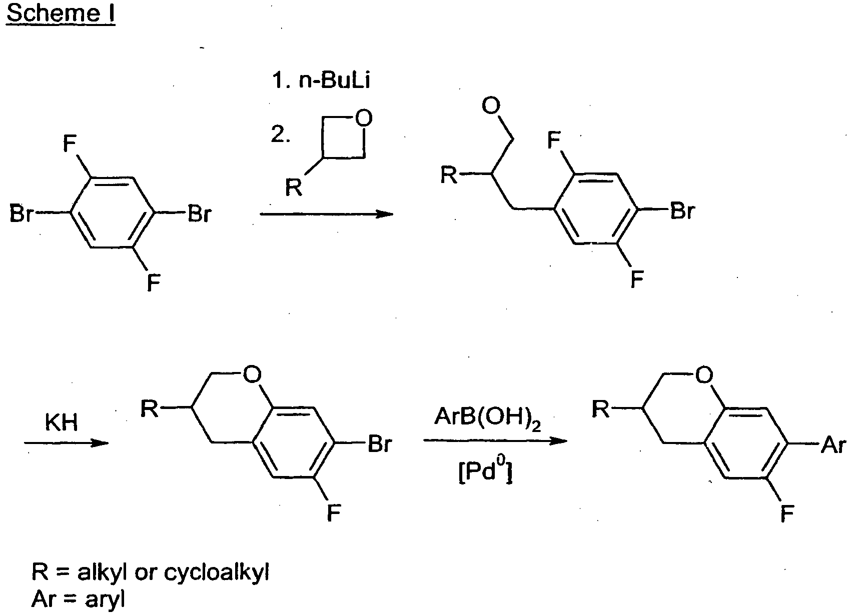

In a similar way the compounds of formula I wherein L¹¹ and L¹² both are F, i.e. 6,8-difluorochromans are obtained from 1-bromo-2,3,5-trifluorobenzene as shown in the following scheme.

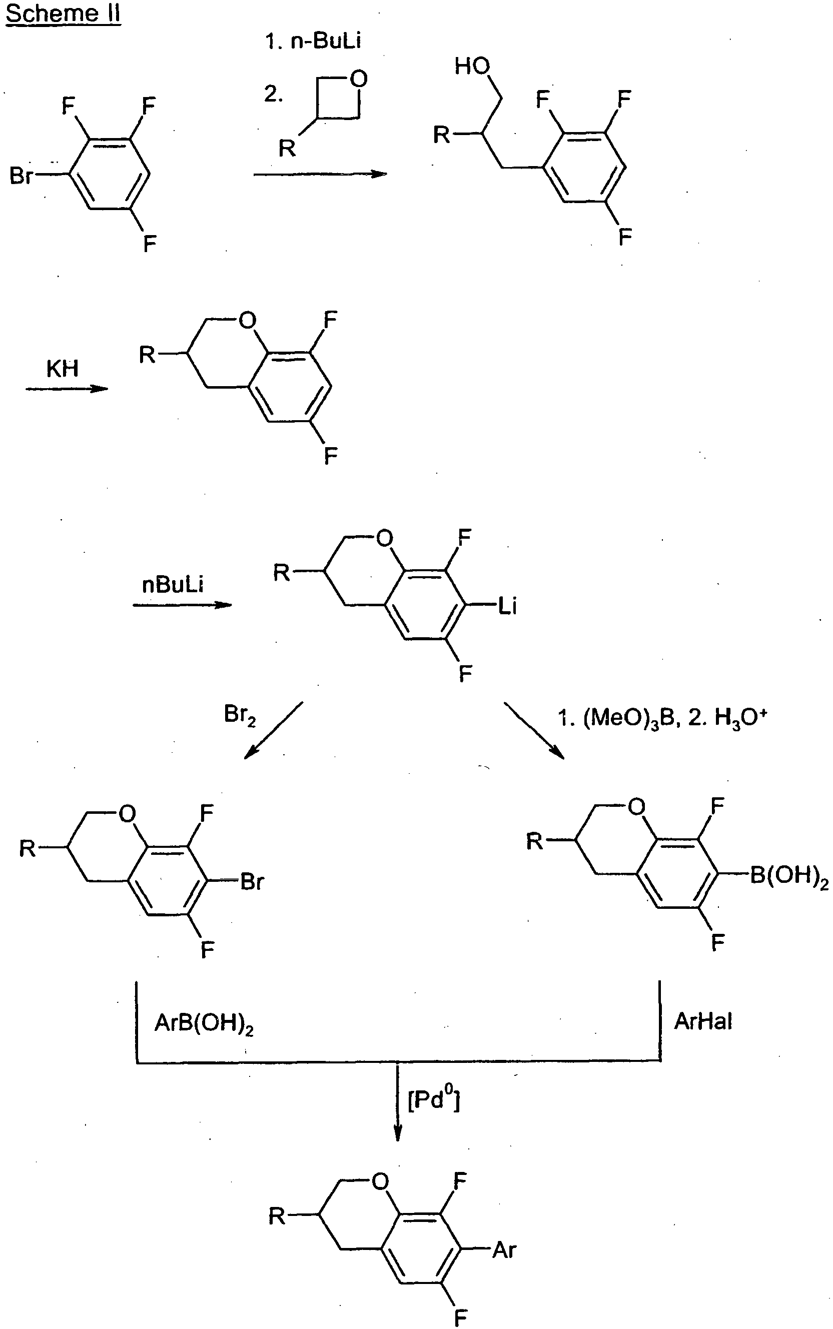

In the present application and especially in the following examples, the structures of the liquid crystal compounds are represented by abbreviations also called acronyms. The transformation of the abbreviations into the corresponding structures is straight forward according to the following two tables A and B. All groups CₙH₂ₙ₊₁ and CₘH₂ₘ₊₁ are straight chain alkyl groups with n respectively m C-atoms. The interpretation of table B is self evident. Table A does only list the abbreviations for the cores of the structures. The individual compounds are denoted by the abbreviation of the core followed by a hyphen and a code specifying the substituents R¹, R², L¹ and L² follows:

| Code for R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nN.F.F | CₙH₂ₙ₊₁ | CN | F | F |
| nF | CₙH₂ₙ₊₁ | F | H | H |
| nF.F | CₙH₂ₙ₊₁ | F | H | F |
| nF.F.F | CₙH₂ₙ₊₁ | F | F | F |
| nOF | OCₙH₂ₙ₊₁ | F | H | H |
| nCl | CₙH₂ₙ₊₁ | Cl | H | H |
| nCl.F | CₙH₂ₙ₊₁ | Cl | H | F |
| nCl.F.F | CₙH₂ₙ₊₁ | Cl | F | F |
| nCF₃ | CₙH₂ₙ₊₁ | CF₃ | H | H |
| nOCF₃ | CₙH₂ₙ₊₁ | OCF₃ | H | H |
| nOCF₃.F | CₙH₂ₙ₊₁ | OCF₃ | H | F |
| nOCF₃.F.F | CₙH₂ₙ₊₁ | OCF₃ | F | F |
| nOCF₂ | CₙH₂ₙ₊₁ | OCHF₂ | H | H |
| nOCF₂.F | CₙH₂ₙ₊₁ | OCHF₂ | H | F |
| nOCF₂.F.F | CₙH₂ₙ₊₁ | OCHF₂ | F | F |
| nS | CₙH₂ₙ₊₁ | NCS | H | H |
| nS.F | CₙH₂ₙ₊₁ | NCS | H | F |
| nS.F.F | CₙH₂ₙ₊₁ | NCS | F | F |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |
| nF.Cl | CₙH₂ₙ₊₁ | Cl | H | F |

**Table A:**

| | |
|---|---|
| 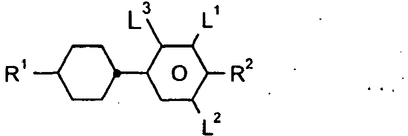 | 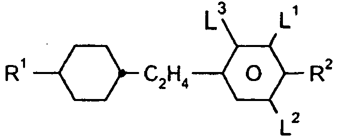 |
| **PCH** | **EPCH** |
| 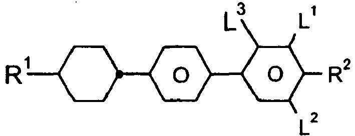 | 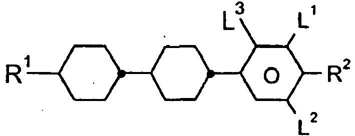 |
| **BCH** | **CCP** |
| 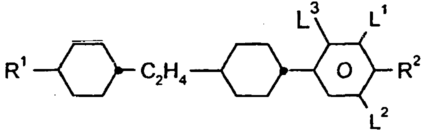 | 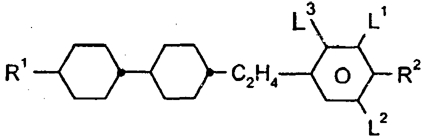 |
| **CECP** | **ECCP** |
| 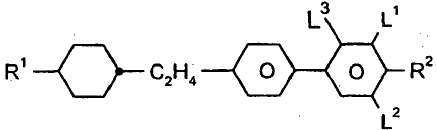 | 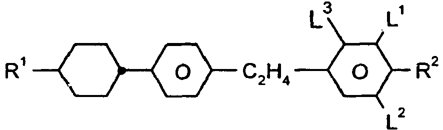 |
| **BECH** | **EBCH** |
| 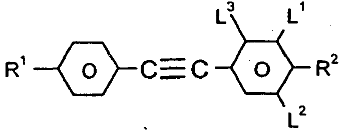 | 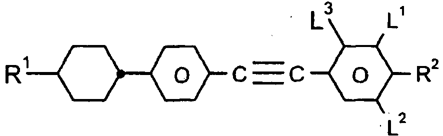 |
| **PTP** | **CPTP** |
| 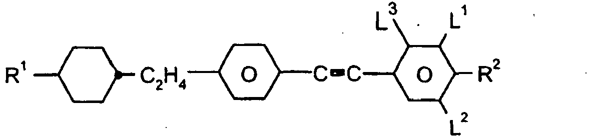 | |
| **CEPTP** | |
| 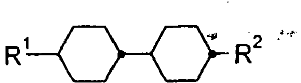 | 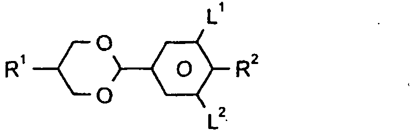 |
| **CCH** | **PDX** |
| 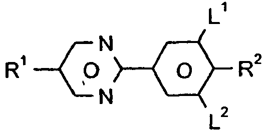 | 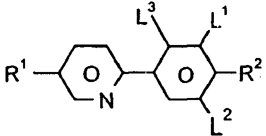 |
| **PYP** | **PYRP** |
| 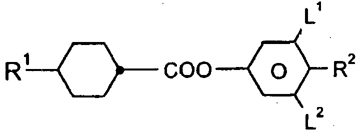 | 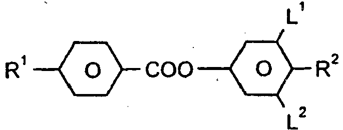 |
| **D** | **ME** |
| 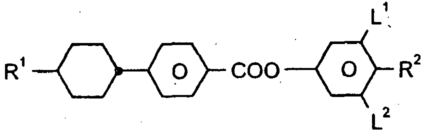 | 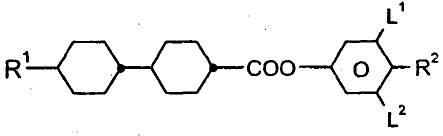 |
| **HP** | **CP** |
| 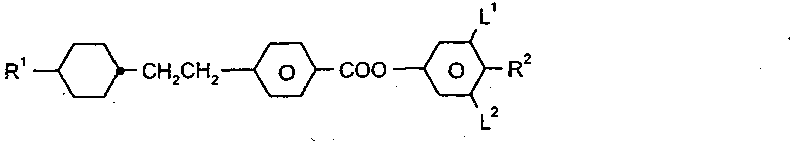 | |
| **EHP** | |
| 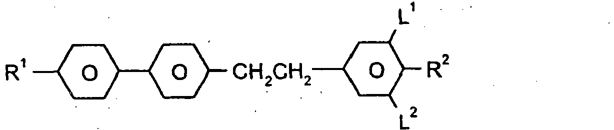 | |
| **ET** | |
| 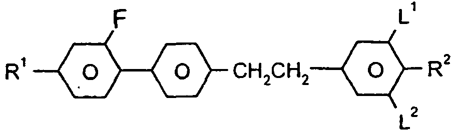 | |
| **FET** | |

**Table B:**

| | |
|---|---|
| 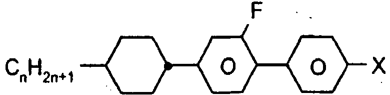 | 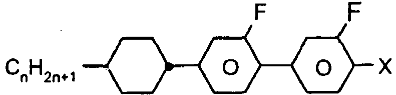 |
| **CGP-n-X** | **CGG-n-X** |
| (X = F, CF3, OCHF2 or OCF3) | (X = F, CF3, OCHF2 or OCF3) |
| 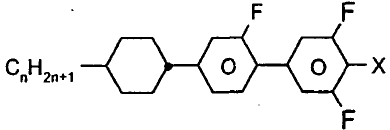 | 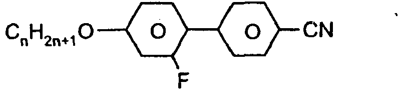 |
| **CGU-n-X** | **B-nO.FN** |
| (X = F, CF3, OCHF2 or OCF3) | |
| 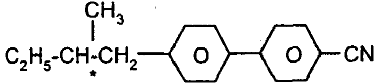 | 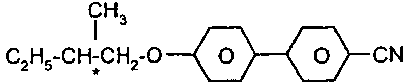 |
| **CB15** | **C15** |
| 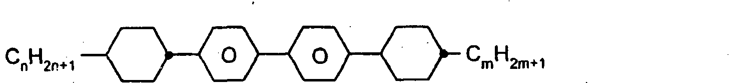 | |
| **CBC-nm** | |
| 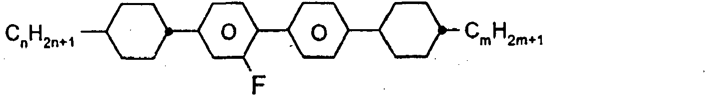 | |
| **CBC-nmF** | |
| 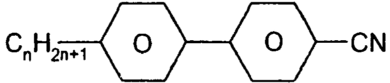 | 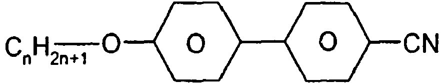 |
| **K3-n** | **M3·n** |
| 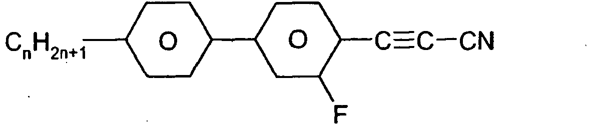 | |
| **PG-n-AN** | |
| 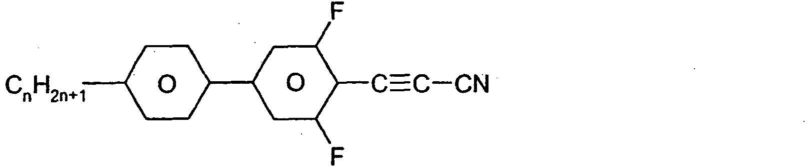 | |
| **PU-n-AN** | |
| 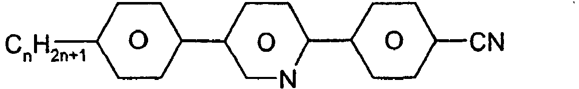 | |
| **PPYRP-nN** | |
| 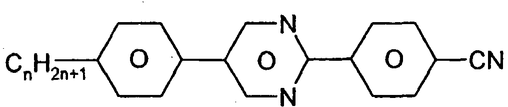 | |
| **PPYP-nN** | |
| 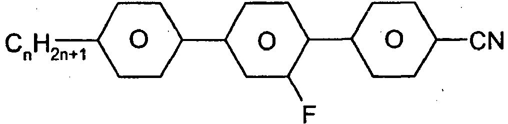 | |
| **PGP-n-N** | |
| 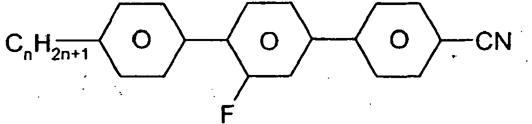 | |
| **PGIP-n-N** | |
| 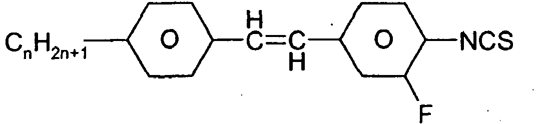 | |
| **PVG-n-S** | |
| 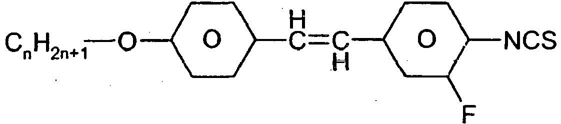 | |
| **PVG-nO-S** | |
| 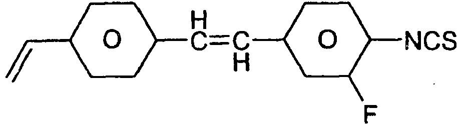 | |
| **PVG-V-S** | |
| 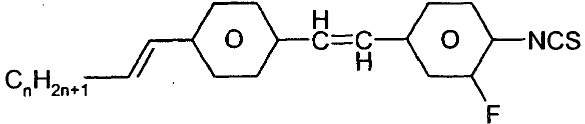 | |
| **PVG-nV-S** | |
| 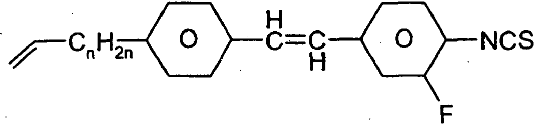 | |
| **PVG-Vn-S** | |
| 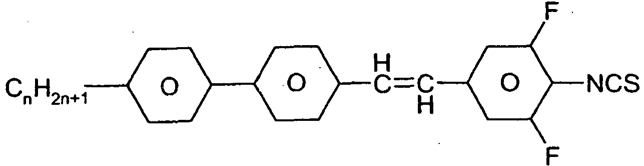 | |
| **PPVU-n-S** | |
| 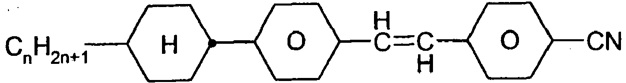 | |
| **CPVP-n-N** | |
| 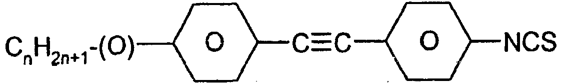 | |
| **PTP-n(0)-S** | |
| 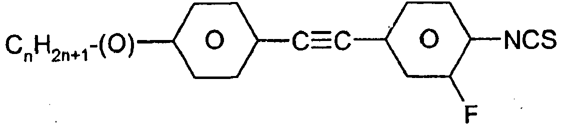 | |
| **PTG-n(0)-S** | |
| 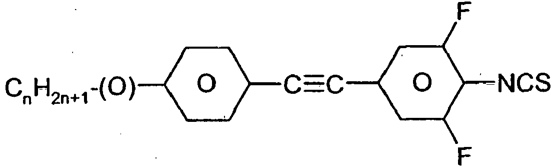 | |
| **PTU-n(0)-S** | |
| 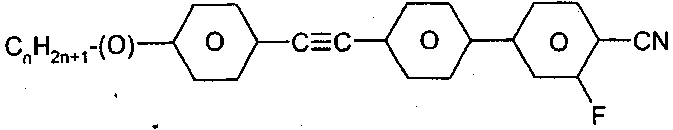 | |
| **PTPG-n(0)-N** | |
| 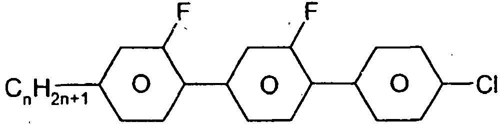 | |
| **GGP-n-CL** | |
| 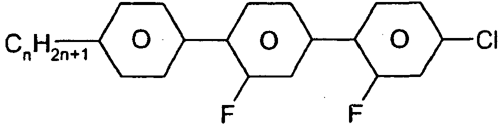 | |
| **PGIGI-n-CL** | |
| 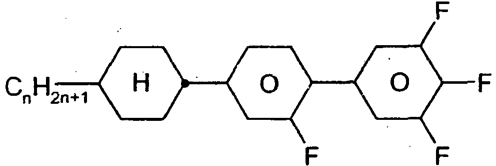 | |
| **CGU-n-F** | |
| 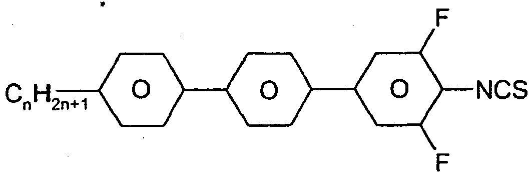 | |
| **PPU-n-S** | |
| 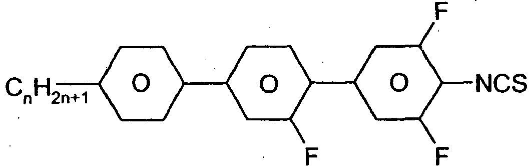 | |
| **PGU-n-S** | |
| 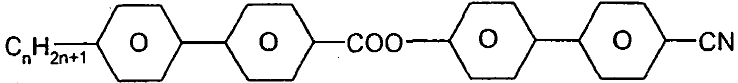 | |
| **BB3·n** | |
| 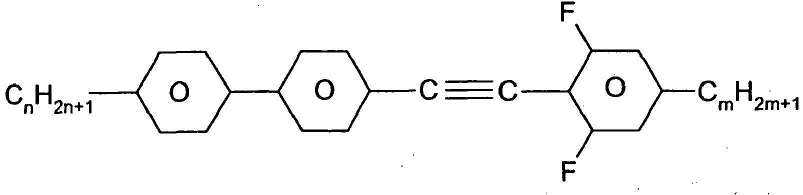 | |
| **PPTUI-n-m** | |
| 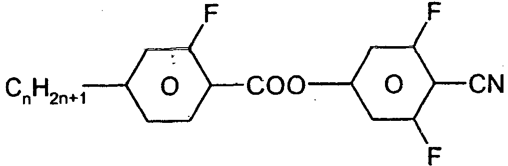 | |
| **GZU-n-N** | |
| 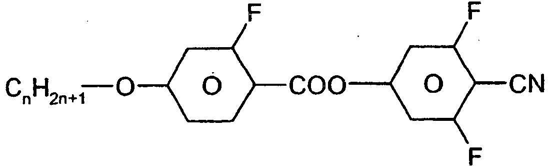 | |
| **GZU-nO-N** | |
| 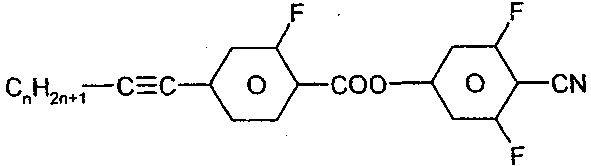 | |
| **GZU-nA-N** | |
| 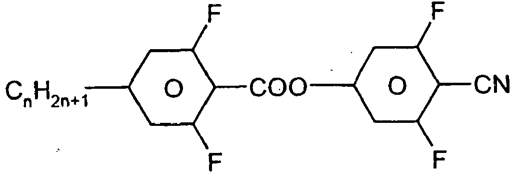 | |
| **UZU-n-N** | |
| 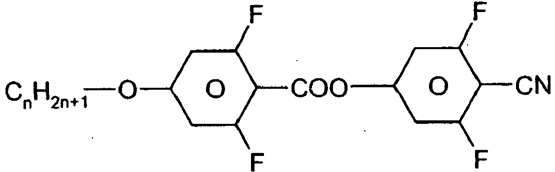 | |
| **UZU-nO-N** | |
| 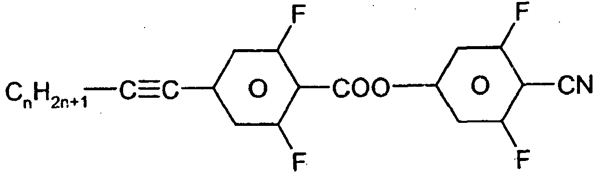 | |
| **UZU-nA-N** | |
| 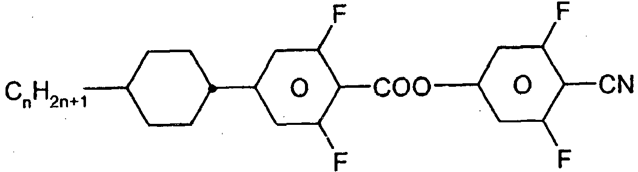 | |
| **CUZU-n-N** | |
| 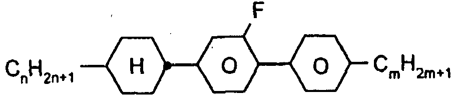 | |
| **BCH-n.Fm** | |
| 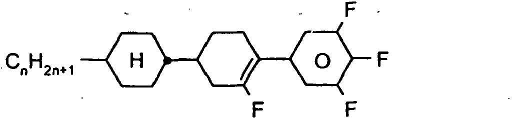 | |
| **CFU-n-F** | |
| 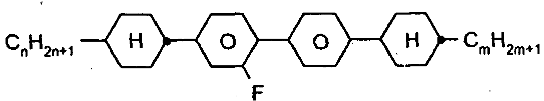 | |
| **CBC-nmF** | |
| 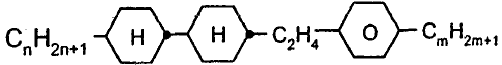 | |
| **ECCP-nm** | |
| 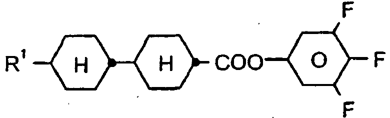 | |
| **CCZU-n-F** | |
| 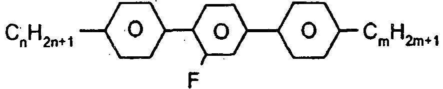 | |
| **T-nFm** | |
| 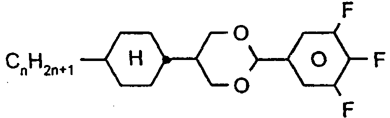 | |
| **CDU-n-F** | |
| 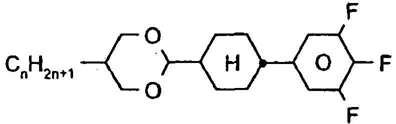 | |
| **DCU-n-F** | |
| 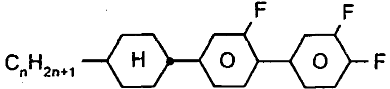 | |
| **CGG-n-F** | |
| 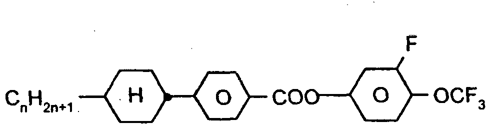 | |
| **CPZG-n-OT** | |
| 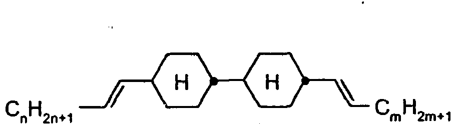 | |
| **CC-nV-Vm** | |
| 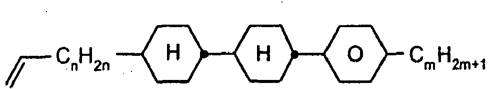 | |
| **CCP-Vn-m** | |
| 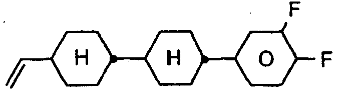 | |
| **CCG-V-F** | |
| 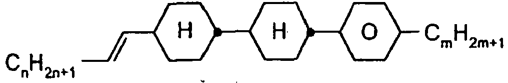 | |
| **CCP-nV-m** | |
| 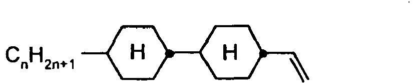 | |
| **CC-n-V** | |
| 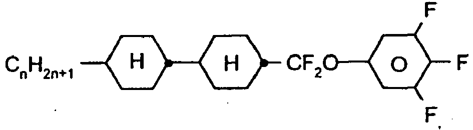 | |
| **CCQU-n-F** | |
| 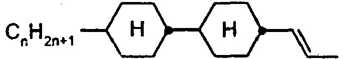 | |
| **CC-n-V1** | |
| 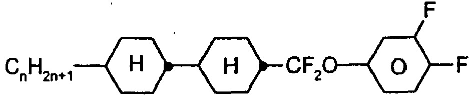 | |
| **CCQG-n-F** | |
| 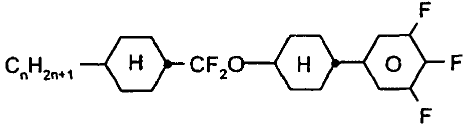 | |
| **CQCU-n-F** | |
| 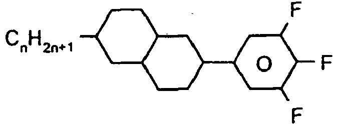 | |
| **Dec-U-n-F** | |
| 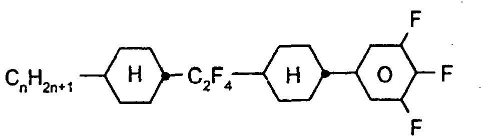 | |
| **CWCU-n-F** | |
| 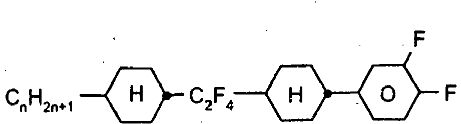 | |
| **CWCG-n-F** | |
| 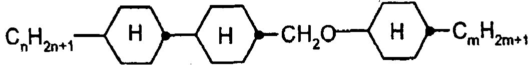 | |
| **CCOC-n-m** | |
| 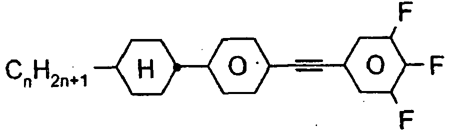 | |
| **CPTU-n-F** | |
| 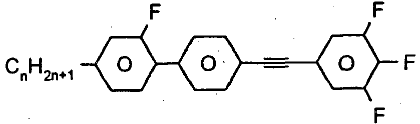 | |
| **GPTU-n-F** | |
| 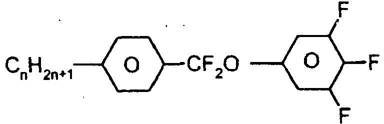 | |
| **PQU-n-F** | |
| 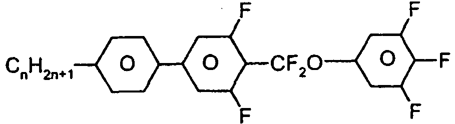 | |
| **PUQU-n-F** | |
| 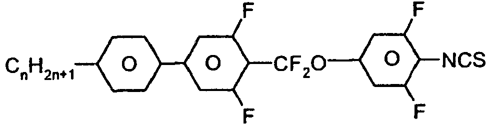 | |
| **PUQU-n-S** | |
| 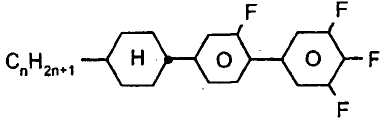 | |
| **CGU-n-F** | |
| 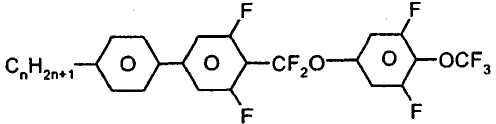 | |
| **PUQU-n-OT** | |
| 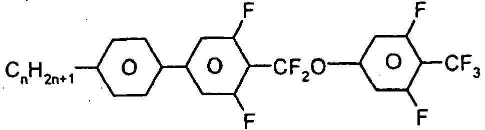 | |
| **PUQU-n-T** | |
| 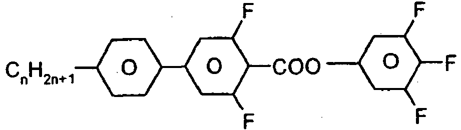 | |
| **PUZU-n-F** | |
| 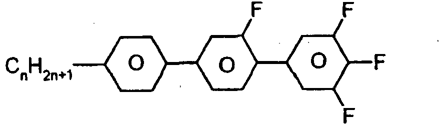 | |
| **PGU-n-F** | |
| 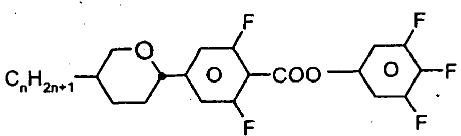 | |
| **AUZU-n-F** | |
| 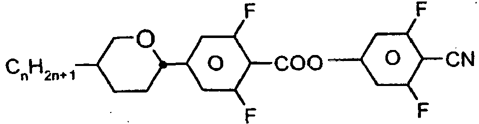 | |
| **AUZU-n-N** | |
| 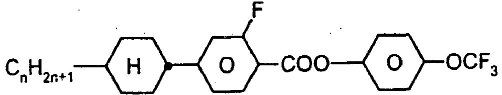 | |
| **CGZP-n-OT** | |
| 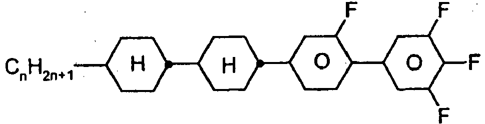 | |
| **CCGU-n-F** | |
| 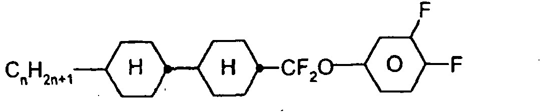 | |
| **CCQG-n-F** | |
| 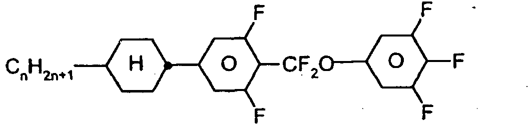 | |
| **CUQU-n-F** | |
| 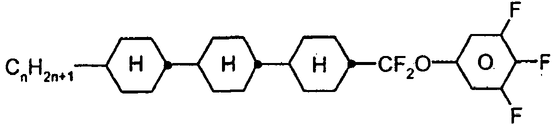 | |
| **CCCQU-n-F** | |
| 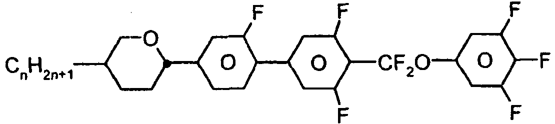 | |
| **AGUGaU-n-F** | |
| 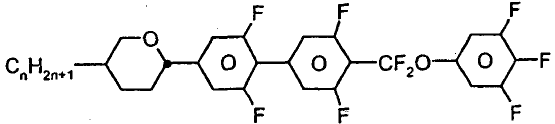 | |
| **AUUQU-n-F** | |
| 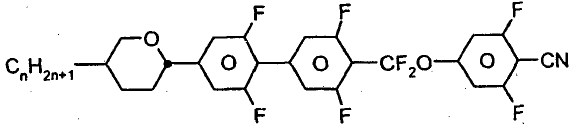 | |
| **AUUQU-n-N** | |
| 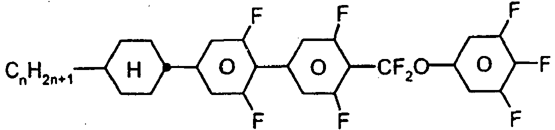 | |
| **CUUQU-n-F** | |
| 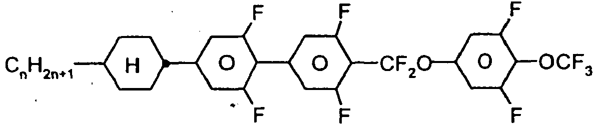 | |
| **CUUQU-n-OT** | |
| 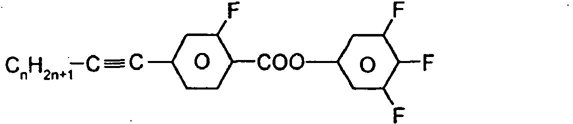 | |
| **GZU-nA-N** | |
| 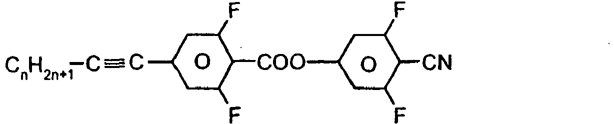 | |
| **UZU-nA-N** | |
| 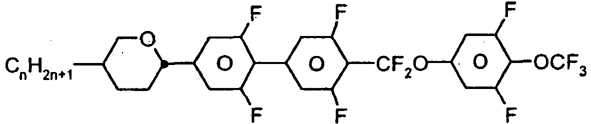 | |
| **AUUQU-n-QT** | |
| 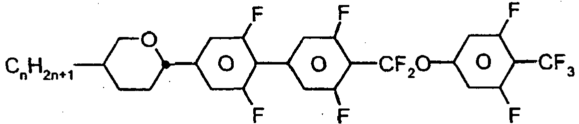 | |
| **AUUQU-n-T** | |
| 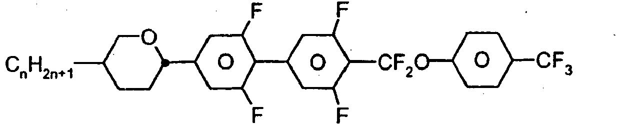 | |
| **AUUQP-n-T** | |
| 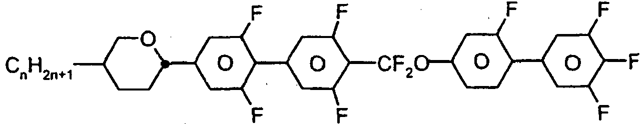 | |
| **AUUQGU-n-F** | |
| 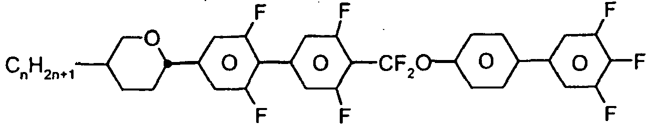 | |
| **AUUQPU-n-F** | |
| 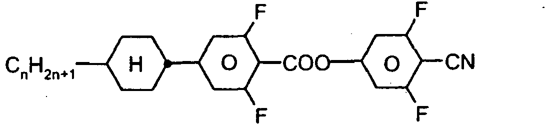 | |
| **CUZP-nN.F.F** | |
| 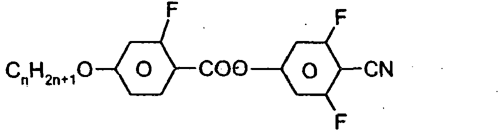 | |
| **GZU-nO-N** | |
| 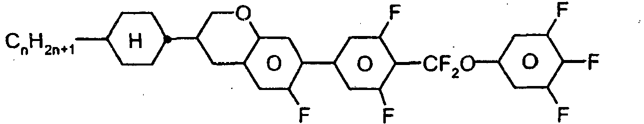 | |
| **CFUQU-n-F** | |
| 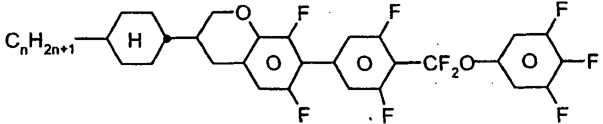 | |
| **CF₂UQU-n-F** | |
| 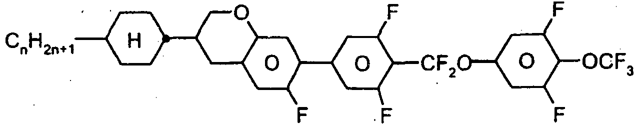 | |
| **CFUQU-n-OT** | |
| 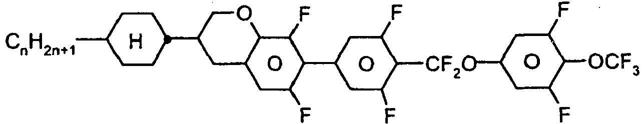 | |
| **CF₂UQU-n-OT** | |
| 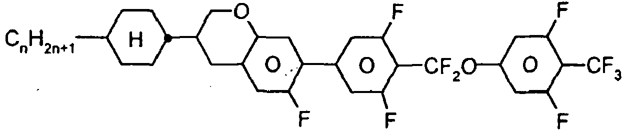 | |
| **CFUQU-n-T** | |
| 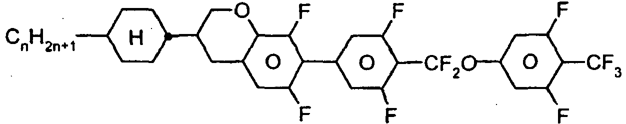 | |
| **CF₂UQU-n-T** | |
| 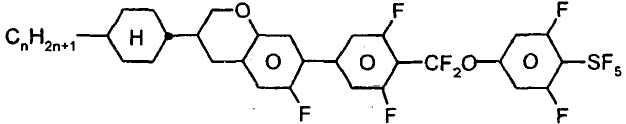 | |
| **CFUQU-n-SF5** | |
| 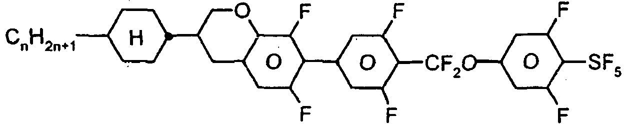 | |
| **CF₂UQU-n-SF5** | |

Particular preference is given to liquid-crystalline mixtures, which comprise at least one, two, three or four compounds from Table B.

**Table C:**

| Table C shows possible dopants according to component D, which are generally added to the mixtures alone or in combination two, three or more) according to the invention. |
|---|
| 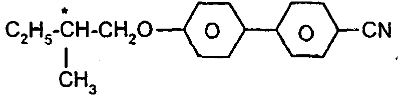 |
| **C15** |
| 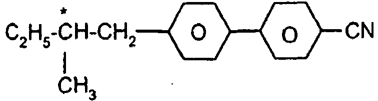 |
| **CB 15** |
| 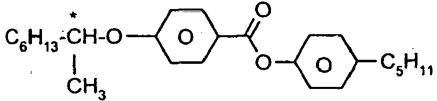 |
| **CM 21** |
| 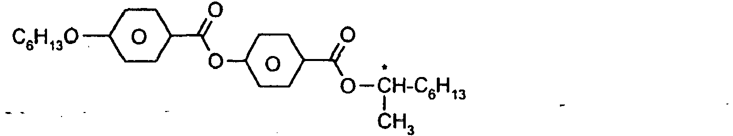 |
| **R/S-811** |
| 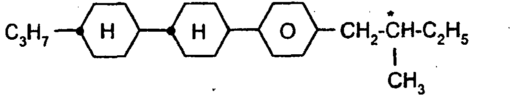 |
| **CM 44** |
| 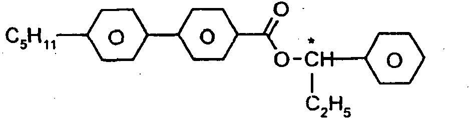 |
| **CM 45** |
| 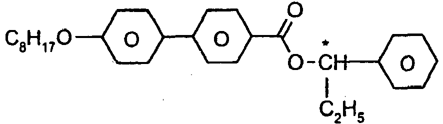 |
| **CM 47** |
| 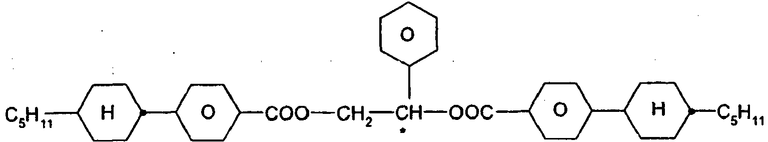 |
| **R/S-1011** |
| 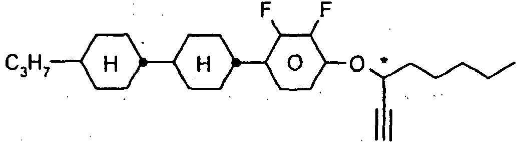 |
| **R/S-3011** |
| 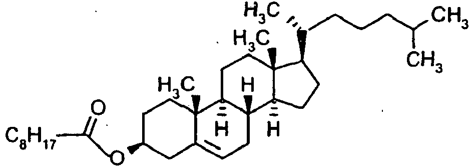 |
| **CN** |
| 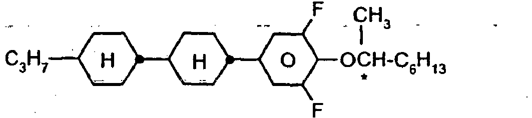 |
| **R/S-2011** |
| 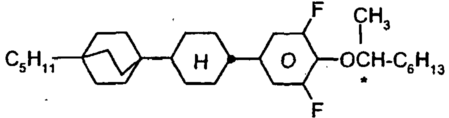 |
| **R/S-4011** |
| 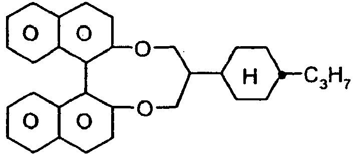 |
| **R/S-5011** |

**Table D**

| |
|---|
| Table D shows possible dopants according to component D, which are generally added to the mixtures alone or in combination two, three or more) according to the invention. |
| 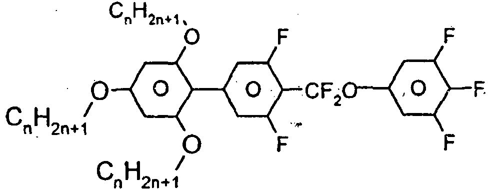 |
| **P(On)₂UQU-nO- F** |
| 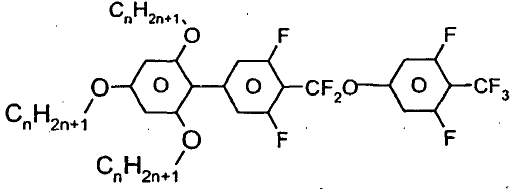 |
| **P(On)₂UQU-nO-T** |
| 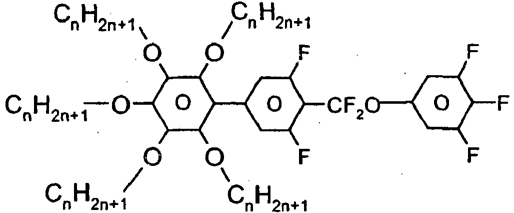 |
| **P(On)₄UQU-nO-FT** |
| 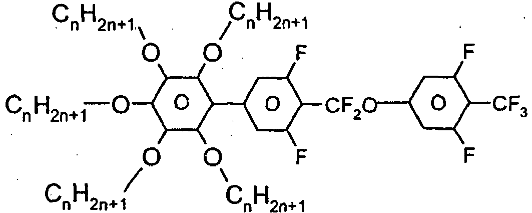 |
| **P(On)₄UQU-nO-T** |

The liquid crystal media according to the instant invention do contain preferably
- four or more compounds selected from the group of compounds of tables A and B and/or
- five or more compounds selected from the group of compounds of table B and/or
- two or more compounds selected from the group of compounds of table A.

### Examples

The examples given in the following are illustrating the present invention. The structure of the exemplary compounds is confirmed by ¹H and ¹³C NMR spectroscopy.

However, the physical data especially of the compounds illustrate to the expert, which properties can be achieved in which ranges. Especially the combination of the various properties, which can be preferably achieved, is thus well defined.

### Examples 1: Preparation of 7-{4-[Difluoro-(3,4,5-trifluorophenoxy)-methyl]-3,5-difluorophenyl}-6-fluoro-3-methylchroman

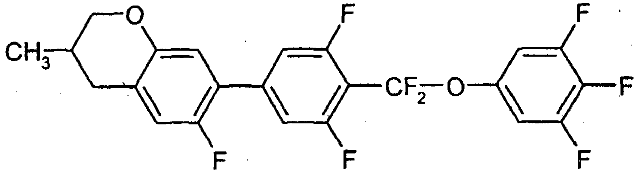

### Step 1.1

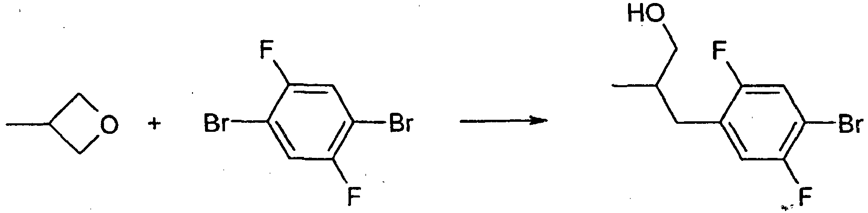

1,4-dibromo-2,5-difluorobenzene (172 g, 0.633 mol) in diethyl ether(1.7 I) is cooled to -70 °C and n-butyllithium (400 ml, 0.637 mol) is added slowly. The reaction mixture is stirred for 30 min and a solution of 3-methyloxetane (40 g, 0.555 mol) is added. After 2 h the cooling bath is removed, the reaction mixture is stirred overnight at ambient temperature and subsequently is quenched with saturated aquous ammonium chloride solution. The aqueous layer is extracted with MTB ether and the combined organic layers are washed with water and dried (Na₂SO₄). The solvent is evaporated and the crude product is filtered through silica with toluene/ ethyl acetate (4:1) to give 3-(4-bromo-2,5-difluoro-phenyl)-2-methyl-propan-1-ol as a colourless oil which is sufficiently pure for the next synthetic step.

### Step 1.2

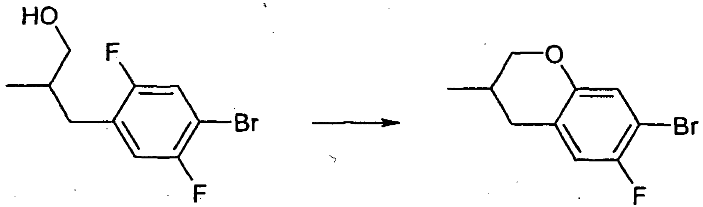

A solution of 3-(4-bromo-2,5-difluoro-phenyl)-2-methyl-propan-1-ol (72,3 g, 0,273 mol) in THF (300 ml) is added dropwise to a suspension of potassium hydride (30 % in mineral oil, 0.322 mol) in THF (2.2 I) at 40°C. The reaction is stirred at 55°C for 4 h, subsequently quenched with isopropanol (20 ml) and poured onto ice water. The aqueous layer is extracted with MTB ether, the combined organic layers are washed with water and dried (over Na₂SO₄). The solvent is evaporated and the residue is filtered through silica with toluene/heptane (1:1) and the crude product is recrystallised from heptane to give 7-bromo-6-fluoro-3-methyl-chroman as a colourless solid.

### Step 1.3

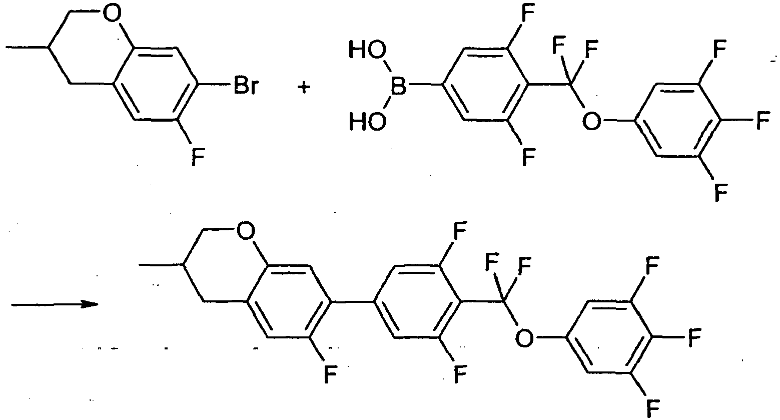

To a solution of 4-[difluoro-(3,4,5-trifluorophenoxy)-methyl]-3,5-difluorobenzene boronic acid (11.1 g, 28.5 mmol) in THF (60 ml), sodium metaborate octahydrate (5 g, 21.8 mmol) in water (12 ml) is added, followed by bis(triphenylpalladium(II)chloride (0.8 g, 1.14 mmol), hydrazinium hydroxide (0.3 ml) and a solution of 7-bromo-6-fluoro-3-methyl-chroman (6.0 g, 28.6 mmol) in THF (40 ml). The reaction mixture is heated under reflux for 5 h. Then, MTB-Ether is added and the solution is washed with water and dried (Na₂SO₄). The solvent is evaporated and the crude product is purified by chromatography on silica (eluent 1-chlorobutane/heptane 1:1) and recrystallised from toluene/heptane to give 7-{4-[Difluoro-(3,4,5-trifluoro-phenoxy)-methyl]-3,5-difluoro-phenyl}-6-fluoro-3-methyl-chroman as colourless crystals.

The product has a phase sequence of: K 119 I.

### Example 2a

Analogously to example 1 the compound of the following formula is prepared.

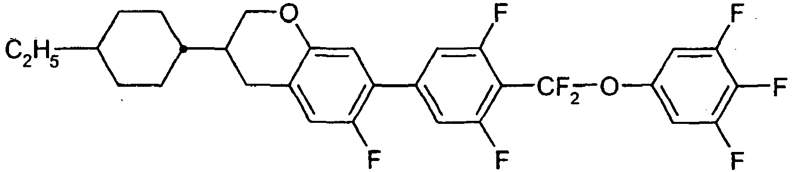

The product has a phase sequence of: K 109 N 161.5 I.

### Example 2b

Analogously to example 1 the compound of the following formula is prepared.

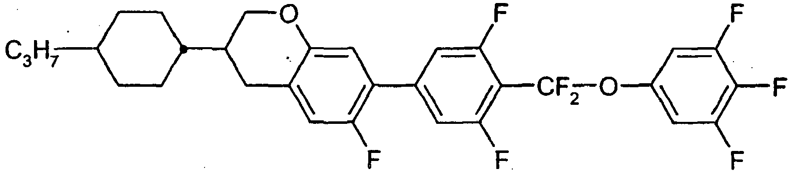

The product has a phase sequence of: K 100 N 180.1 I.

### Example 2c

Analogously to example 1 the compound of the following formula is prepared.

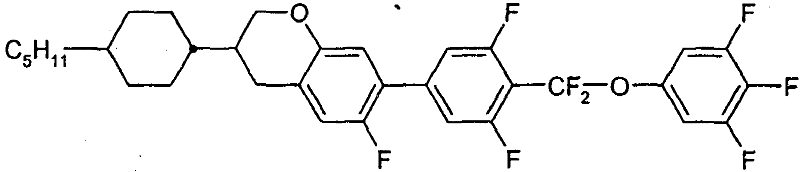

The product has a phase sequence of: K 81 S_{A} (75) N 177.0 I.

### Example 3a

Analogously to example 1 the compound of the following formula is prepared.

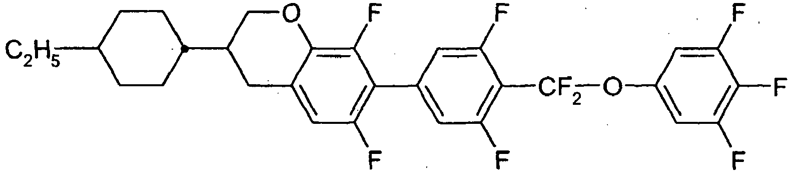

The product has a phase sequence of: K 110 N 145.7 I.

### Example 3b

Analogously to example 1 the compound of the following formula is prepared.

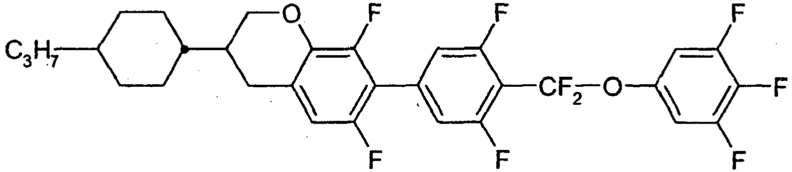

The product has a phase sequence of: K 106 N 166.6 I.

### Example 4a

Analogously to example. 1 the compound of the following formula is prepared.

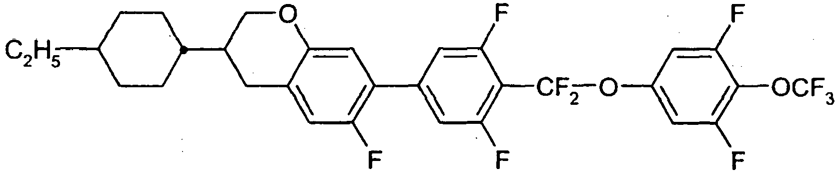

The product has a phase sequence of: K 104 S_{A} 125 N 173.7 I.

### Example 4b

Analogously to example 1 the compound of the following formula is prepared.

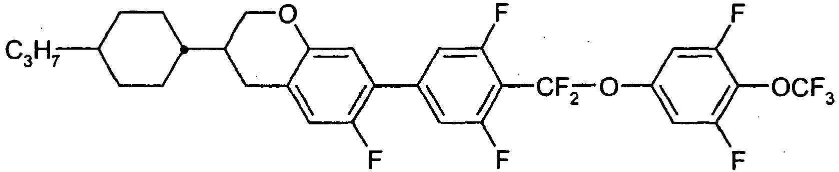

The product has a phase sequence of: K 95 S_{A} 141 N 190.0 I.

### Example 4c

Analogously to example 1 the compound of the following formula is prepared.

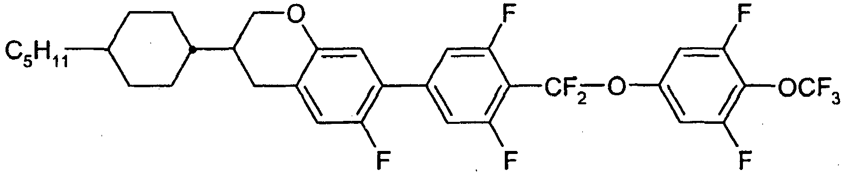

The product has a phase sequence of: K 89 S_{A} 150 N 186.6 I.

### Example 5a

Analogously to example 1 the compound of the following formula is prepared.

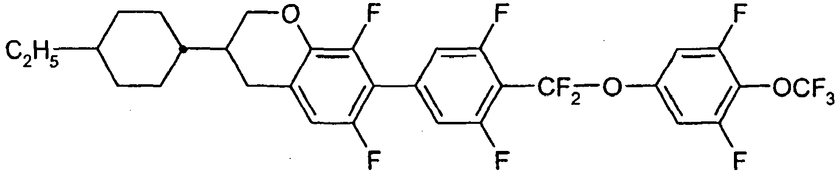

The product has a phase sequence of: K 98 S_{A} 110 N 157.8 I.

### Example 5b

Analogously to example 1 the compound of the following formula is prepared.

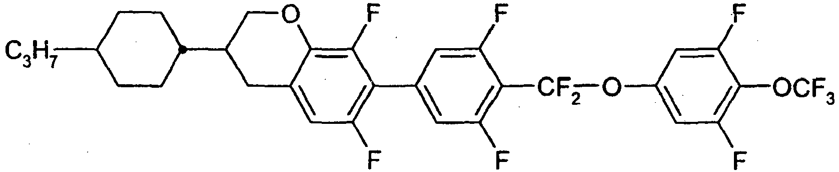

The product has a phase sequence of: K 88 S_{A} 124 N 176.8 I.

### Example 6a

Analogously to example 1 the compound of the following formula is prepared.

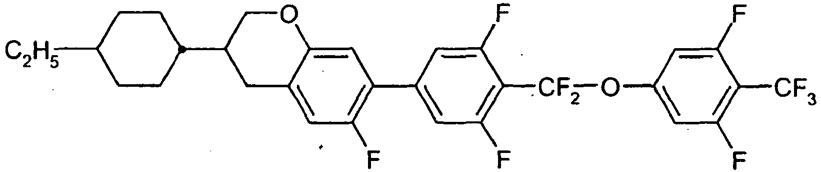

The product has a phase sequence of: K 122 N 152.3 I.

### Example 6b

Analogously to example 1 the compound of the following formula is prepared.

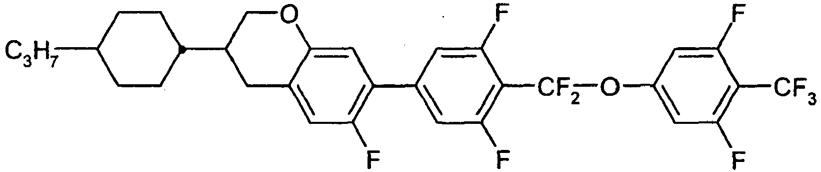

The product has a phase sequence of: K 107 S_{A} (83) N 169.4 I.

### Example 6c

Analogously to example 1 the compound of the following formula is prepared.

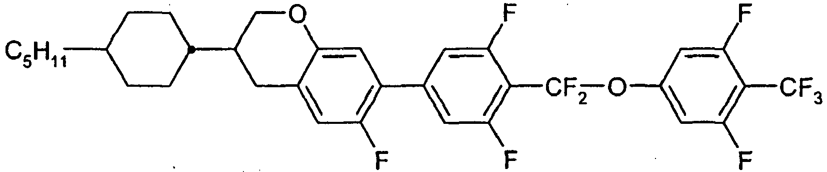

The product has a phase sequence of: K 83 S_{A} 104 N 167.7 I.

### Example 7

Analogously to example 1 the compound of the following formula is prepared.

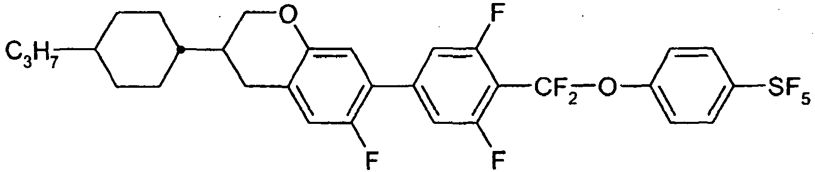

The product has a phase sequence of: K 88 S_{A} 136 N 181.5 I.

### Example 8a

Analogously to example 1 the compound of the following formula is prepared.

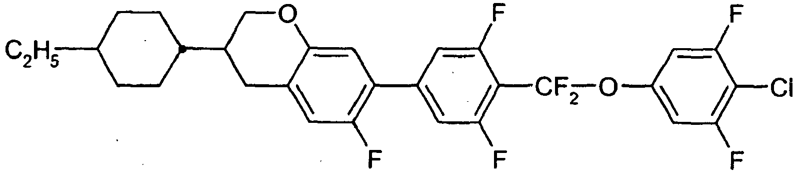

The product has a phase sequence of: K 134 N 180.7 I.

### Example 8b

Analogously to example 1 the compound of the following formula is prepared.

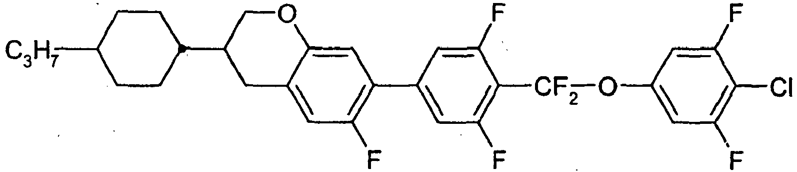

The product has a phase sequence of: K 110 N 197.8 I.

### Example 8c

Analogously to example 1 the compound of the following formula is prepared.

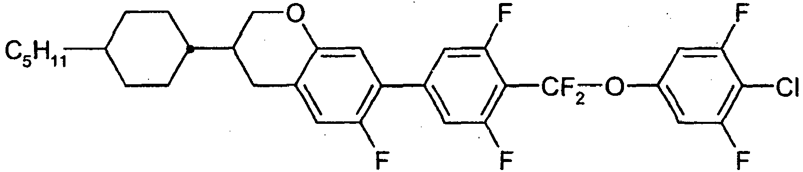

The product has a phase sequence of: K 106 S_{A} (104) N 193.8 I.

### Example 9a

Analogously to example 1 the compound of the following formula is prepared.

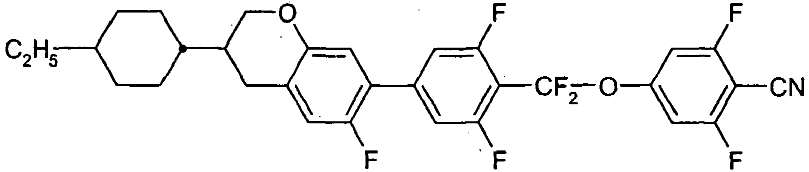

The product has a phase sequence of: K 145 N 204.1 I.

### Example 9b

Analogously to example 1 the compound of the following formula is prepared.

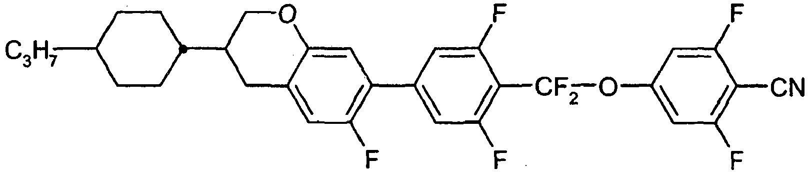

The product has a phase sequence of: K 142 N 215.2 I.

### Example 9c

Analogously to example 1 the compound of the following formula is prepared.

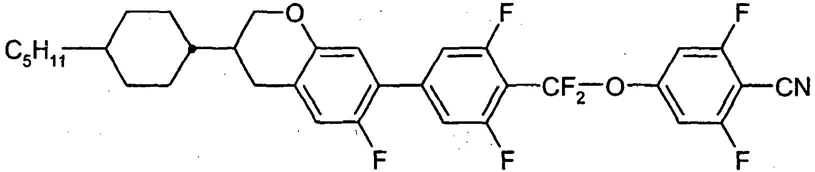

The product has a phase sequence of: K 132 N 208.9 I.

### Comparative Use-example 1

The following liquid crystalline mixture (C-1) is prepared and investigated with respect to its general physical properties. The composition and properties are given in the following table, Table 1.

**Table 1: Composition and properties liquid crystal mixture C-1**

| Composition | | | Physical Properties | |
|---|---|---|---|---|
| Compound | | Concentration / mass-% | | |
| No. | Abbreviation | | T(N, I) | =55.0 °C |
| 1 | CC-5-V | 10.0 | | |
| 2 | PCH-53 | 30.0 | nₑ(20°C, 589 nm) | =1.5545 |
| 3 | CCP-2F.F.F | 15.0 | Δn(20°C, 589 nm) | =0.0708 |
| 4 | CCP-3F.F.F | 15.0 | | |
| 5 | CCP-5F.F.F | 10.0 | ε_{∥}(20°C, 1 kHz) | =6.8 |
| 6 | CCP-31 | 10.0 | Δε(20°C, 1 kHz) | =3.8 |
| 7 | CCG-V-F | 10.0 | | |
| Σ | | 100.0 | | |

Next the voltage holding ratio (VHR) of the mixture is determined in test cells of the TN-type with a cell gap of approximately 5 µm, an orientation layer AL-3046 from Japan Synthetic Rubber, Japan and an electrode area of 1 cm² using a VHRM-105 of Autronic Melchers, Karlsruhe, Germany. The measurement voltage is 1 V. The VHR of the mixture in the test cells is determined immediately at a temperature of 20 °C and subsequently at a temperature of 100 °C, 5 minutes after insertion into a preheated oven. Next the test cells are exposed to UV irradiation at a temperature of 20 °C in a Suntest equipment of Heraeus, Germany with a dose/ energy 75 mW/cm² of over a time of 2 h. Subsequently the VHR is determined again at a temperature of 20 °C and after 5 minutes at 100 °C. The results are shown in table 2 below.

**Table 2: Results of VHR and e-o**

| Use-Ex. # | | C.E.-1 | C.E.-2 | C.E.-3 | C.E.-4 | C.E.-5 |
|---|---|---|---|---|---|---|
| Mixture # | | C-1 | C-2 | C-3 | C-4 | C-5 |
| | | | | | | |
| T(N,I) / °C | | 55.0 | 74.5 | 56.5 | 91.0 | 75.0 |
| | | | | | | |

| t(UVexp.)/min | T/°C | Voltage Holding Ratio / % | | | | |
|---|---|---|---|---|---|---|
| 0 | 20 | 99.8 | 99.6 | 76.4 | 42 | 84.2 |
| 0 | 100 | 99.4 | 96.8 | 16 | 16 | 30 |
| 120 | 20 | 99.5 | 82.3 | 21 | 29 | 17 |
| 120 | 100 | 94.7 | 35 | 26 | 13 | 13 |
| | | | | | | |
| c(R-5011)/ / % | | 5.0 | | | | |

| Characteristic Temperatures | | | | | | |
|---|---|---|---|---|---|---|
| T₂°/°C | | n.d. | n.d. | 43.0 | n.d. | 48.5 |
| T₃°/°C | | n.d. | n.d. | 37.5 | n.d. | 45.0 |
| T₁°/°C | | n.d. | n.d. | 36.0 | n.d. | 42.5 |
| ΔT(BP)°/° | | n.d. | n.d. | 7.0 | n.d. | 6.0 |
| ΔT(FR)°/° | | n.d. | n.d. | 5.5 | n.d. | 3.5 |

| Characteristic Voltages | | | | | | |
|---|---|---|---|---|---|---|
| T_{op.} °/°C | | n.d. | n.d. | 38.0 | n.d. | 44.5 |
| Vₘₐₓ/V | | n.d. | n.d. | 46.0 | n.d. | 56.5 |
| T_{op.} °/°C. | | n.d. | n.d. | 40.0 | n.d. | 46.5 |
| Vₘₐₓ/V | | n.d. | n.d. | 51.5 | n.d. | 70.5 |

| | | | | | | |
|---|---|---|---|---|---|---|
| Remarks: n.d.: not determined. | | | | | | |

5% of the chiral agent R-5011 are solved in the achiral liquid crystal mixture C-1 and the electro-optical response of resultant mixture in an IPS-type cell is investigated. The mixture is filled into an electro optical test cell with inter-digital electrodes on one substrate side. The electrode width is 10 µm, the distance between adjacent electrodes is 10 µm and the cell gap is also 10 µm. This test cell is evaluated electro-optically between grossed polarisers. The mixtures C-1 and C-2 can not be reasonably addressed with the voltages available.

The mixture of the comparative example 1 (C-1) has an excellently high voltage holding ratio, even after exposure to UV-radiation. However, it has a very low dielectric anisotropy only and, thus, can not be dressed in a device operating in the blue phase.

### Comparative Use-example 2

The following liquid crystalline mixture C-2, which has a significantly higher dielectric anisotropy than C-1, is prepared and investigated with respect to its general physical properties. The composition and properties are given in the following table, Table 3.

**Table 3: Composition and properties liquid crystal mixture C-2**

| Composition | | | Physical Properties | |
|---|---|---|---|---|
| Compound | | Concentration / mass-% | | |
| No. | Abbreviation | | T(N, I) | = 74.5 °C |
| 1 | CCP-2F.F.F | 12.0 | | |
| 2 | CCP-3F.F.F | 13.0 | nₑ(20°C, 589 nm) | = 1.5708 |
| 3 | CCP-5F.F.F | 8.0 | Δn(20°C,589nm) | = 0.0930 |
| 4 | CCP-2OCF₃ | 10.0 | | |
| 5 | CCP-3OCF₃ | 8.0 | ε_{∥}(20°C, 1 kHz) | = 14.6 |
| 6 | CCP-4OCF₃ | 7.0 | Δε(20°C, 1 kHz) | = 10.5 |
| 7 | CCP-5OCF₃ | 8.0 | | |
| 8 | CGU-2-F | 12.0 | | |
| 9 | CGU-3-F | 12.0 | | |
| 10 | CGU-5-F | 10.0 | | |
| Σ | | 100.0 | | |

This mixture C-2 is investigated for its VHR and, after addition of 5% of the chiral agent R-5011, for it's electro-optical response like mixture C-1 in comparative example 1. The results are shown in table 2, too.

### Comparative Use-example 3

The following liquid crystalline mixture C-3, which again has a significantly higher dielectric anisotropy than C-2, is prepared and investigated with respect to its general physical properties. The composition and properties are given in the following table, Table 4.

**Table 4: Composition and properties liquid crystal mixture C-3**

| Composition | | | Physical Properties | |
|---|---|---|---|---|
| Compound | | Concentration / mass-% | | |
| No. | Abbreviation | | T(N, I) | = 56.5 °C |
| 1 | GZU-3A-N | 15.0 | | |
| 2 | GZU-4A-N | 15.0 | nₑ(20°C, 589 nm) | = 1.6663 |
| 3 | GZU-4O-N | 15.0 | Δn(20°C, 589 nm) | = 0.1637 |
| 4 | UZU-3A-N | 8.0 | | |
| 5 | CUZU-2-N | 9.0 | | |
| 6 | CUZU-3-N | 9.0 | | |
| 7 | CUZU-4-N | 9.0 | | |
| 8 | HP-3N.F | 6.0 | | |
| 9 | HP-4N.F | 6.0 | | |
| 10 | HP-5N.F | 8.0 | | |
| Σ | | 100.0 | | |

The mixture C-3 is investigated for its VHR and, after addition of 5% of the chiral agent R-5011, for its electro-optical response like mixture V-1 in comparative example 1. The results are shown in table 2, too.

The voltage holding ratio values of this mixture (C-3) are significantly lower even than the respective values of the mixture C-2 at all conditions.

At low temperatures, the filled e-o cells show the typical texture of a chiral nematic mixture, with an optical transmission between crossed polarisers without applied voltage. On heating, at a temperature of 36°C the mixture becomes isotropic, being dark between the crossed polarisers. This indicates the transition from the chiral nematic phase to the blue phase at 36°C. This temperature is called T₁ or Tₜᵣₐₙₛ.

Up to a temperature of 43°C the cell shows a clear electro optical effect under applied voltage, for example at 38°C, applying a voltage of 46 V leads to a maximum of the optical transition. This temperature is called T₂ and the respective voltage is called Vₘₐₓ or V₁₀₀ at T₂. At a temperature of 43°C the voltage needed for a visible electro-optical effect starts to increase strongly, indicating the transition from the blue phase to the isotropic phase at this temperature.

The voltage at which X % of the optical transmission is reached is called V_{X} (e.g.: V₉₀. for 90 % transmission).

The temperature range (ΔT(BP)), where the mixture can be used electro-optically in the blue phase is identified as ranging from about 36°C to about 43°C, i.e. as being 7° wide (= T₂ - T₁ = 43°C - 36°C). The results are listed in table 2 below. Further the response times for switching on (τₒₙ) and for switching off (τ_{off}) are been determined. The response times decrease with increasing temperature above T₁ and the temperature at which both response times have fallen below 5 ms each is called T₃. This is the case in this comparative use example at a temperature of about 39.3°C or slightly above. Thus, the range of usable flat behaviour i.e. the usable flat range (ΔT(FR)), which is defined as ΔT(FR) = T₂ - T₃, in case T₂ ≥ T₃ and ΔT(FR) = 0, in case T₂ < T₃, is (43.0°C-39.3°C) = 3.7° in this comparative use example.

### Comparative Use-example 4

The following liquid crystalline mixture C-4, which again has a similar dielectric anisotropy like C-3, is prepared and investigated with respect to its general physical properties. The composition and properties are given in the following table, Table 5.

**Table 5: Composition and properties liquid crystal mixture C-4**

| Composition | | | Physical Properties | |
|---|---|---|---|---|
| Compound | | Concentration / mass-% | | |
| No. | Abbreviation | | T(N, I) | = 91.0 °C |
| 1 | AUUQU-3-N | 11.6 | | |
| 2 | AUZU-3-N | 11.6 | | |
| 3 | AUZU-5-N | 11.6 | | |
| 4 | GZU-3A-N | 9.3 | | |
| 5 | HP-3N.F | 7.0 | | |
| 6 | AUUQU-3-OT | 11.6 | | |
| 7 | AUUQU-3-T | 9.3 | | |
| 8 | AUUQU-3-F | 10.5 | | |
| 9 | AUUQGU-3-F | 9.3 | | |
| 10 | PUZU-3-F | 8.2 | | |
| Σ | | 100.0 | | |

The mixture C-4 is investigated for its VHR and, after addition of 5% of the chiral agent R-5011, for its electro-optical response like mixture C-1 in comparative example 1. The results are shown in table 2, too.

The voltage holding ratio values of this mixture (C-4) are dramatically lower even than the respective values of the mixture C-2 at all conditions.

### Comparative Use-example 5

The following liquid crystalline mixture C-5, which again has a similar dielectric anisotropy like C-3, is prepared and investigated with respect to its general physical properties. The composition and properties are given in the following table, Table 6.

**Table 6: Composition and properties liquid crystal mixture C-5**

| Composition | | | Physical Properties | |
|---|---|---|---|---|
| Compound | | Concentration / mass-% | | |
| No. | Abbreviation | | T(N, I) | = 75.0 °C |
| 1 | AUUQU-2-F | 11.0 | | |
| 2 | AUUQU-3-F | 13.0 | | |
| 3 | AUUQU-4-F | 6.0 | | |
| 4 | AUUQU-5-F | 5.5 | | |
| 5 | AUUQU-7-F | 6.0 | | |
| 6 | AUUQU-3-T | 11.0 | | |
| 7 | AUUQU-3-OT | 13.0 | | |
| 8 | AUUQGU-3-F | 7.0 | | |
| 9 | PUZU-2-F | 5.5 | | |
| 10 | PUZU-3-F | 11.0 | | |
| 11 | PUZU-5-F | 11.0 | | |
| Σ | | 100.0 | | |

The mixture C-5 is investigated for its VHR and, after addition of 5% of the chiral agent R-5011, for its electro-optical response like mixture C-1 in comparative example 1. The results are shown in table 2, too.

The voltage holding ratio values of this mixture (V-5) at 20 °C before irradiation with UV are reasonably good, however, the values are dramatically lower even than the respective values of the mixture C-2 at all other conditions.

The electro-optical properties of this mixture are comparable to those of mixture C-3, however, both the operation voltage and its temperature dependence are inferior to those of mixture C-3.

### Use-example 1

The following liquid crystalline mixture E-1, which again has a similar dielectric anisotropy like C-3, is prepared and investigated with respect to its general physical properties. The composition and properties are given in the following table, table 7.

**Table 7: Composition and properties liquid crystal mixture E-1**

| Composition | | | Physical Properties | |
|---|---|---|---|---|
| Compound | | Concentration / mass-% | | |
| No. | Abbreviation | | T(N, I) | = 79.5 °C |
| 1 | AUUQU-2-F | 8.0 | | |
| 2 | AUUQU-3-F | 9.0 | | |
| 3 | AUUQU-4-F | 5.0 | | |
| 4 | CFUQU-2-T | 9.0 | | |
| 5 | CFUQU-3-T | 9.0 | | |
| 6 | CFUQU-5-T | 8.0 | | |
| 7 | CFUQU-2-OT | 9.0 | | |
| 8 | ME-2N.F | 9.0 | | |
| 9 | ME-3N.F | 10.0 | | |
| 10 | ME-5N.F | 9.0 | | |
| 11 | GZU-3A-N | 8.0 | | |
| 12 | AUUQU-3-N | 7.0 | | |
| Σ | | 100.0 | | |

The mixture E-1 is investigated for its VHR and, after addition of 5% of the chiral agent R-5011, for its electro-optical response like mixture C-1 in comparative example 1. The results are shown in table 8.
The voltage holding ratio values of this mixture, E-1, at 20 °C both before and after irradiation with UV are comparatively good, and the values after exposure to UV are significantly better than e.g. the respective values of the mixtures C-3 and C-5.

The electro-optical properties of this mixture are comparable to those of mixture C-3, however, both the operation voltage and its temperature dependence are slightly inferior to those of mixture C-3.

**Table 8: Results of VHR and e-o**

| Use-Ex. # | | 1 | 2 | 3 | 4 | 5 |
|---|---|---|---|---|---|---|
| Mixture # | | E-1 | E-2 | E-3 | E-4 | E-5 |
| | | | | | | |
| T(N,I) /°C | | 79.5 | 91.0 | 102.5 | 100.0 | 108.0 |
| | | | | | | |

| t(UVexp.)/min | T / °C | Voltage Holding Ratio / % | | | | |
|---|---|---|---|---|---|---|
| 0 | 20 | 65.0 | 80.4 | n.d. | n.d. | n.d. |
| 0 | 100 | 30 | 78.2 | n.d. | n.d. | n.d. |
| 120 | 20 | 55.2 | 79.2 | n.d. | n.d. | n.d. |
| 120 | 100 | 13 | 50 | n.d. | n.d. | n.d. |
| | | | | | | |
| c(R-5011)/ / % | | 5.0 | | | 9.0 | |
| c(P(O₃)₄UQU-O3-F) | | 0 | | | 5.0 | |

| Characteristic Temperatures | | | | | | |
|---|---|---|---|---|---|---|
| T₂ /°C | | 66.0 | 75.5 | n.d. | 51.5 | 60.0 |
| T₃ /°C | | 61.0 | 70.0 | n.d. | 34.5 | 39.0 |
| T₁/°C | | 58.5 | 67.5 | n.d. | 23.0 | 32.5 |
| ΔT(BP) /° | | 7.5 | 8.0 | n.d. | 28.5 | 27.5 |
| ΔT(FR) /° | | 5.0 | 5.5 | n.d. | 17.0 | 21.0 |

| Characteristic Voltages | | | | | | |
|---|---|---|---|---|---|---|
| Tₒₚ. °/°C | | 60.5 | 69.5 | n.d. | 25.0 | 34.5 |
| V₉₀/V | | 41 | 58 | n.d. | 42.5 | 53 |
| Vₘₐₓ/N | | 49 | 70 | n.d. | 49 | 62 |
| T_{op.} /°C | | 62.5 | 71.5 | n.d. | 27.0 | 36.5 |
| V₉₀/V | | 52 | 68 | n.d. | 46 | 59 |
| Vₘₐₓ/V | | 60 | 78 | n.d. | 54 | 68 |

| | | | | | | |
|---|---|---|---|---|---|---|
| Remarks: n.d.: not determined. | | | | | | |

### Use-example 2

In this use-example the liquid crystalline mixture E-2 is prepared and investigated. The mixture E-2 has the respective composition and properties shown in table 9.

**Table 9: Composition and properties liquid crystal mixture E-2**

| Composition | | | Physical Properties | |
|---|---|---|---|---|
| Compound | | Concentration / mass-% | | |
| No. | Abbreviation | | T(N, I) | = 91.0 °C |
| 1 | AUUQU-2-F | 11.0 | | |
| 2 | AUUQU-3-F | 11.0 | | |
| 3 | AUUQU-4-F | 7.0 | | |
| 4 | AUUQU-5-F | 5.0 | | |
| 5 | AUUQU-7-F | 6.0 | | |
| 6 | CFUQU-2-OT | 8.0 | | |
| 7 | CF₂UQU-3-OT | 8.0 | | |
| 8 | CFUQU-2-T | 9.0 | | |
| 9 | CFUQU-3-T | 8.0 | | |
| 10 | CFUQU-5-T | 7.0 | | |
| 11 | PUQU-3-F | 9.0 | | |
| 12 | PUQU-3-F | 11.0 | | |
| Σ | | 100.0 | | |

The results are shown in table 8, too. The mixture of this example, E-2, has rather good VHR values. They are even almost comparable to those of mixture C-2 of comparative example 2 and better than all respective values of all electrically addressable mixtures (C-3 to C-5 of comparative examples 3 to 5) at the respective conditions, with the sole exception of the value at 20 °C prior to UV irradiation for the mixture C-5 (comparative example 5). At the same time the mixture of this example, mixture E-2, in contrast to those of comparative examples 1 and 2 (mixtures C-1 and C-2) can be electrically addressed in the blue phase after addition of an appropriate amount of an appropriate chiral dopant.

### Use-example 3

In this use-example the liquid crystalline mixture E-3 is prepared and investigated. The mixture E-3 has the respective composition and properties shown in table 10.

**Table 10: Composition and properties liquid crystal mixture E-3**

| Composition | | | Physical Properties | |
|---|---|---|---|---|
| Compound | | Concentration / mass-% | | |
| No. | Abbreviation | | T(N, I) | = 102.5 °C |
| 1 | AUUQU-3-N | 11.0 | | |
| 2 | CUZU-2-N | 11.0 | | |
| 3 | CUZU-3-N | 11.0 | | |
| 4 | GZU-3A-N | 8.0 | | |
| 5 | GZU-4A-N | 5.0 | | |
| 6 | HP-3N.F | 8.0 | | |
| 7 | AUUQU-3-F | 9.0 | | |
| 8 | AUUQU-3-T | 10.0 | | |
| 9 | AUUQGU-3-F | 8.0 | | |
| 10 | PUZU-2-F | 4.0 | | |
| 11 | PUZU-3-F | 5.0 | | |
| 12 | CFUQU-3-F | 10.0 | | |
| Σ | | 100.0 | | |

The mixture of this example, E-3, is investigated as described above.

### Use-example 4

In this use-example the liquid crystalline mixture E-4 is prepared and investigated, having the composition and properties shown in table 12.

**Table 12: Composition and properties liquid crystal mixture E-4**

| Composition | | | Physical Properties | |
|---|---|---|---|---|
| Compound | | Concentration | | |
| No. | Abbreviation | / mass-% | T(N, I) | = 100.0 °C |
| 1 | AUUQU-3-N | 11.0 | | |
| 2 | AUZU-2-N | 10.0 | | |
| 3 | AUZU-3-N | 9.0 | | |
| 4 | GZU-3A-N | 9.0 | | |
| 5 | GZU-4A-N | 8.0 | | |
| 6 | HP-3N.F | 4.0 | | |
| 7 | AUUQU-2-F | 8.0 | | |
| 8 | AUUQU-3-F | 8.0 | | |
| 9 | AUUQU-4-F | 4.0 | | |
| 10 | AUUQGU-3-F | 7.0 | | |
| 11 | CFUQU-3-F | 11.0 | | |
| 12 | CF₂UQU-3-F | 11.0 | | |
| Σ | | 100.0 | | |

The mixture of this example, E-4, is investigated as described above. Prior to the determination of the electro-optical properties simultaneously both 9 % of the chiral agent R-5011 and 5 % of the compound P(O3)₄UQU-3O-F are added to the mixture E-4. The results are shown in table 8, too.

### Use-example 5

In this use-example the liquid crystalline mixture E-5 is prepared and investigated, having the composition and properties shown in table 13.

**Table 13: Composition and properties liquid crystal mixture E-5**

| Composition | | | Physical Properties | |
|---|---|---|---|---|
| Compound | | Concentration | | |
| No. | Abbreviation | / mass-% | T(N, I) | = 108.0 °C |
| 1 | AUUQU-3-N | 10.0 | | |
| 2 | AUZU-2-N | 9.0 | | |
| 3 | AUZU-3-N | 8.0 | | |
| 4 | GZU-3A-N | 9.0 | | |
| 5 | GZU-4A-N | 6.0 | | |
| 6 | HP-3N.F | 5.0 | | |
| 7 | AUUQU-2-F | 8.0 | | |
| 8 | AUUQU-3-F | 9.0 | | |
| 9 | CFUQU-2-F | 9.0 | | |
| 10 | CFUQU-3-F | 10.0 | | |
| 11 | CF₂UQU-3-F | 10.0 | | |
| 12 | CFUQU-5-SF5 | 7.0 | | |
| Σ | | 100.0 | | |

The mixture of this example, E-5, is investigated as described above. Prior to the determination of the electro-optical properties simultaneously both 9 % of the chiral agent R-5011 and 5 % of the compound P(O3)₄UQU-3O-F are added to the mixture E-5. The results are shown in table 8 too.

### Use-example 6

In this use-example the liquid crystalline mixture E-6 is prepared and investigated, having the composition and properties shown in table 13.

**Table 14: Composition and properties liquid crystal mixture E-6**

| Composition | | | Physical Properties |
|---|---|---|---|
| Compound | | Concentration | |
| No. | Abbreviation | / mass-% | |
| 1 | ME2N.F | 9.0 | |
| 2 | ME3N.F | 9.0 | |
| 3 | ME5N.F | 6.0 | |
| 4 | AUUQU-2-F | 8.0 | |
| 5 | AUUQU-3-F | 9.0 | |
| 6 | AUUQU-4-F | 5.0 | |
| 7 | CFUQU-3-F | 8.0 | |
| 8 | CFUQU-2-N | 5.0 | |
| 9 | CFUQU-3-N | 4.0 | |
| 10 | CFUQU-2-T | 10.0 | |
| 11 | CFUQU-3-T | 9.0 | |
| 12 | CFUQU-5-T | 9.0 | |
| 13 | CFUQU-2-OT | 9.0 | |
| Σ | | 100.0 | |

The mixture of this example, E-6, is investigated as described above. Prior to the determination of the electro-optical properties simultaneously both 5 % of the chiral agent R-5011 and 5 % of the compound P(O3)₂UQU-3O-T are added to the mixture E-6. The results are shown in table 15.

**Table 15: Results of VHR and e-o**

| Use-Ex. # | | 6 | 7 | 8 | 9 | 10 |
|---|---|---|---|---|---|---|
| Mixture # | | E-6 | E-7 | E-8 | E-9 | E-10 |
| | | | | | | |
| T(N,I) / °C | | n.d. | n.d. | n.d. | n.d. | n.d. |
| | | | | | | |

| t(UVexp.)/min | T /°C | Voltage Holding Ratio / % | | | | |
|---|---|---|---|---|---|---|
| 0 | 20 | n.d. | n.d. | n.d. | n.d. | n.d. |
| 0 | 100 | n.d. | n.d. | n.d. | n.d. | n.d. |
| 120 | 20 | n.d. | n.d. | n.d. | n.d. | n.d. |
| 120 | 100 | n.d. | n.d. | n.d. | n.d. | n.d. |
| | | | | | | |
| c(R-5011)/ / % | | 5.0 | | | | |
| c(P(O3)₂UQU-O3-T) | | 5.0 | 0 | | | |

| Characteristic Temperatures | | | | | | |
|---|---|---|---|---|---|---|
| T₂ /°C | | n.d. | 76.0 | n.d. | n.d. | n.d. |
| T₃ /°C | | n.d. | 71.5 | n.d. | n.d. | n.d. |
| T₁ /°C | | 66.5 | 68.5 | n.d. | n.d. | n.d. |
| ΔT(BP)/° | | >11 | 7.5 | n.d. | n.d. | n.d. |
| ΔT(FR) /° | | n.d. | 4.5 | n.d. | n.d. | n.d. |

| Characteristic Voltages | | | | | | |
|---|---|---|---|---|---|---|
| T_{op.} °/°C | | 68.5 | 70.5 | n.d. | n.d. | n.d. |
| V₁₀/V | | 20.0 | 29.0 | n.d. | n.d. | n.d. |
| V₉₀/V | | 37.5 | 52.0 | n.d. | n.d. | n.d. |

| | | | | | | |
|---|---|---|---|---|---|---|
| Remarks:\ n.d.: not determined. | | | | | | |

### Use-example 7

In this use-example the liquid crystalline mixture E-7 is prepared and investigated, having the composition and properties shown in table 16.

**Table 16: Composition and properties liquid crystals mixture E-7**

| Composition | | | Physical Properties |
|---|---|---|---|
| Compound | | Concentration | |
| No. | Abbreviation | / mass-% | |
| 1 | ME2N.F | 10.0 | |
| 2 | ME3N.F | 12.0 | |
| 3 | ME5N.F | 11.0 | |
| 4 | AUUQU-2-F | 8.0 | |
| 5 | AUUQU-3-F | 9.0 | |
| 6 | AUUQU-4-F | 5.0 | |
| 7 | CFUQU-3-F | 8.0 | |
| 8 | CFUQU-2-T | 10.0 | |
| 9 | CFUQU-3-T | 9.0 | |
| 10 | CFUQU-5-T | 9.0 | |
| 11 | CFUQU-2-OT | 9.0 | |
| Σ | | 100.0 | |

The mixture of this example, E-7, is investigated as described above. Prior to the determination of the electro-optical properties 5 % of the chiral agent R-5011 are added to the mixture E-7. The results are shown in table 15, too.

### Use-example 8

In this use-example the liquid crystalline mixture E-8 is prepared and investigated, having the composition and properties shown in table 17.

**Table 17: Composition and properties liquid crystal mixture E-8**

| Composition | | | Physical Properties |
|---|---|---|---|
| Compound | | Concentration | |
| No. | Abbreviation | / mass-% | |
| 1 | ME3N.F | 8.0 | |
| 2 | ME5N.F | 8.0 | |
| 3 | AUUQU-2-N | 6.0 | |
| 4 | AUUQU-3-N | 8.0 | |
| 5 | AUUQU-2-F | 8.0 | |
| 6 | AUUQU-3-F | 10.0 | |
| 7 | AUUQU-4-F | 5.0 | |
| 8 | CFUQU-2-T | 7.0 | |
| 9 | CFUQU-3-T | 8.0 | |
| 10 | CFUQU-5-T | 7.0 | |
| 11 | CFUQU-2-OT | 9.0 | |
| 12 | CFUQU-3-OT | 7.0 | |
| 13 | CF2UQU-3-OT | 9.0 | |
| Σ | | 100.0 | |

The mixture of this example, E-8, is investigated as described above. Prior to the determination of the electro-optical properties 5 % of the chiral agent R-5011 are added to the mixture E-8. The results are shown in table 15, too.

### Use-example 9

In this use-example the liquid crystalline mixture E-9 is prepared and investigated, having the composition and properties shown in table 18.

**Table 18: Composition and properties liquid crystal mixture E-9**

| Composition | | | Physical Properties |
|---|---|---|---|
| Compound | | Concentration | |
| No. | Abbreviation | / mass-% | |
| 1 | ME2N.F | 9.0 | |
| 2 | ME3N.F | 10.0 | |
| 3 | ME5N.F | 9.0 | |
| 4 | GZU-3A-N | 8.0 | |
| 5 | AUUQU-3-N | 7.0 | |
| 6 | AUUQU-2-F | 8.0 | |
| 7 | AUUQU-3-F | 9.0 | |
| 8 | AUUQU-5-F | 5.0 | |
| 9 | CFUQU-2-T | 9.0 | |
| 10 | CFUQU-3-T | 9.0 | |
| 11 | CFUQU-5-T | 8.0 | |
| 12 | CFUQU-2-OT | 9.0 | |
| Σ | | 100.0 | |

The mixture of this example, E-9, is investigated as described above. Prior to the determination of the electro-optical properties 5 % of the chiral agent R-5011 are added to the mixture E-9. The results are shown in table 15, too.

### Use-example 10

In this use-example the liquid crystalline mixture E-10 is prepared and investigated, having the composition and properties shown in table 19.

**Table 19: Composition and properties liquid crystal mixture E-10**

| Composition | | | Physical Properties |
|---|---|---|---|
| Compound | | Concentration | |
| No. | Abbreviation | / mass-% | |
| 1 | PUQU-2-F | 9.0 | |
| 2 | PUQU-3-F | 11.0 | |
| 3 | AUUQU-2-F | 11.0 | |
| 4 | AUUQU-3-F | 11.0 | |
| 5 | AUUQU-4-F | 7.0 | |
| 6 | AUUQU-5-F | 5.0 | |
| 7 | AUUQU-7-F | 6.0 | |
| 8 | CFUQU-2-OT | 8.0 | |
| 9 | CFUQU-3-OT | 8.0 | |
| 10 | CFUQU-2-T | 9.0 | |
| 11 | CFUQU-3-T | 8.0 | |
| 12 | CFUQU-4-T | 7.0 | |
| Σ | | 100.0 | |

The mixture of this example, E-10, is investigated as described above. Prior to the determination of the electro-optical properties 5 % of the chiral agent R-5011 are added to the mixture E-10. The results are shown in table 15, too.

### Use-example 11

In this use-example the liquid crystalline mixture E-11 is prepared and investigated, having the composition and properties shown in table 20.

**Table 20: Composition and properties liquid crystal mixture E-11**

| Composition | | | Physical Properties |
|---|---|---|---|
| Compound | | Concentration | |
| No. | Abbreviation | / mass-% | |
| 1 | PUQU-2-F | 8.0 | |
| 2 | PUQU-3-F | 17.0 | |
| 3 | AUUQU-2-F | 10.0 | |
| 4 | AUUQU-3-F | 10.0 | |
| 5 | AUUQU-4-F | 7.0 | |
| 6 | AUUQU-5-F | 5.0 | |
| 7 | AUUQU-7-F | 5.0 | |
| 8 | FUQU-1-OT | 4.0 | |
| 9 | CFUQU-2-OT | 7.0 | |
| 10 | CF2UQU-3-OT | 7.0 | |
| 11 | CFUQU-2-T | 7.0 | |
| 12 | CFUQU-3-T | 7.0 | |
| 13 | CFUQU-5-T | 6.0 | |
| Σ | | 100.0 | |

The mixture of this example, E-11, is investigated as described above. The results are shown in table 21.

**Table 21: Results of VHR and e-o**

| Use-Ex. # | | 11 | 12 | 13 | 14 | 15 |
|---|---|---|---|---|---|---|
| Mixture # | | E-11 | E-12 | E-13 | E-14 | E-15 |
| | | | | | | |
| T(N,I) / °C | | n.d. | 53.0 | 53.5 | 65.0 | 63.0 |
| | | | | | | |

| t(UVexp.)/min | T/°C | Voltage Holding Ratio / % | | | | |
|---|---|---|---|---|---|---|
| 0 | 20 | n.d. | n.d. | n.d. | n.d. | n.d. |
| 0 | 100 | 44 | 54 | n.d. | 60 | n.d. |
| 120 | 20 | 82.0 | 81.1 | n.d. | 80.3 | n.d. |
| 120 | 100 | 40 | 26 | n.d. | 22 | n.d. |
| | | | | | | |
| c(R-5011)/ / % | | 5.0 | | | | |

| Characteristic Temperatures | | | | | | |
|---|---|---|---|---|---|---|
| T₂ /°C | | n.d. | n.d. | 36.0 | n.d. | 45.0 |
| T₃ /°C | | n.d. | n.d. | 30.0 | n.d. | 41.0 |
| T₁ /°C | | n.d. | n.d. | 27.5 | n.d. | 39.0 |
| ΔT(BP) /° | | n.d. | n.d. | 8.5 | n.d. | 6.0 |
| ΔT(FR) /° | | n.d. | n.d. | 6.0. | n.d. | 4.0 |

| Characteristic Voltages | | | | | | |
|---|---|---|---|---|---|---|
| T_{op.} °/°C | | n.d. | n.d. | 29.5 | n.d. | 41.0 |
| V₁₀/V | | n.d. | n.d. | 29.0 | n.d. | 25.0 |
| V₉₀/V | | n.d. | n.d. | 55.0 | n.d. | 49.0 |

| | | | | | | |
|---|---|---|---|---|---|---|
| Remarks: n.d.: not determined. | | | | | | |

### Use-example 12

In this use-example the liquid crystalline mixture E-12 is prepared and investigated, having the composition and properties shown in table 22.

**Table 22: Composition and properties liquid crystal mixture E-12**

| Composition | | | Physical Properties | |
|---|---|---|---|---|
| Compound No. | Abbreviation | Concentration / mass-% | T(N, I) = | 53.0 °C |
| 1 | PUQU-2-F | 8.0 | | |
| 2 | PUQU-3-F | 10.0 | | |
| 3 | AUZU-2-F | 8.0 | | |
| 4 | AUZU-3-F | 9.0 | | |
| 5 | AUZU-4-F | 9.0 | | |
| 6 | AUUQU-2-F | 9.0 | | |
| 7 | AUUQU-3-F | 9.0 | | |
| 8 | AUUQU-4-F | 6.0 | | |
| 9 | AUUQU-5-F | 5.0 | | |
| 10 | AUUQU-7-F | 5.0 | | |
| 11 | CFUQU-2-T | 9.0 | | |
| 12 | CFUQU-3-T | 7.0 | | |
| 13 | CFUQU-5-T | 6.0 | | |
| Σ | | 100.0 | | |

The mixture of this example, E-12 is investigated as described above. The results are shown in table 21, too.

### Use-example 13

In this use-example the liquid crystalline mixture E-13 is prepared and investigated, having the composition and properties shown in table 23.

**Table 23: Composition and properties liquid crystal mixture E-13**

| Composition | | | Physical Properties | |
|---|---|---|---|---|
| Compound No. | Abbreviation | Concentration / mass-% | T(N, I) = | 53.5 °C |
| 1 | PUQU-3-F | 4.0 | | |
| 2 | AUZU-2-F | 7.0 | | |
| 3 | AUZU-3-F | 8.0 | | |
| 4 | AUZU-4-F | 8.0 | | |
| 5 | AUZU-2-T | 7.0 | | |
| 6 | AUZU-3-T | 7.0 | | |
| 7 | AUUQU-2-F | 8.0 | | |
| 8 | AUUQU-3-F | 8.0 | | |
| 9 | AUUQU-4-F | 5.0 | | |
| 10 | AUUQU-5-F | 5.0 | | |
| 11 | AUUQU-7-F | 5.0 | | |
| 12 | FUQU-1-OT | 6.0 | | |
| 13 | FUQU-3-F | 6.0 | | |
| 14 | CFUQU-3-T | 6.0 | | |
| 15 | CFUQU-5-T | 5.0 | | |
| 16 | CFUQU-3-OT | 5.0 | | |
| Σ | | 100.0 | | |

The mixture of this example, E-13, is investigated as described above. Prior to the determination of the electro-optical properties 5 % of the chiral agent R-5011 are added to the mixture E-13. The results are shown in table 21, too.

### Use-example 14

In this use-example the liquid crystalline mixture E-14 is prepared and investigated, having the composition and properties shown in table 23.

**Table 24: Composition and properties liquid crystal mixture E-14**

| Composition | | | Physical Properties | |
|---|---|---|---|---|
| Compound No. | Abbreviation | Concentration / mass-% | T(N, I) = | 65.0 °C |
| 1 | AUZU-3-F | 6.0 | | |
| 2 | AUZU-4-F | 5.0 | | |
| 3 | AUZU-5-F | 4.0 | | |
| 4 | AUZU-2-T | 6.0 | | |
| 5 | AUZU-3-T | 7.0 | | |
| 6 | AUZU-5-T | 7.0 | | |
| 7 | AUUQU-2-F | 7.0 | | |
| 8 | AUUQU-3-F | 10.0 | | |
| 9 | AUUQU-5-F | 7.0 | | |
| 10 | AUUQU-7-F | 6.0 | | |
| 11 | FUQU-1-OT | 6.0 | | |
| 12 | FUQU-3-T | 5.0 | | |
| 13 | FUQU-3-F | 4.0 | | |
| 14 | CFUQU-3-T | 6.0 | | |
| 15 | CFUQU-5-T | 7.0 | | |
| 16 | CFUQU-3-OT | 7.0 | | |
| Σ | | 100.0 | | |

The mixture of this example, E-14, is investigated as described above.

### Use-example 15

In this use-example the liquid crystalline mixture E-15 is prepared and investigated, having the composition and properties shown in table 24.

**Table 24: Composition and properties liquid crystal mixture E-15**

| Composition | | | Physical Properties | |
|---|---|---|---|---|
| Compound No. | Abbreviation | Concentration / mass-% | T(N, I) = | 63.0 °C |
| 1 | AUZU-2-T | 8.0 | | |
| 2 | AUZU-3-T | 9.0 | | |
| 3 | AUZU-5-T | 6.0 | | |
| 4 | AUUQU-3-T | 10.0 | | |
| 5 | AUUQU-3-OT | 9.0 | | |
| 6 | DUUQU-2-F | 8.0 | | |
| 7 | DUUQU-3-F | 7.0 | | |
| 8 | DUUQU-4-F | 8.0 | | |
| 9 | DUUQU-5-F | 7.0 | | |
| 10 | FUQU-1-OT | 7.0 | | |
| 11 | FUQU-3-T | 8.0 | | |
| 12 | CFUQU-2-T | 4.0 | | |
| 13 | CFUQU-3-T | 5.0 | | |
| 14 | CFUQU-5-T | 4.0 | | |
| Σ | | 100.0 | | |

The mixture of this example, E-15, is investigated as described above. Prior to the determination of the electro-optical properties 5 % of the chiral agent R-5011 are added to the mixture E-15. The results are shown in table 21, too.

## Claims

1. Mesogenic media based on a mixture of positive dielectrically compounds, **characterised in that** it comprises
a) at least one compound of the formula I, 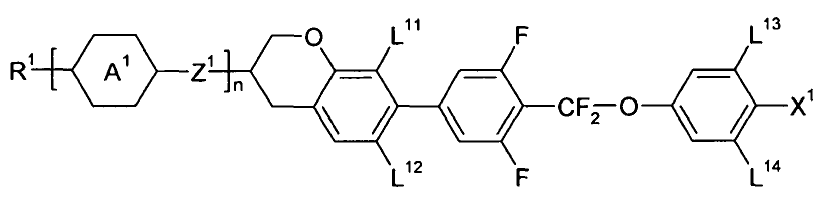 wherein
L¹¹ to L¹⁴ are, independently of each other, H or F, 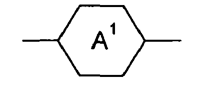 is an aromatic and/or alicyclic ring, or a group comprising two or more fused aromatic or alicyclic rings, wherein these rings optionally contain one or more hetero atoms selected from N, O and/or S, and are optionally mono-, di- or polysubstituted by R,
R is halogen, CN, NCS, SCN, SF₅, SO₂CF₃ or alkyl, which is straight chain or branched, has 1 to 20 C-atoms, is unsubstituted, mono- or poly-substituted by F, Cl, or CN, and in which one or more CH₂ groups are optionally replaced, in each case independently from one another, by -O-, -S-, -NH-, -NR⁰¹-,-SiR⁰¹R⁰²-, -CO-, -COO-, -OCO-, -OCO-O-, -S-CO-, -CO-S-, -CY⁰¹=CY⁰¹- or -C≡C- in such a manner that O and/or S atoms are not linked directly to one another,
R¹ has the meaning given for R
Z¹ is -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰¹- -CR⁰¹=CH- -CY⁰¹=CY⁰²-, -C≡C-, -(CH₂)₄-, -CH=CH-CO-O-, -O-CO-CH=CH-or a single bond,
Y⁰¹ and Y⁰² are, independently of each other, F, Cl or CN, and alternatively one of them may be H,
R⁰¹ and R⁰² are, independently of each other, H or alkyl with 1 to 12 C-atoms,
n is 0 or 1,
X¹ is halogen, OCF₃, CF₃, OCHF₂, CN, NCS, SCN, SF₅ or SO₂CF₃, and
b) 1-20 % by weight of at least one chiral compound or more chiral compounds with a HTP of ≥ 20 µm ⁻¹, and
said mesogenic media of a) and b) exhibiting a Blue Phase.

2. Mesogenic media according to Claim 1, **characterised in that** it comprises at least one compound of the formula I, wherein R¹ denotes halogen, n-alkyl, n-alkoxy with 1 to 9 C-atoms or alkenyl, alkenyloxy or alkoxyalkyl with 2 to 9 C-atoms.

3. Mesogenic media according to Claim 1 or 2, **characterised in that** it comprises at least one compound of the formula I, wherein Z¹ denotes -CO-O-, -O-CO-, -CF₂-O-, -O -CF₂ - or a single bond.

4. Mesogenic media according to one or more of Claims 1 to 3, **characterised in that** it comprises at least one compound of the formula I, wherein n denotes 1.

5. Mesogenic media according to one or more of Claims 1 to 4, **characterised in that** it comprises one or more compounds of formula II 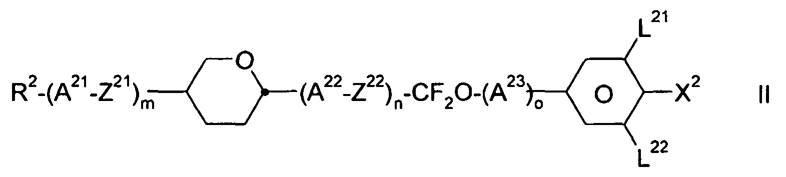 wherein
R² has the meaning given under formula I for R¹,
A²¹, A²² and A²³ are, each independently of each other, 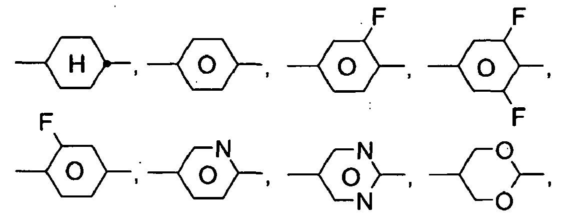 whereby each of A²¹ and A²² may have the same or a different meaning if present twice,
Z²¹ and Z²² are, each independently of each other, a single bond, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O- or -O-CO-, whereby each of Z²¹ and Z²² may have the same or a different meaning if present twice,
X² is halogen, -CN, -NCS, -SF₅, -SO₂CF₃, alkyl, alkenyl, alkenyloxy or alkylalkoxy or alkoxy radical each mono- or polysubstituted by CN and/or halogen,
L²¹ and L²² are, each independently of each other, H or F, and
m is 0, 1 or 2,
n is 0, 1, 2 or 3,
o is 0, 1 or 2, and
m + n + o is 3 or less,

6. Mesogenic media according to one or more of Claims 1 to 5, **characterised in that** it comprises at least one or more compounds of formula III 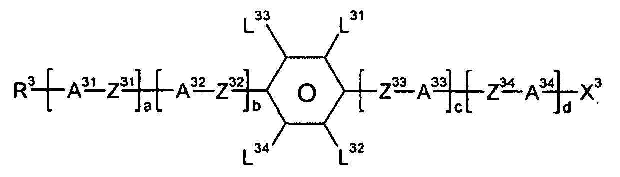 wherein
a, b, c and d are each independently of each other 0, 1 or 2, whereby
a+b+c+d is 4 or less,
A³¹, A³², A³³ and A³⁴ are, each independently of each other, 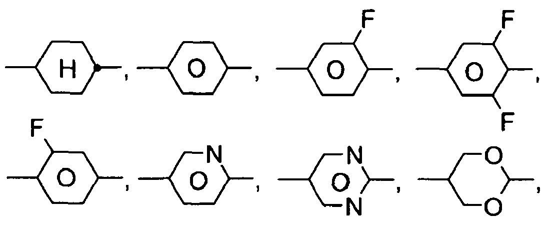 whereby each of A³¹, A³², A³³ and A³⁴ may have the same or a different meaning if present twice,
Z³¹, Z³², Z³³ and Z³⁴ are, each independently of each other, a single bond, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡D-, -CH₂O-, -OCH₂-, -CF₂O-, -OCF₂-, -CO-O- or -O-CO-, whereby each of Z³¹, Z³², Z³³ and Z³⁴ may have the same or a different meaning if present twice,
R³ is an alkyl or alkoxy radical having from 1 to 15 carbon atoms, wherein one or more methylene groups of said alkyl or alkoxy radical may be replaced independently of each other by -O-, -S-, -SiR^{x}R^{y}-, -CH=CH-, -C≡D-, -CO-O- and/or -O-CO-such that oxygen and/or sulfur atoms are not linked directly to each other, said alkyl or alkoxy radical being unsubstituted or mono-substituted with a CN group or mono- or poly-substituted with halogen,
L³¹, L³², L³³ and L³⁴ are each independently of each other hydrogen, halogen, a CN group, an alkyl or alkoxy radical having from 1 to 15 carbon atoms wherein one or more methylene groups of said alkyl or alkoxy radical may be replaced independently of each other by -O-, -S-, -SiR^{x}R^{y}-, -CH=CH-, -C≡D-, -CO-O- and/or -O-CO- such that oxygen and/or sulfur atoms are not linked directly to each other, said alkyl or alkoxy radical being unsubstituted or mono-substituted with a -CN group or mono- or poly-substituted with halogen, with the proviso that at least one of L³¹, L³², L³³ and L³⁴ is not hydrogen,
X³ is F, Cl, CF₃, OCF₃, CN, NCS, SF₅ or SO₂-R^{z},
R^{x} and R^{y} are independently of each other hydrogen or an alkyl radical having from 1 to 7 carbon atoms, and
R^{z} is an alkyl radical having from 1 to 7 carbon atoms, said alkyl radical being unsubstituted or mono- or poly-substituted with halogen.

7. Mesogenic media according to one or more of Claims 1 to 6, **characterised in that** it additionally comprises one or more compounds enhancing the phase range of the blue phase and/or decrease the temperature dependence of the electro-optical effect.

8. Mesogenic media according to one or more of Claims 1 to 7, **characterised in that** the medium comprises additionally a polymer precursor which upon polymerisation stabilize the phase range of the blue phase and/or decrease the temperature dependence of the electro-optical effect.

9. Mesogenic media according to one or more of Claims 1 to 8, **characterised in that** the medium additionally comprises one or more compounds of formula Z 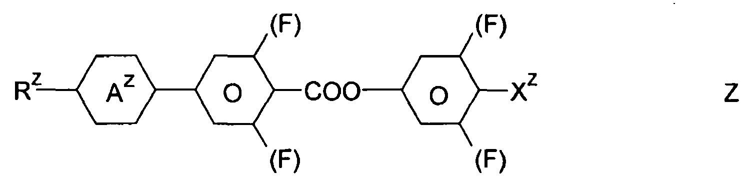 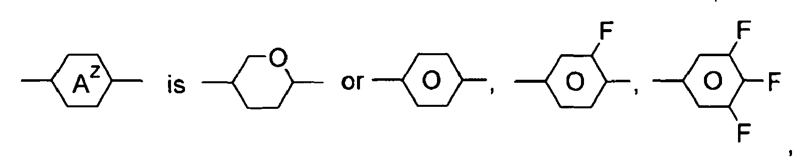 wherein
X^{z} is F, Cl, CN, NCS, OCF₃, CF₃ or SF₅, and
R^{z} is an alkyl radical having from 1 to 7 carbon atoms, said alkyl radical being unsubstituted or mono- or poly-substituted with halogen.

10. Mesogenic media according to one or more of Claims 1 to 9, **characterised in that** the medium additionally comprises one or more compounds of formula N 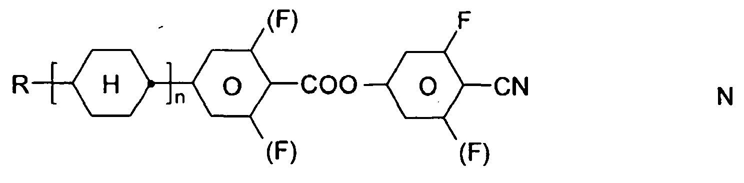 wherein
R has the meaning given under formula I for R,
"Alkyl" is alkyl with 1 to 7 C-atoms, and
n is 0 or 1.

11. Mesogenic media according to one or more of Claims 1 to 10, **characterised in that** the medium additionally comprises one or more compounds selected from the group of ester compounds of formula E 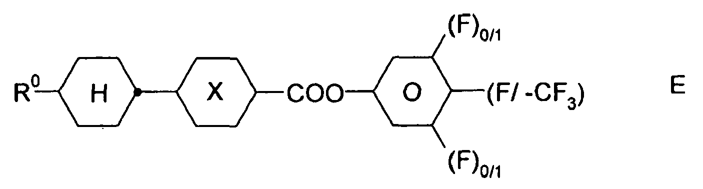 in which R⁰ has the meaning given for R under formula I and 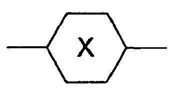 is

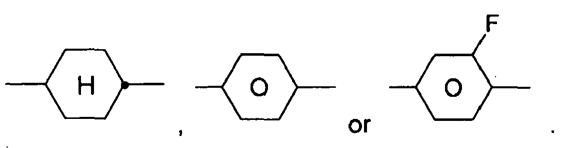

12. Mesogenic media according to one or more of Claims 1 to 11, **characterised in that** the medium additionally comprises one compound or more compounds selected from the group of formulae Q-1 and Q-2 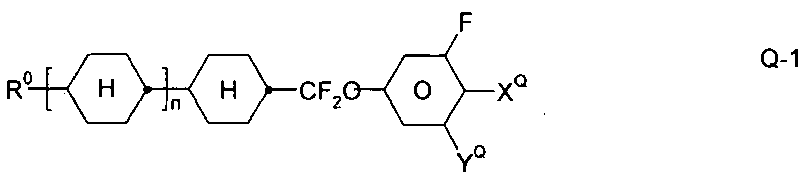 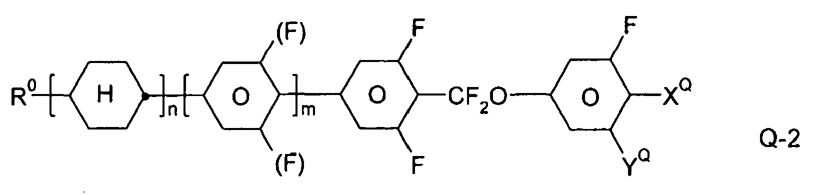 wherein R⁰ has the meaning given for R under formula I and n and m are, independently of each other 0 or 1.

13. Mesogenic media according to one or more of Claims 1 to 12, **characterised in that** the medium additionally comprises one or more dioxane compounds, selected from the group of formulae Dx-1 and Dx-2 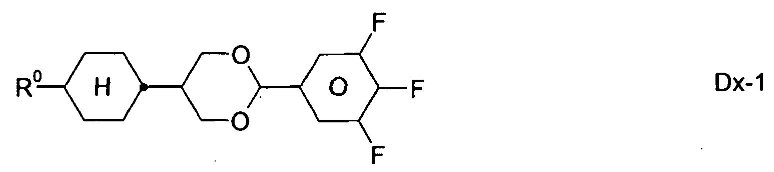 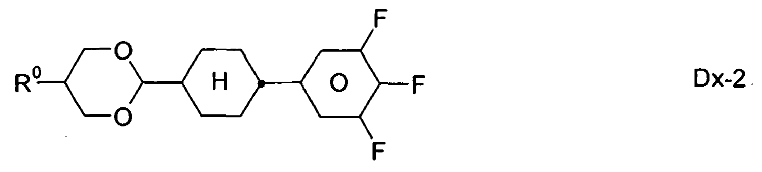 wherein R⁰ has the meaning given for R under formula I.

14. Light controlling element, **characterized in that** it comprises a mesogenic media according to one or more of Claims 1 and 13.

15. Electro-optical display, **characterised in that** it contains a mesogenic media according to one or more of Claims 1 to 13.

## Patentansprüche

1. Mesogenes Medium basierend auf einer Mischung dielektrisch positiver Verbindungen, **dadurch gekennzeichnet, dass** es
a) mindestens eine Verbindung der Formel I 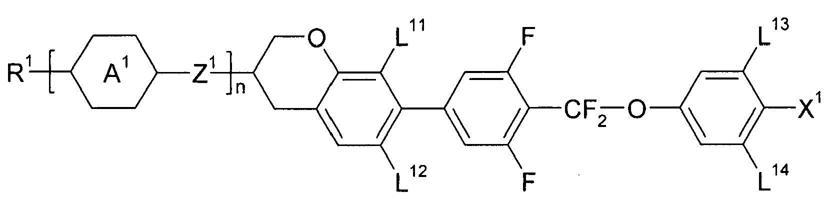 in der
L¹¹ bis L¹⁴ unabhängig voneinander H oder F bedeuten, 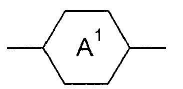 einen aromatischen und/oder alicyclischen Ring oder eine Gruppe mit zwei oder mehr anellierten aromatischen oder alicyclischen Ringen bedeutet, wobei diese Ringe gegebenenfalls ein oder mehrere aus N, O und/oder S ausgewählte Heteroatome enthalten und gegebenenfalls durch R ein-, zwei- oder mehrfach substituiert sind,
R Halogen, CN, NCS, SCN, SF₅, SO₂CF₃ oder Alkyl bedeutet, das geradkettig oder verzweigt ist, 1 bis 20 C-Atome aufweist, unsubstituiert, ein- oder mehrfach durch F, Cl oder CN substituiert ist und in dem gegebenenfalls eine oder mehrere CH₂-Gruppen jeweils unabhängig voneinander so durch -O-, -S-, -NH-, -NR⁰¹-, -SiR⁰¹R⁰²-, -CO-, -COO-, -OCO-, -OCO-O-, -S-CO-, -CO-S-, -CY⁰¹=CY⁰¹- oder -C≡C-ersetzt sind, dass O- und/oder S-Atome nicht direkt miteinander verknüpft sind,
R¹ die für R angegebene Bedeutung besitzt,
Z¹ -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰¹- -CR⁰¹=CH- -CY⁰¹=CY⁰²-, -C≡C-, -(CH₂)₄-, -CH=CH-CO-O-, -O-CO-CH=CH-oder eine Einfachbindung bedeutet,
Y⁰¹ und Y⁰² unabhängig voneinander F, Cl oder CN bedeuten und eines von ihren auch H bedeuten kann,
R⁰¹ und R⁰² unabhängig voneinander, H oder Alkyl mit 1 bis 12 C-Atomen bedeuten,
n für 0 oder 1 steht,
X¹ Halogen, OCF₃, CF₃, OCHF₂, CN, NCS, SCN, SF₅ oder SO₂CF₃ bedeutet, und
b) 1-20 Gew.-% mindestens einer chiralen Verbindung oder mehrerer chiraler Verbindungen mit einem HTP von ≥ 20 µm ⁻¹
enthält und wobei das mesogene Medium aus a) und b) eine blaue Phase aufweist.

2. Mesogenes Medium nach Anspruch 1, **dadurch gekennzeichnet, dass** es mindestens eine Verbindung der Formel I enthält, in der R¹ Halogen, n-Alkyl, n-Alkoxy mit 1 bis 9 C-Atomen oder Alkenyl, Alkenyloxy oder Alkoxyalkyl mit 2 bis 9 C-Atomen bedeutet.

3. Mesogenes Medium nach Anspruch 1 oder 2, **dadurch gekennzeichnet, dass** es mindestens eine Verbindung der Formel I enthält, in der Z¹ -CO-O-, -O-CO-, -CF₂-O-, -O -CF₂ - oder eine Einfachbindung bedeutet.

4. Mesogenes Medium nach einem oder mehreren der Ansprüche 1 bis 3, **dadurch gekennzeichnet, dass** es mindestens eine Verbindung der Formel I enthält, in der n für 1 steht.

5. Mesogenes Medium nach einem oder mehreren der Ansprüche 1 bis 4, **dadurch gekennzeichnet, dass** es eine oder mehrere Verbindungen der Formel II 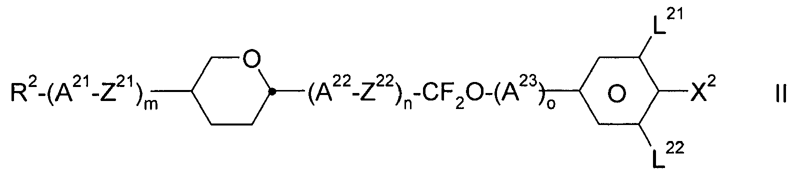 enthält, in der
R² die unter Formel I für R¹ angegebene Bedeutung besitzt,
A²¹, A²² und A²³ jeweils unabhängig voneinander 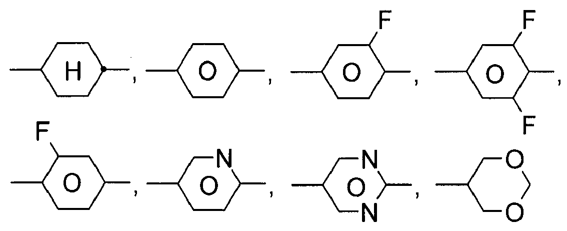 bedeuten, wobei A²¹ und A²² bei zweifachem Vorhandensein jeweils die gleiche oder eine unterschiedliche Bedeutung besitzen können,
Z²¹ und Z²² jeweils unabhängig voneinander eine Einfachbindung, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C=C-, -CH₂O-, -OCH₂-, -CF₂O-, -OCF₂-, -CO-O- oder -O-CO-, bedeuten, wobei Z²¹ und Z²² bei zweifachem Vorhandensein jeweils die gleiche oder eine unterschiedliche Bedeutung besitzen können,
X² Halogen, -CN, -NCS, -SF₅, -SO₂CF₃, Alkyl-, Alkenyl-, Alkenyloxy- oder Alkylalkoxy- oder Alkoxyrest bedeutet, der jeweils ein- oder mehrfach durch CN und/oder Halogen substituiert ist,
L²¹ und L²² jeweils unabhängig voneinander H oder F bedeuten und
m für 0, 1 oder 2 steht,
n für 0, 1, 2 oder 3 steht,
o für 0, 1 oder 2 steht und
m + n + o 3 oder weniger beträgt.

6. Mesogenes Medium nach einem oder mehreren der Ansprüche 1 bis 5, **dadurch gekennzeichnet, dass** es mindestens eine oder mehrere Verbindungen der Formel III 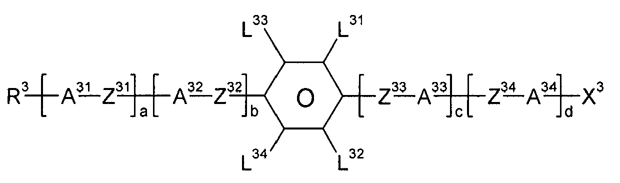 enthält, in der
a, b, c und d jeweils unabhängig voneinander für 0, 1 oder 2 stehen, wobei
a + b + c + d 4 oder weniger beträgt,
A³¹, A³², A³³ und A³⁴ jeweils unabhängig voneinander 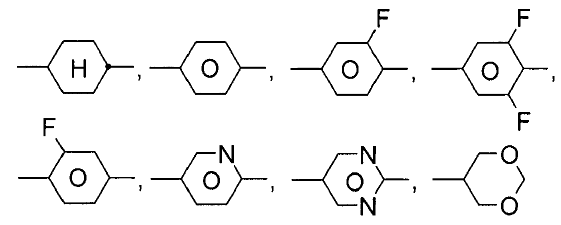 bedeuten, wobei A³¹, A³², A³³ und A³⁴ bei zweifachem Vorhandensein jeweils die gleiche oder eine unterschiedliche Bedeutung besitzen können,
Z³¹, Z³², Z³³ und Z³⁴ jeweils unabhängig voneinander eine Einfachbindung, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡D-, -CH₂O-, -OCH₂-, -CF₂O-, -OCF₂-, -CO-O- oder -O-CO-bedeuten, wobei Z³¹, Z³², Z³³ und Z³⁴ bei zweifachem Vorhandensein jeweils die gleiche oder eine unterschiedliche Bedeutung besitzen können,
R³ einen Alkyl- oder Alkoxyrest mit 1 bis 15 Kohlenstoffatomen bedeutet, in dem eine oder mehrere Methylengruppen des Alkyl- oder Alkoxyrestes unabhängig voneinander so durch -O-, -S-, -SiR^{x}R^{y}-, -CH=CH-, -C≡D-, -CO-O- und/oder -O-CO- ersetzt sein können, dass Sauerstoff- und/ oder Schwefelatome nicht direkt miteinander verknüpft sind, wobei der Alkyl- oder Alkoxyrest unsubstituiert oder einfach mit einer CN-Gruppe oder ein- oder mehrfach mit Halogen substituiert ist,
L³¹, L³², L³³ und L³⁴ jeweils unabhängig voneinander Wasserstoff, Halogen, eine CN-Gruppe, einen Alkyl- oder Alkoxyrest mit 1 bis 15 Kohlenstoffatomen, in dem eine oder mehrere Methylengruppen des Alkyl- oder Alkoxyrestes unabhängig voneinander so durch -O-, -S-, -SiR^{x}R^{y}-, -CH=CH-, -C≡D-, -CO-O- und/ oder -O-CO- ersetzt sein können, dass Sauerstoff- und/oder Schwefelatome nicht direkt miteinander verknüpft sind, wobei der Alkyl- oder Alkoxyrest unsubstituiert oder einfach mit einer -CN-Gruppe oder ein- oder mehrfach mit Halogen substituiert ist, bedeuten, mit der Maßgabe, dass mindestens eines von L³¹, L³², L³³ und L³⁴ nicht Wasserstoff bedeutet,
X³ F, Cl, CF₃, OCF₃, CN, NCS, SF₅ oder SO₂-R^{z} bedeutet,
R^{x} und R^{y} unabhängig voneinander Wasserstoff oder einen Alkylrest mit 1 bis 7 Kohlenstoffatomen bedeuten und
R^{z} einen Alkylrest mit 1 bis 7 Kohlenstoffatomen bedeutet, wobei der Alkylrest unsubstituiert oder einoder mehrfach mit Halogen substituiert ist.

7. Mesogenes Medium nach einem oder mehreren der Ansprüche 1 bis 6, **dadurch gekennzeichnet, dass** es zusätzlich eine oder mehrere Verbindungen enthält, die den Phasenbereich der blauen Phase verbessern und/oder die Temperaturabhängigkeit des elektrooptischen Effekts verringern.

8. Mesogenes Medium nach einem oder mehreren der Ansprüche 1 bis 7, **dadurch gekennzeichnet, dass** das Medium zusätzlich einen Polymervorläufer enthält, der bei der Polymerisierung den Phasenbereich der blauen Phase stabilisiert und/oder die Temperaturabhängigkeit des elektrooptischen Effekts verringert.

9. Mesogenes Medium nach einem oder mehreren der Ansprüche 1 bis 8, **dadurch gekennzeichnet, dass** das Medium zusätzlich eine oder mehrere Verbindungen der Formel Z 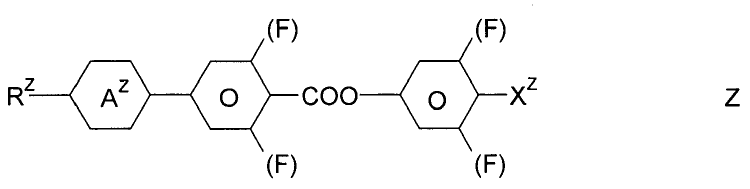 enthält, in der 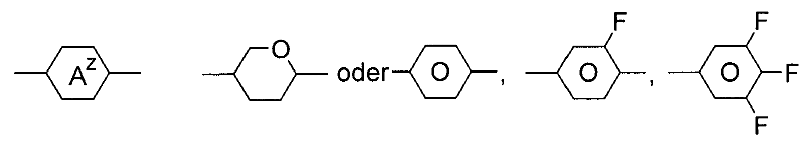 bedeutet,
X^{z} F, Cl, CN, NCS, OCF₃, CF₃ oder SF₅ bedeutet und
R^{z} einen Alkylrest mit 1 bis 7 Kohlenstoffatomen bedeutet, wobei der Alkylrest unsubstituiert oder ein- oder mehrfach mit Halogen substituiert ist.

10. Mesogenes Medium nach einem oder mehreren der Ansprüche 1 bis 9, **dadurch gekennzeichnet, dass** das Medium zusätzlich eine oder mehrere Verbindungen der Formel N 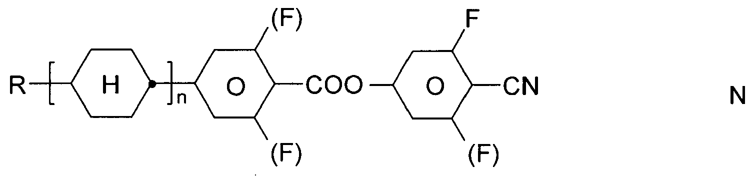 enthält, in der
R die unter Formel I für R angegebene Bedeutung besitzt,
"Alkyl" Alkyl mit 1 bis 7 C-Atomen bedeutet und
n für 0 oder 1 steht.

11. Mesogenes Medium nach einem oder mehreren der Ansprüche 1 bis 10, **dadurch gekennzeichnet, dass** das Medium zusätzlich eine oder mehrere Verbindungen enthält, die aus der Gruppe der Esterverbindungen der Formel E 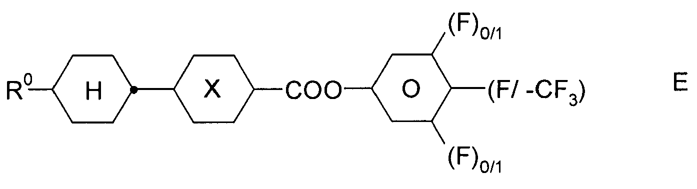 ausgewählt sind, in der R⁰ die für R unter Formel I angegebene Bedeutung besitzt und 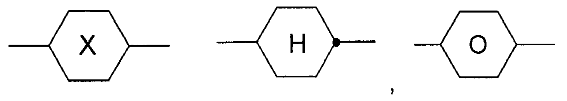 oder 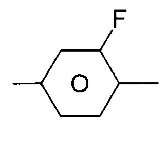 bedeutet.

12. Mesogenes Medium nach einem oder mehreren der Ansprüche 1 bis 11, **dadurch gekennzeichnet, dass** das Medium zusätzlich eine Verbindung oder mehrere Verbindungen enthält, die aus der Gruppe der Formeln Q-1 und Q-2 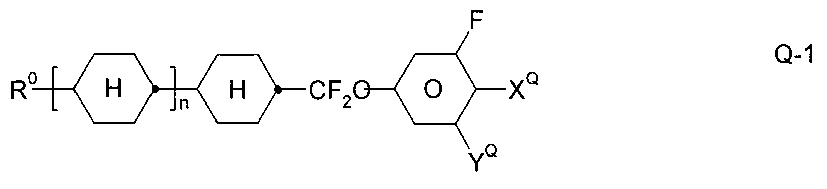 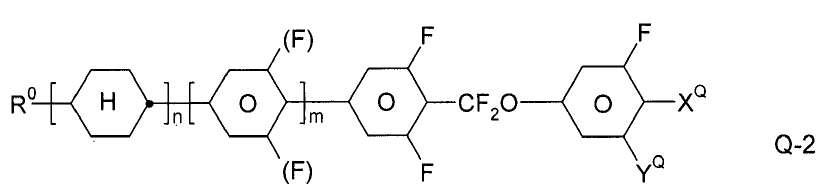 ausgewählt sind, in denen R⁰ die für R unter Formel I angegebene Bedeutung besitzt und
n und m unabhängig voneinander für 0 oder 1 stehen.

13. Mesogenes Medium nach einem oder mehreren der Ansprüche 1 bis 12, **dadurch gekennzeichnet, dass** das Medium zusätzlich eine oder mehrere Dioxanverbindungen enthält, die aus der Gruppe der Formeln Dx-1 und Dx-2 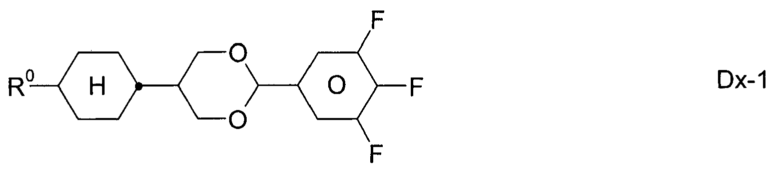 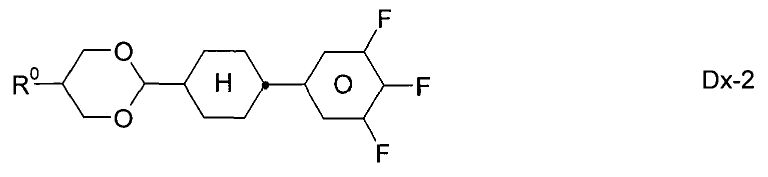 ausgewählt sind, in denen R⁰ die für R unter Formel I angegebene Bedeutung besitzt.

14. Lichtsteuerndes Element, **dadurch gekennzeichnet, dass** es ein mesogenes Medium nach einem oder mehreren der Ansprüche 1 und 13 enthält.

15. Elektrooptische Anzeige, **dadurch gekennzeichnet, dass** sie ein mesogenes Medium nach einem oder mehreren der Ansprüche 1 bis 13 enthält.

## Revendications

1. Milieu mésogène basé sur un mélange de composés diélectriquement positifs, **caractérisé en ce qu'**il comprend :
a) au moins un composé de la formule I : 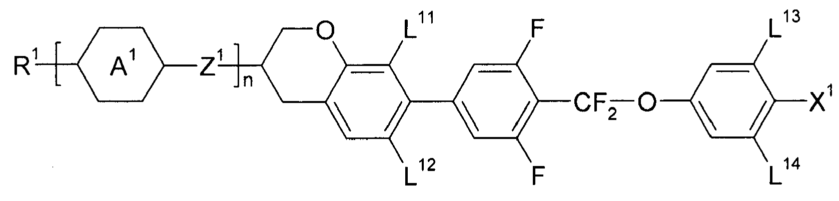 dans laquelle :
L¹¹ à L¹⁴ sont, indépendamment les uns des autres, H ou F, 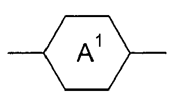 est un cycle aromatique et/ou alicyclique, ou un groupe comprenant deux cycles aromatiques ou alicycliques fusionnés ou plus, dans lequel ces cycles contiennent en option un ou plusieurs hétéroatome(s) choisi(s) parmi N, O et/ou S, et sont en option mono-substitués, disubstitués ou polysubstitués par R,
R est halogène, CN, NCS, SCN, SF₅, SO₂CF₃ ou alkyle, lequel est en chaîne droite ou ramifié, comporte 1 à 20 atome(s) de C, est non substitué, monosubstitué ou polysubstitué par F, Cl, ou CN, et où un ou plusieurs groupe(s) CH₂ est/sont en option remplacé(s), dans chaque cas indépendamment les uns des autres, par -O-, -S-, -NH-, -NR⁰¹- -SiR⁰¹R⁰²- -CO-, -COO-, -OCO-, -OCO-O-, -S-CO-, -CO-S-,-CY⁰¹=CY⁰¹- ou -C≡C- de telle sorte que des atomes de O et/ou de S ne soient pas liés directement les uns aux autres,
R¹ présente la signification donnée pour R,
Z¹ est -CO-O-, -O-CO-, -S-CO-, -CO-S-, -CO-NR⁰¹-, -NR⁰¹-CO-, -OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰¹-, -CR⁰¹=CH-, -CY⁰¹=CY⁰²-, -C≡C-, -(CH₂) ₄-, -CH=CH-CO-O-, -O-CO-CH=CH- ou une liaison simple,
Y⁰¹ et Y⁰² sont, indépendamment l'un de l'autre, F, Cl ou CN, et à titre d'alternative, l'un d'eux peut être H,
R⁰¹ et R⁰² sont, indépendamment l'un de l'autre, H ou alkyle avec 1 à 12 atome(s) de C,
n est 0 ou 1,
X¹ est halogène, OCF₃, CF₃, OCHF₂, CN, NCS, SCN, SF₅ ou SO₂CF₃, et
b) 1-20 % en poids d'au moins un composé chiral ou de plusieurs composés chiraux présentant un HTP ≥ 20 µm ⁻¹, et
ledit milieu mésogène de a) et b) présentant une phase bleue.

2. Milieu mésogène selon la revendication 1, **caractérisé en ce qu'**il comprend au moins un composé de la formule I, dans laquelle R¹ représente halogène, n-alkyle, n-alcoxy avec 1 à 9 atome(s) de C ou alkényle, alkényloxy ou alcoxyalkyle avec 2 à 9 atomes de C.

3. Milieu mésogène selon la revendication 1 ou 2, **caractérisé en ce qu'**il comprend au moins un composé de la formule I, dans laquelle Z¹ représente -CO-O-, -O-CO-, -CF₂-O-, -O -CF₂ - ou une liaison simple.

4. Milieu mésogène selon une ou plusieurs des revendications 1 à 3, **caractérisé en ce qu'**il comprend au moins un composé de la formule I, dans laquelle n représente 1.

5. Milieu mésogène selon une ou plusieurs des revendications 1 à 4, **caractérisé en ce qu'**il comprend un ou plusieurs composé(s) de la formule II : 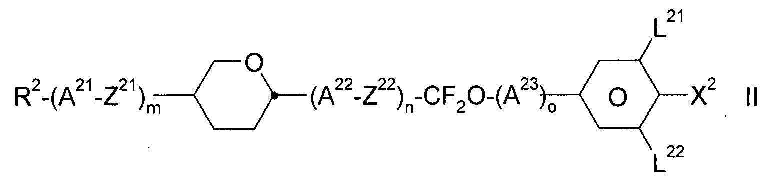 dans laquelle :
R² présente la signification donnée sous la formule I pour R¹,
A²¹, A²² et A²³ sont, chacun indépendamment des autres, 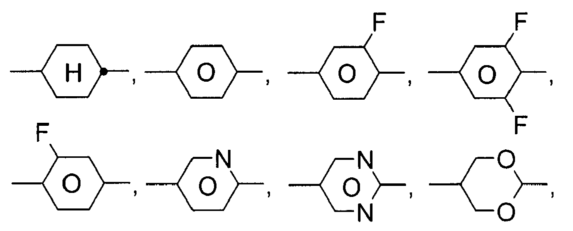 d'où il résulte que chacun de A²¹ et A²² peut présenter la même signification ou une signification différente s'il est présent deux fois,
Z²¹ et Z²² sont, chacun indépendamment de l'autre, une liaison simple, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡C-, -CH₂O-, -OCH₂-, -CF₂O-, -OCF₂-, -CO-O-ou -O-CO-, d'où il résulte que chacun de Z²¹ et Z²² peut présenter la même signification ou une signification différente s'il est présent deux fois,
X² est halogène, -CN, -NCS, -SF₅, -SO₂CF₃, un radical alkyle, alkényle, alkényloxy ou alkylalcoxy ou alcoxy, chacun étant monosubstitué ou polysubstitué par CN et/ou halogène,
L²¹ et L²² sont, chacun indépendamment de l'autre, H ou F, et
m est 0, 1 ou 2,
n est 0, 1, 2 ou 3,
o est 0, 1 ou 2, et
m + n + o est 3 ou moins.

6. Milieu mésogène selon une ou plusieurs des revendications 1 à 5, **caractérisé en ce qu'**il comprend au moins un ou plusieurs composé(s) de la formule III : 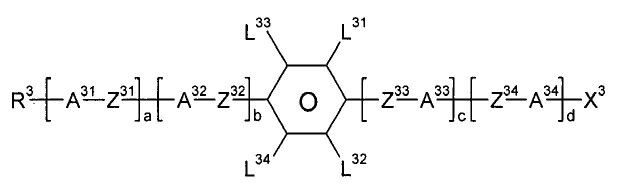 dans laquelle :
a, b, c et d sont, chacun indépendamment des autres, 0, 1 ou 2, d'où il résulte que
a + b + c + d est 4 ou moins,
A³¹, A³², A³³ et A³⁴ sont, chacun indépendamment des autres, 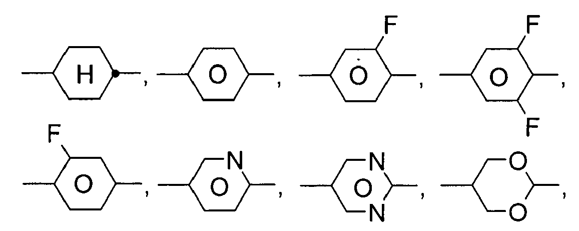 d'où il résulte que chacun de A³¹, A³², A³³ et A³⁴ peut présenter la même signification ou une signification différente s'il est présent deux fois,
Z³¹, Z³², Z³³ et Z³⁴ sont, chacun indépendamment des autres, une liaison simple, -(CH₂)₄)-, -CH₂CH₂-, -CF₂-CF₂-, -CF₂-CH₂-, -CH₂-CF₂-, -CH=CH-, -CF=CF-, -CF=CH-, -(CH₂)₃O-, -O(CH₂)₃-, -CH=CF-, -C≡D-, -CH₂O-, -OCH₂-, -CF₂O-, -OCF₂-, -CO-O- ou -O-CO-, d'où il résulte que chacun de Z³¹, Z³², Z³³ et Z³⁴ peut présenter la même signification ou une signification différente s'il est présent deux fois,
R³ est un radical alkyle ou alcoxy comportant 1 à 15 atome(s) de carbone, où un ou plusieurs groupe(s) méthylène dudit radical alkyle ou alcoxy peut/peuvent être remplacé(s), indépendamment les uns des autres, par -O-, -S-, -SiR^{x}R^{y}-, -CH=CH-, -C≡D-, -CO-O- et/ou -O-CO- de telle sorte que des atomes d'oxygène et/ou de soufre ne soient pas liés directement les uns aux autres, ledit radical alkyle ou alcoxy étant non substitué ou monosubstitué par un groupe CN ou monosubstitué ou polysubstitué par halogène,
L³¹, L³², L³³ et L³⁴ sont, chacun indépendamment des autres, hydrogène, halogène, un groupe CN, un radical alkyle ou alcoxy comportant 1 à 15 atome(s) de carbone, où un ou plusieurs groupe(s) méthylène dudit radical alkyle ou alcoxy peut/peuvent être remplacé(s), indépendamment les uns des autres, par -O-, -S-, -SiR^{x}R^{y}-, -CH=CH-, -C≡D-, -CO-O- et/ou -O-CO- de telle sorte que des atomes d'oxygène et/ou de soufre ne soient pas liés directement les uns aux autres, ledit radical alkyle ou alcoxy étant non substitué ou mono-substitué par un groupe -CN ou monosubstitué ou polysubstitué par halogène, étant entendu qu'au moins l'un de L³¹, L³², L³³ et L³⁴ n'est pas hydrogène,
X³ est F, Cl, CF₃, OCF₃, CN, NCS, SF₅ ou SO₂-R^{z},
R^{x} et R^{y} sont, indépendamment l'un de l'autre, hydrogène ou un radical alkyle comportant 1 à 7 atome(s) de carbone, et
R^{z} est un radical alkyle comportant 1 à 7 atome(s) de carbone, ledit radical alkyle étant non substitué ou monosubstitué ou polysubstitué par halogène.

7. Milieu mésogène selon une ou plusieurs des revendications 1 à 6, **caractérisé en ce qu'**il comprend de façon additionnelle un ou plusieurs composé(s) qui augmente(nt) l'étendue de phase de la phase bleue et/ou qui diminue(nt) la dépendance vis-à-vis de la température de l'effet électro-optique.

8. Milieu mésogène selon une ou plusieurs des revendications 1 à 7, **caractérisé en ce que** le milieu comprend de façon additionnelle un précurseur de polymère qui, suite à une polymérisation, stabilise l'étendue de phase de la phase bleue et/ou diminue la dépendance vis-à-vis de la température de l'effet électro-optique.

9. Milieu mésogène selon une ou plusieurs des revendications 1 à 8, **caractérisé en ce que** le milieu comprend de façon additionnelle un ou plusieurs composé(s) de la formule Z : 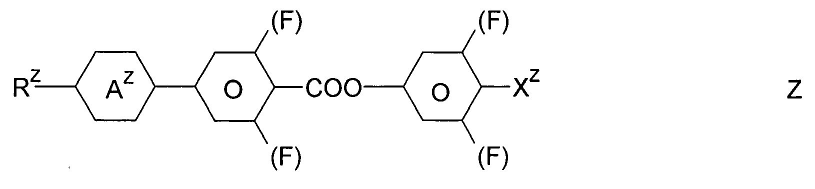 dans laquelle 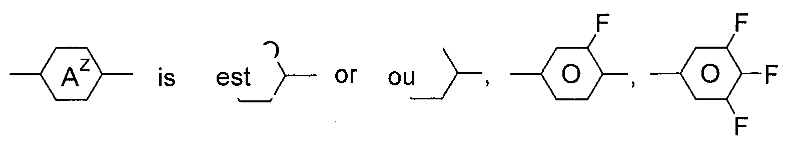
X^{z} est F, CI, CN, NCS, OCF₃, CF₃ ou SF₅, et
R^{z} est un radical alkyle comportant 1 à 7 atome(s) de carbone, le radical alkyle étant non substitué ou monosubstitué ou polysubstitué par halogène.

10. Milieu mésogène selon une ou plusieurs des revendications 1 à 9, **caractérisé en ce que** le milieu comprend de façon additionnelle un ou plusieurs composé(s) de la formule N : 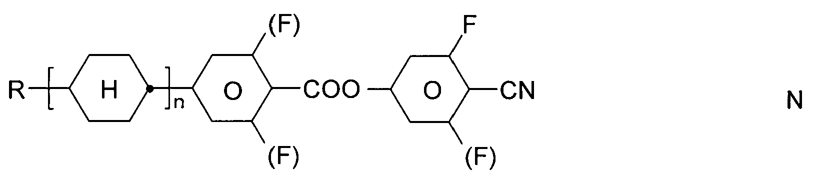 dans laquelle
R présente la signification donnée pour la formule I pour R,
"alkyl" est alkyle avec 1 à 7 atome(s) de C, et
n est 0 ou 1.

11. Milieu mésogène selon une ou plusieurs des revendications 1 à 10, **caractérisé en ce que** le milieu comprend de façon additionnelle un ou plusieurs composé(s) choisi(s) parmi le groupe de composés ester de la formule E : 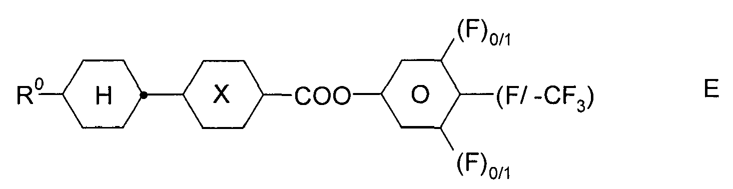 dans laquelle R⁰ présente la signification donnée pour R sous la formule I et 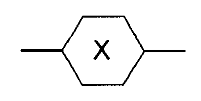 est

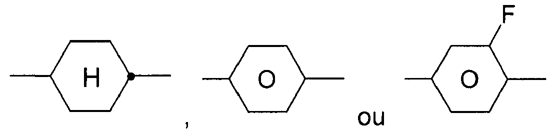

12. Milieu mésogène selon une ou plusieurs des revendications 1 à 11, **caractérisé en ce que** le milieu comprend de façon additionnelle un ou plusieurs composé(s) choisi(s) parmi le groupe des formules Q-1 et Q-2 : 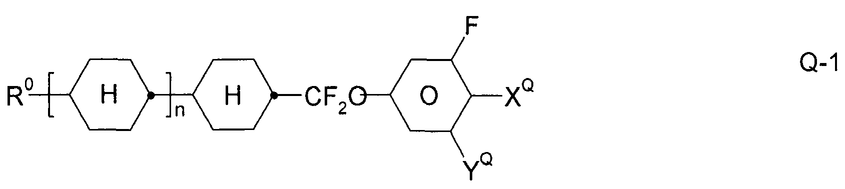 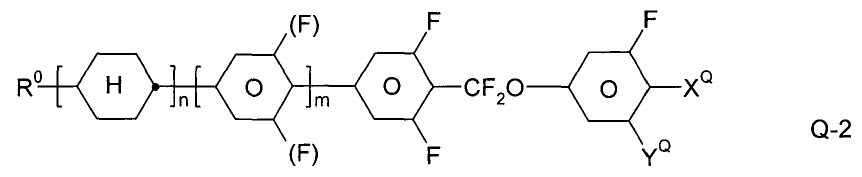 dans lesquelles R⁰ présente la signification donnée pour R sous la formule I et
n et m sont, indépendamment l'un de l'autre, 0 ou 1.

13. Milieu mésogène selon une ou plusieurs des revendications 1 à 12, **caractérisé en ce que** le milieu comprend de façon additionnelle un ou plusieurs composé(s) dioxane, choisi(s) parmi le groupe des formules Dx-1 et Dx-2 : 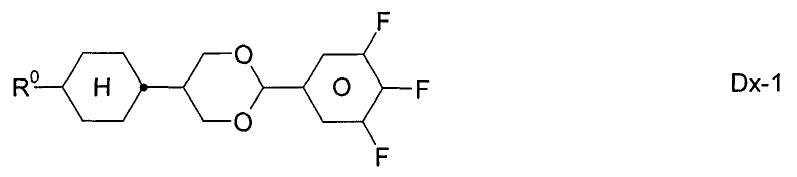 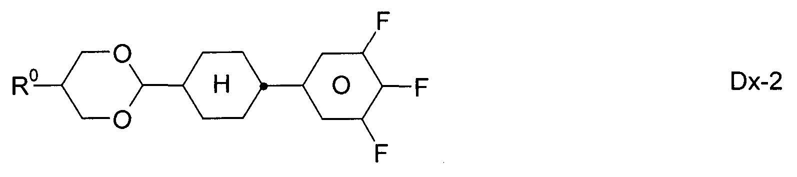 dans lesquelles R⁰ présente la signification donnée pour R sous la formule I.

14. Elément de commande de lumière, **caractérisé en ce qu'**il comprend un milieu mésogène selon une ou plusieurs des revendications 1 et 13.

15. Affichage électro-optique, **caractérisé en ce qu'**il contient un milieu mésogène selon une ou plusieurs des revendications 1 à 13.
